# EUROPEAN PATENT SPECIFICATION

(11) **EP 3 289 077 B1**
(45) Date of publication and mention of the grant of the patent: **15.04.2020**
(21) Application number: 16724301.3
(22) Date of filing: 29.04.2016
(51) Int. Cl.: C12N 9/22, C12N 11/00, C12N 11/06

(54) **METHOD FOR IN VITRO TRANSCRIPTION USING AN IMMOBILIZED RESTRICTION ENZYME**
VERFAHREN FÜR IN VITRO TRANSKRIPTION MITTELS EINES IMMOBILISIERTEN RESTRIKTIONSENZYMS
PROCÉDÉ DE TRANSCRIPTION IN VITRO AVEC UN ENZYME DE RESTRICTION IMMOBILISEE

(30) Priority: 30.04.2015 WO PCT/EP2015/059559
(43) Date of publication of application: 07.03.2018
(73) Proprietor: CureVac AG, 72076 Tübingen (DE)
(72) Inventor: ROOS, Tilmann, 72076 Tübingen (DE); YAZDAN PANAH, Benyamin, 72076 Tübingen (DE); KUNZE, Martin, 72076 Tübingen (DE)
(74) Representative: Lasar, Andrea Gisela
(86) International application number: PCT/EP2016/059651
(87) International publication number: WO 2016/174227

(56) References cited:
- S. C. WALKER: "General plasmids for producing RNA in vitro transcripts with homogeneous ends", NUCLEIC ACIDS RESEARCH, vol. 31, no. 15, 1 August 2003 (2003-08-01), pages 82e-82, XP055213720, DOI: 10.1093/nar/gng082
- VARSHNEY H ET AL: "Immobilization of the restriction endonuclease EcoRI usefulness of a polyclonal antibody support", ENZYME AND MICROBIAL TECHNOLOGY, STONEHAM, MA, US, vol. 25, no. 3-5, 1 August 1999 (1999-08-01), pages 172-176, XP027534570, ISSN: 0141-0229 [retrieved on 1999-08-01]
- Yolanda Y Davidson ET AL: "Immobilization of the restriction enzymes HaeIII and HindIII on porous silica particles via a glutaraldehyde linkage for the micro-digestion of dsDNA with analysis by capillary electrophoresis", , 1 January 2001 (2001-01-01), XP055213725, Retrieved from the Internet: URL:http://onlinelibrary.wiley.com/doi/10. 1002/1615-9314%2820010101%2924:1%3C10::AID -JSSC10%3E3.0.CO;2-N/epdf [retrieved on 2015-09-16]
- MONCEF NASRI ET AL: "Immobilization of the restriction endonucleasesII andIII", APPLIED BIOCHEMISTRY AND BIOTECHNOLOGY ; PART A: ENZYME ENGINEERING AND BIOTECHNOLOGY, HUMANA PRESS INC, NEW YORK, vol. 15, no. 2, 1 August 1987 (1987-08-01) , pages 119-130, XP035176405, ISSN: 1559-0291, DOI: 10.1007/BF02801313
- Pratul K Agarwal ET AL: "COMMUNICATION TO THE EDITOR Construction of a Multi RE Module: Exploitation of Mechanochemistry of Restriction Endonucleases", , 20 October 1999 (1999-10-20), XP055213726, Retrieved from the Internet: URL:http://onlinelibrary.wiley.com/doi/10. 1002/%28SICI%291097-0290%2819991020%2965:2 %3C233::AID-BIT15%3E3.0.CO;2-S/epdf [retrieved on 2015-09-16]
- MATEO ET AL: "Improvement of enzyme activity, stability and selectivity via immobilization techniques", ENZYME AND MICROBIAL TECHNOLOGY, STONEHAM, MA, US, vol. 40, no. 6, 29 March 2007 (2007-03-29) , pages 1451-1463, XP022003968, ISSN: 0141-0229, DOI: 10.1016/J.ENZMICTEC.2007.01.018
- JOHANNA MANSFELD ET AL: "Probing the Unfolding Region in a Thermolysin-like Protease by Site-Specific Immobilization +", BIOCHEMISTRY, vol. 38, no. 26, 1 June 1999 (1999-06-01), pages 8240-8245, XP055213727, ISSN: 0006-2960, DOI: 10.1021/bi990008p
- HELMUT K. W. KALLWASS ET AL: "Site-specific immobilization of an L-lactate dehydrogenase via an engineered surface cysteine residue", BIOTECHNOLOGY LETTERS, vol. 15, no. 1, 1 January 1993 (1993-01-01), pages 29-34, XP055213728, ISSN: 0141-5492, DOI: 10.1007/BF00131548

## Description

### Field of the invention

The present invention relates to a method for *in vitro* transcription of a linear template DNA which is produced using an immobilized restriction endonuclease. The invention also relates to mutated restriction enzymes which are suitable for immobilization and a solid support to which these restriction enzymes are immobilized. Further, the present invention relates to an enzyme reactor containing said immobilized restriction endonuclease which enzyme reactor can be used for preparing linearized template DNA. Finally, the present invention relates to the use of said enzyme reactor for preparing a linear template DNA for *in vitro* transcription. In addition, the present invention relates to a kit comprising the immobilized restriction endonuclease.

### Background of the invention

In gene therapy and many other therapeutically relevant biochemical and biotechnological applications the use of nucleic acids for therapeutic and diagnostic purposes is of major importance. As an example, rapid progress has occurred in recent years in the field of gene therapy and promising results have been achieved. Nucleic acids are therefore regarded as important tools for gene therapy and prophylactic and therapeutic vaccination against infectious and malignant diseases.

Therapeutic ribonucleic acid (RNA) molecules represent a promising class of drugs. RNA-based therapeutics include mRNA molecules encoding antigens for use as vaccines (Fotin-Mleczek et al. (2012) J. Gene Med. 14(6): 428-439). In addition, it is envisioned to use RNA molecules for replacement therapies, e.g. for providing missing proteins such as growth factors or enzymes to patients (Kariko et al. (2012) Mol. Ther. 20(5): 948-953; Kormann et al. (2012) Nat. Biotechnol. 29(2): 154-157). Furthermore, the therapeutic use of non-coding immunostimulatory RNA molecules (Heidenreich et al. Int J Cancer. 2014 Dec 21. doi: 10.1002/ijc.29402.) and other non-coding RNAs such as microRNAs and long non-coding RNAs is considered (Esteller (2011) Nat. Rev. Genet. 15(12): 861-74).

RNA-based therapeutics exhibit some superior properties over DNA cell transfection. As generally known, transfection of DNA molecules may lead to serious problems. E.g. application of DNA molecules bears the risk that the DNA integrates into the host genome. Integration of foreign DNA into the host genome can have an influence on expression of the host genes and possibly triggers expression of an oncogene or destruction of a tumor suppressor gene. Furthermore, a gene - and therefore the gene product - which is essential to the host may be inactivated by integration of the foreign DNA into the coding region of this gene. There may be a particular danger if integration of the DNA takes place into a gene which is involved in regulation of cell growth. Nevertheless, DNA still represents an important tool, even though some risks are associated with the application of DNA. These risks do not occur, if RNA, particularly mRNA, is used instead of DNA. An advantage of using RNA rather than DNA is that no virus-derived promoter element has to be administered *in vivo* and no integration into the genome may occur. Furthermore, the RNA does not have to cross the barrier to the nucleus.

Short RNA molecules can be synthesized by chemical methods, whereas longer RNAs are typically produced by *in vitro* transcription reactions using a suitable DNA template with a bacteriophage-derived promoter, an RNA polymerase, for example bacteriophage SP6, T3 or T7 RNA polymerase and ribonucleoside triphosphates (NTPs). 5' cap structures can also be introduced into *in vitro* transcribed RNA (Pascolo S. (2006) Methods Mol Med. 127: 23-40). Walker et al. provide general plasmids for producing RNA *in vitro* transcripts with homogenous ends (Walker et al. Nucleic Acids Research, 2003, Vol. 31, No. 15: 82e-82).

The enzymes commonly in use for transcribing a DNA template into RNA *in vitro* comprise the highly efficient viral SP6, T3, and T7 RNA Polymerases. These enzymes recognize and specifically bind to distinct DNA motifs, called promoters from where the RNA synthesis is initiated. To generate a homogeneous pool of RNA molecules of a distinct size, precise termination of the *in vitro* RNA synthesis reaction is required. This is particularly important for the generation of mRNA molecules from plasmid DNA templates as plasmid DNA often also encodes poly-A motifs to generate the 3' poly-A-tail of the mRNA. A proper poly-A-tail is necessary for the translation initiation of the mRNA (via PBP and eIF-4) into proteins. Therefore, a precise termination of the RNA polymerase reaction is essential for generating high-quality mRNAs having the same length and displaying a high translation efficiency when applied *in vivo* as a potential pharmaceutical. Furthermore, for RNAs used for therapeutic purposes, it is essential that all synthesized RNA molecules have the same characteristics, particularly the same length to guarantee that the medicament always shows the same (therapeutic) effects.

However, the native T3, T7, and SP6 polymerase terminators have a termination efficiency of only approximately 80%, meaning that these enzymes are prone for transcriptional read-through. Therefore, these polymerases also produce a range of larger RNA molecules that could also bear sequence elements of the plasmid vector at the 3' end. Therefore, the termination of the RNA polymerase reaction has to be guaranteed by means other than the transcriptional terminators.

The common way of controlling the termination of the RNA polymerase reaction is the linearization of the purified circular DNA vector (e.g. plasmid DNA) precisely after the RNA coding sequence/insert. This is usually performed prior to the *in vitro* transcription reaction. For linearization, restriction endonucleases are commonly used that cut the DNA at or near specific recognition nucleotide sequences, known as restriction sites.

Typically, a batch approach is used to linearize circular DNA. To this end, 1-2 Units restriction endonuclease are mixed with 0.1-1 µg DNA in the appropriate buffer and incubated at 37°C (Sambrook et al., Molecular Cloning, a laboratory manual, 2nd edition, Cold Spring Harbor Laboratory Press 1989). After the linearization of plasmid DNA with the respective restriction endonuclease, the linear DNA has to be purified from buffer, solvents and enzymes before it can be used as a template for the RNA polymerase reaction. Further, restriction endonucleases have to be heat-inactivated after linearization has occurred. This is an ineffective method, as restriction endonucleases can only be used once for the linearization of one batch/reaction. Moreover, restriction endonucleases may aggregate in solution (Chi et al. (2003) Pharmaceutical Research 20.9: 1325-1336) which additionally reduces restriction efficiency and leads to denaturation and inactivation of the endonucleases which eventually decreases the efficiency (time and costs) of the linearization process.

The purification of linearized template DNA is commonly performed via silica membranes (in a small scale laboratory setting) or via precipitation using hazardous chemicals such as phenol and chloroform. Subsequently, such toxic substances and other impurities (e.g., heat inactivated restriction endonucleases) have to be removed using further time-consuming purification and cleaning procedures. Furthermore, in the production process of clinical-grade RNA quality controls have to be introduced in the process to measure the presence of these organic solvents in the final product. Despite its capability of generating linear template DNA for RNA synthesis in a small batch scale, the common approach illustrated above remains highly cost- and time intensive and less controllable and therefore does not meet industrial demands for large-scale pharmaceutical RNA production.

Hence, there is still a need for an efficient process for preparing a linear template DNA used for *in vitro* transcription.

This and other problems are solved by the claimed subject matter.

### Summary of the invention

A solution of these problems is the use of restriction endonucleases which are immobilized to a solid support. The present invention is particularly suitable for enhancing and improving linearization of circular DNA for large-scale production of *in vitro* transcribed RNA. The use of an enzyme reactor containing the immobilized restriction endonuclease allows a controlled DNA linearization procedure in the reactor. Moreover, heat inactivation of the restriction enzyme after linearization is not necessary and hazardous organic solvents such as phenol and chloroform are not necessary for purification of the linearized template DNA. Another advantage of the enzyme reactor is that immobilized restriction endonucleases in the reactor can be reused for several restriction cycles which additionally decreases the costs for restriction endonucleases. The immobilization of restriction endonucleases provides an excellent base for increasing availability of enzyme to the circular DNA substrate with greater turnover over a considerable period of time. Therefore, the linearization procedure performed using the immobilized restriction endonuclease is more time-efficient than the batch process commonly used in the art. Furthermore, the herein disclosed immobilization strategies for restriction endonucleases may stabilize their structure, activities and stability. Immobilization of restriction endonucleases may therefore also improve the efficiency of the enzymatic linearization reaction.

Summarizing the above, the use of immobilized restriction endonucleases meets industrial demands for large-scale production *of in vitro* transcribed RNA as it is more economic (e.g., less consumption of restriction endonucleases, simple separation of linearized template DNA, less purification steps necessary to remove organic solvents and impurities), more ecologic (e.g., less chemicals are consumed for linearized template DNA purification) and more controllable (the automated DNA linearization process is less prone to human errors, inlet and outlet flow can be controlled).

To facilitate a directed and stable immobilization of a restriction endonuclease, the inventors generated a mutated bacterial restriction endonuclease (*Escherichia coli* EcoRI), wherein the naturally occurring cysteine residue of the protein at position 218 has been substituted with an alanine residue, and wherein an C-terminal cysteine has been introduced (attached via a linker element to the C-terminus of the protein). The inventors found that said mutated EcoRI showed enzymatic activity in solution. In a further experiment, the inventors found that said mutated EcoRI immobilized on a solid support via a covalent disulfide bond showed similar enzymatic activity compared to the respective soluble enzyme.

Finally, the invention relates to an enzyme reactor comprising the immobilized restriction enzyme which provides for the scale-up of the template linearization reaction. Automation of the linearization reaction and the separation of the linearized template together with the repeated utilization of enzymes provides thus for an ecologic and economic production of linear template DNA for RNA *in vitro* transcription.

In further experiments, the inventors used immobilized EcoRI enzyme to generate a linear DNA template in an enzyme reactor and used the linear template for RNA *in vitro* transcription.

The present invention also teaches general rules and strategies of linearization reactor designs and their application(s).

Systems and methods of making and using such immobilized restriction endonucleases and methods for generating immobilized restriction endonucleases which may be used in an enzyme reactor are provided in this invention.

Accordingly, the present invention relates to a method for *in vitro* transcription of a linear template DNA, comprising the steps of:
a) contacting a circular DNA having at least one recognition sequence for a restriction enzyme with said restriction enzyme, wherein the restriction enzyme is immobilized to a solid support, thereby linearizing the circular DNA and providing the linear template DNA; and
b) transcribing the linear template DNA into RNA *in vitro,* and
c) obtaining the resulting RNA.

Preferably, the restriction enzyme is a type II restriction enzyme and more preferably it is selected from the group consisting of EcoRI, BciVI, XbaI, NdeI, AflII, SacI, KpnI, SmaI, BamHI, XbaI, SalI, Sbfl, PstI and HindIII.

Also preferably, the restriction enzyme is immobilized by covalent binding and more preferably the covalent binding is a disulfide bridge or a thioether bond and most preferably the restriction enzyme is immobilized by covalent binding to a thiol-activated support material.

The restriction enzyme may be bound to the thiol-activated support material via at least one cysteine residue, preferably a cysteine residue which is not present in the wild-type restriction enzyme.

More preferably, the at least one cysteine residue which is bound to the thiol-activated support material has been introduced by substituting an amino acid within the wild-type restriction enzyme with cysteine or by inserting a cysteine into the wild-type restriction enzyme, preferably by adding a cysteine residue to the N or C terminus of the restriction enzyme.

The restriction enzyme may be selected from the group consisting of:
a) EcoRI according to SEQ ID No. 1, wherein the lysine on position 277 of SEQ ID No. 1 is replaced with cysteine or a functional variant of EcoRI having at least 80% sequence identity to SEQ ID No. 1, wherein the lysine on a position corresponding to position 277 of SEQ ID No. 1 is replaced with cysteine;
b) EcoRI according to SEQ ID No.1 or a functional variant thereof having at least 80% sequence identity to SEQ ID No. 1, wherein a cysteine residue is added to the C-terminus, preferably via a linker;
c) HindIII according to SEQ ID No. 2, wherein the leucine on position 300 of SEQ ID No. 2 is substituted with cysteine or a functional variant of HindIII having at least 80% sequence identity to SEQ ID No. 2, wherein the leucine on a position corresponding to position 300 of SEQ ID No. 2 is substituted with cysteine;
d) HindIII according to SEQ ID No. 2, wherein a cysteine residue is added to the C-terminus, preferably via a linker, or a functional variant of HindIII having at least 80% sequence identity to SEQ ID No. 2, wherein a cysteine residue is added to the C-terminus, preferably via a linker; and
e) BciVI according to SEQ ID No. 3, wherein a cysteine residue is added to the C-terminus, preferably via a linker, or a functional variant thereof having at least 80% sequence identity to SEQ ID No. 3, wherein a cysteine residue is added to the C-terminus, preferably via a linker.

More preferably, the restriction enzyme has a sequence selected from the group consisting of SEQ ID Nos. 4, 7, 10, 11, 12, 15 and 27.

The linker may comprise 1 to 20 amino acids, preferably 5 to 15 and more preferably 12 amino acids. The linker may consist of glycine and/or serine residues. Most preferably, the linker consists of 12 glycine residues or has the sequence GGGGSGGGGS (SEQ ID No. 49).

If present, one or more cysteine residues in the wild-type restriction enzyme are replaced with another amino acid.

Hence, the restriction enzyme may be selected from the group consisting of:
a) EcoRI according to SEQ ID No. 1, wherein the cysteine on position 218 of SEQ ID No. 1 is replaced with another amino acid, preferably with alanine, valine or serine, or a functional variant of EcoRI having at least 80% sequence identity to SEQ ID No. 1, wherein the cysteine on a position corresponding to position 218 of SEQ ID No. 1 is replaced with another amino acid, preferably with alanine, valine or serine; or
b) BciVI according to SEQ ID No. 3, wherein the cysteine on position 271 of SEQ ID No. 3 is replaced with another amino acid, preferably with alanine, valine or serine, or a functional variant of BciVI having at least 80% sequence identity to SEQ ID No. 3, wherein the cysteine on a position corresponding to position 271 of SEQ ID No. 3 is replaced with another amino acid, preferably with alanine, valine or serine.

Preferably, the restriction enzyme is selected from the group consisting of:
a) EcoRI according to SEQ ID No. 1, wherein the lysine on position 277 of SEQ ID No. 1 is replaced with cysteine and the cysteine on position 218 of SEQ ID No. 1 is replaced with alanine, valine or serine or a functional variant of EcoRI having at least 80% sequence identity to SEQ ID No. 1, wherein the lysine on a position corresponding to position 277 of SEQ ID No. 1 is replaced with cysteine and the cysteine on a position corresponding to position 218 of SEQ ID No. 1 is replaced with alanine, valine or serine;
b) EcoRI according to SEQ ID No.1, wherein a cysteine residue is added to the C-terminus, preferably via a linker, and the cysteine on position 218 of SEQ ID No. 1 is replaced with alanine, valine or serine or a functional variant of EcoRI having at least 80% sequence identity to SEQ ID No. 1 wherein a cysteine residue is added to the C-terminus, preferably via a linker, and the cysteine on a position corresponding to position 218 of SEQ ID No. 1 is replaced with alanine, valine or serine;
c) BciVI according to SEQ ID No. 3, wherein a cysteine residue is added to the C-terminus, preferably via a linker, and the cysteine on position 271 of SEQ ID No. 3 is replaced with serine, valine or alanine or a functional variant of BciVI having at least 80% sequence identity to SEQ ID No. 3, wherein a cysteine residue is added to the C-terminus, preferably via a linker, and the cysteine on a position corresponding to position 271 of SEQ ID No. 3 is replaced with serine, valine or alanine.

The linker may comprise 1 to 20 amino acids, preferably 5 to 15 and more preferably 12 amino acids. The linker may consist of glycine and/or serine residues. Most preferably, the linker consists of 12 glycine residues or has the sequence GGGGSGGGGS (SEQ ID No. 49).

More preferably, the sequence of the restriction enzyme is selected from the group consisting of SEQ ID Nos. 5, 6, 8, 9, 13, 14, 16, 17, 20 to 22 and 28 to 30.

The enzyme may further comprise an amino acid sequence facilitating purification, such as an oligohistidine tag. Preferably, the oligohistidine tag comprises six histidine residues (SEQ ID No. 68). More preferably, the sequence of the restriction enzyme is selected from the group consisting of SEQ ID Nos. 23-26 and 31-34.

In a further preferred embodiment, the method as defined above is performed in an enzyme reactor.

Also preferably, the method further comprises a step of purifying the linear template DNA after providing the linear template DNA, more preferably, the linear template DNA is purified by filtration.

The disclosure also relates to restriction enzyme EcoRI according to SEQ ID No. 1, wherein the lysine on position 277 of SEQ ID No. 1 is replaced with cysteine and, optionally, the cysteine on position 218 of SEQ ID No. 1 is replaced with alanine, valine or serine or a functional variant of EcoRI having at least 80% sequence identity to SEQ ID No. 1, wherein the lysine on a position corresponding to position 277 of SEQ ID No. 1 is replaced with cysteine and, optionally, the cysteine on a position corresponding to position 218 of SEQ ID No. 1 is replaced with alanine, valine or serine. Preferably, the restriction enzyme has the amino acid sequence according to any of SEQ ID Nos. 4, 5, 6 and 20.

The disclosure also relates to restriction enzyme EcoRI according to SEQ ID No. 1, wherein a cysteine residue is added to the C-terminus and, optionally, the cysteine on position 218 of SEQ ID No. 1 is replaced with alanine, valine or serine or a functional variant of EcoRI having at least 80% sequence identity to SEQ ID No. 1, wherein a cysteine residue is added to the C-terminus and, optionally, the cysteine on a position corresponding to position 218 of SEQ ID No. 1 is replaced with alanine, valine or serine. Preferably, the restriction enzyme has the amino acid sequence according to any of SEQ ID Nos. 15, 16, 17 and 22.

The disclosure also relates to restriction enzyme EcoRI according to SEQ ID No. 1, wherein a cysteine residue is added to the C-terminus via a linker, preferably a linker comprising 12 glycine residues or a linker having the sequence GGGGSGGGGS (SEQ ID No. 49), and, optionally, the cysteine on position 218 of SEQ ID No. 1 is replaced with alanine, valine or serine or a functional variant of EcoRI having at least 80% sequence identity to SEQ ID No. 1, wherein a cysteine residue is added to the C-terminus via a linker, preferably a linker comprising 12 glycine residues or a linker having the sequence GGGGSGGGGS (SEQ ID No. 49), and, optionally, the cysteine on a position corresponding to position 218 of SEQ ID No. 1 is replaced with alanine, valine or serine. Preferably, the restriction enzyme has the amino acid sequence according to any of SEQ ID Nos. 7, 8, 9, 21 and 27-30.

The disclosure also relates to restriction enzyme EcoRI according to SEQ ID No. 1, wherein a cysteine residue is added to the C-terminus via a linker, preferably a linker having the sequence GGGGSGGGGS, and wherein the restriction enzyme further comprises a oligohistidine tag, preferably a oligohistidine tag comprising six histidine residues, optionally, the cysteine on position 218 of SEQ ID No. 1 is replaced with alanine, valine or serine or a functional variant of EcoRI having at least 80% sequence identity to SEQ ID No. 1, wherein a cysteine residue is added to the C-terminus via a linker, preferably a linker having the sequence GGGGSGGGGS, and wherein the restriction enzyme further comprises a oligohistidine tag, preferably a oligohistidine tag comprising six histidine residues, and, optionally, the cysteine on a position corresponding to position 218 of SEQ ID No. 1 is replaced with alanine, valine or serine. Preferably, the restriction enzyme has the amino acid sequence according to any of SEQ ID Nos. 23-26 and 31-34.

The disclosure also relates to restriction enzyme HindIII according to SEQ ID No. 2, wherein the leucine on position 300 of SEQ ID No. 2 is substituted with cysteine or wherein a cysteine residue is added to the C-terminus, preferably via a linker, or a functional variant of HindIII having at least 80% sequence identity to SEQ ID No. 2, wherein the leucine on a position corresponding to position 300 of SEQ ID No. 2 is substituted with cysteine or wherein a cysteine residue is added to the C-terminus, preferably via a linker. Preferably, the restriction enzyme has the amino acid sequence according to any of SEQ ID Nos. 10 and 11.

The disclosure also relates to restriction enzyme BciVI according to SEQ ID No. 3, wherein a cysteine residue is added to the C-terminus, preferably via a linker, and, optionally, the cysteine on position 271 of SEQ ID No. 3 is replaced with serine, valine or alanine or a functional variant of BciVI having at least 80% sequence identity to SEQ ID No. 3, wherein a cysteine residue is added to the C-terminus, preferably via a linker, and, optionally, the cysteine on a position corresponding to position 271 of SEQ ID No. 3 is replaced with serine, valine or alanine. Preferably, the restriction enzyme has the amino acid sequence according to any of SEQ ID Nos. 12, 13 and 14.

The disclosure also relates to a solid support to which the restriction enzyme as defined above is immobilized, preferably by a disulfide bridge or a thioether bond.

Further, the present invention relates to an enzyme reactor comprising a restriction enzyme which is immobilized to a solid support and a module for transcribing the linearized template DNA into RNA *in vitro,* wherein the module for *in vitro* transcription comprises a RNA polymerase, a nucleotide mixture and an *in vitro* transcription buffer.

Preferably, the restriction enzyme is a type II restriction enzyme, more preferably the restriction enzyme is selected from the group consisting of EcoRI, BciVI, XbaI, NdeI, AflII, SacI, KpnI, SmaI, BamHI, XbaI, SalI, Sbfl, PstI and HindIII.

Also preferably, the restriction enzyme is immobilized by covalent binding, more preferably the covalent binding is a disulfide bridge or a thioether bond and/or the restriction enzyme is immobilized by covalent binding to a thiol-activated support material.

The restriction enzyme may be bound to the thiol-activated support material via a cysteine residue, preferably a cysteine residue which is not present in the wild-type restriction enzyme.

Preferably, the cysteine residue has been introduced by substituting an amino acid within the wild-type restriction enzyme with cysteine or by adding a cysteine residue to the N or C terminus of the restriction enzyme.

The restriction enzyme is selected from the group consisting of:
a) EcoRI according to SEQ ID No. 1, wherein the lysine on position 277 of SEQ ID No. 1 is replaced with cysteine or a functional variant of EcoRI having at least 80% sequence identity to SEQ ID No. 1, wherein the lysine on a position corresponding to position 277 of SEQ ID No. 1 is replaced with cysteine;
b) EcoRI according to SEQ ID No.1, or a functional variant thereof having at least 80% sequence identity to SEQ ID No. 1, wherein a cysteine residue is added to the C-terminus, preferably via a linker;
c) HindIII according to SEQ ID No. 2, wherein the leucine on position 300 of SEQ ID No. 2 is substituted with cysteine or a functional variant of HindIII having at least 80% sequence identity to SEQ ID No. 2, wherein the leucine on a position corresponding to position 300 of SEQ ID No. 2 is substituted with cysteine;
d) HindIII according to SEQ ID No. 2, wherein a cysteine residue is added to the C-terminus, preferably via a linker, or a functional variant of HindIII having at least 80% sequence identity to SEQ ID No. 2, wherein a cysteine residue is added to the C-terminus, preferably via a linker; and
e) BciVI according to SEQ ID No. 3 or a functional variant thereof having at least 80% sequence identity to SEQ ID No. 3, wherein a cysteine residue is added to the C-terminus, preferably via a linker.

If present, one or more cysteine present in the wild-type restriction enzyme may be replaced with another amino acid.

The restriction enzyme may be selected from the group consisting of:
a) EcoRI according to SEQ ID No. 1, wherein the cysteine on position 218 of SEQ ID No. 1 is replaced with another amino acid or a functional variant of EcoRI having at least 80% sequence identity to SEQ ID No. 1, wherein the cysteine on a position corresponding to position 218 of SEQ ID No. 1 is replaced with another amino acid; or
b) BciVI according to SEQ ID No. 3, wherein the cysteine on position 271 of SEQ ID No. 3 is replaced with another amino acid or a functional variant of BciVI having at least 80% sequence identity to SEQ ID No. 3, wherein the cysteine on a position corresponding to position 271 of SEQ ID No. 3 is replaced with another amino acid.

Preferably, the restriction enzyme is selected from the group consisting of:
a) EcoRI according to SEQ ID No. 1, wherein the lysine on position 277 of SEQ ID No. 1 is replaced with cysteine and the cysteine on position 218 of SEQ ID No. 1 is replaced with alanine, valine or serine or a functional variant of EcoRI having at least 80% sequence identity to SEQ ID No. 1, wherein the lysine on a position corresponding to position 277 of SEQ ID No. 1 is replaced with cysteine and the cysteine on a position corresponding to position 218 of SEQ ID No. 1 is replaced with alanine, valine or serine;
b) EcoRI according to SEQ ID No.1, wherein a cysteine residue is added to the C-terminus, preferably via a linker, more preferably via a linker comprising 12 glycine residues, and the cysteine on position 218 of SEQ ID No. 1 is replaced with alanine, valine or serine or a functional variant of EcoRI having at least 80% sequence identity to SEQ ID No. 1, wherein a cysteine is added to the C-terminus, preferably via a linker, more preferably via a linker comprising 12 glycine residues and the cysteine on a position corresponding to position 218 of SEQ ID No. 1 is replaced with alanine, valine or serine; and
c) BciVI according to SEQ ID No. 3, wherein a cysteine residue is added to the C-terminus, preferably via a linker, and the cysteine on position 271 of SEQ ID No. 3 is replaced with serine, valine or alanine or a functional variant of BciVI having at least 80% sequence identity to SEQ ID No. 3, wherein a cysteine residue is added to the C-terminus, preferably via a linker, and the cysteine on a position 271 corresponding to position 271 of SEQ ID No. 3 is replaced with serine, valine or alanine.

More preferably, the sequence of the restriction enzyme is selected from the group consisting of SEQ ID Nos. 5, 6, 8, 9, 13, 14, 16, 17, 20 to 22 and 28 to 30.

The enzyme may further comprise an amino acid sequence facilitating purification, such as a oligohistidine tag. Preferably, the oligohistidine tag comprises six histidine residues. More preferably, the sequence of the restriction enzyme is selected from the group consisting of SEQ ID Nos. 23-26 and 31-34.

In a preferred embodiment the enzyme reactor further comprises a filter suitable for separating linearized template DNA from plasmid DNA.

The present invention also relates to a kit comprising:
a restriction endonuclease which is immobilized onto a solid support, and
a suitable restriction buffer, further comprising a RNA polymerase, a nucleotide mixture and an *in vitro* transcription buffer.

Preferably, the kit comprises a restriction endonuclease as described herein.

The kit may further comprise reagents for RNA *in vitro* transcription.

Another aspect of the invention relates to the use of an enzyme reactor as defined above for preparing a linear template DNA for *in vitro* transcription.

### Brief description of the figures

The figures shown in the following are merely illustrative and shall describe the present invention in a further way. These figures shall not be construed to limit the present invention thereto.

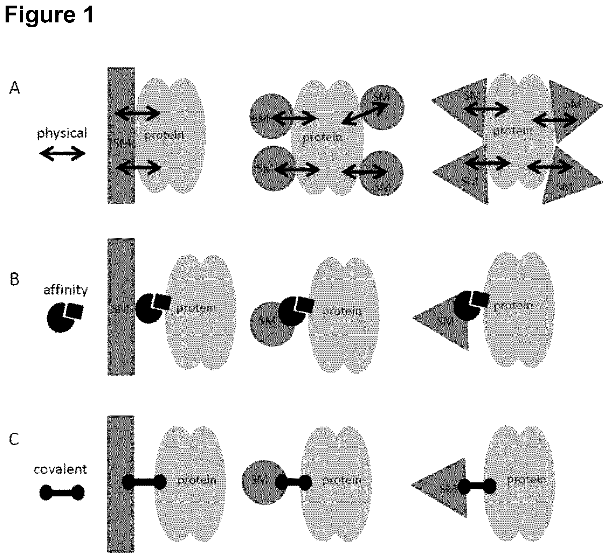

**Figure 1** **Representative immobilization procedures for a restriction endonuclease**
   Restriction endonucleases (protein) may be coupled by passive physical forces (A), by affinity capture (B) or by covalent bond (C) to a suitable support material (SM). As support materials, a planar surface (elongated rectangle), and two different globular supports are exemplified (round circle, triangle). (A): The coupling via physical adsorption (arrow) can occur on various, often random residues on a protein. Physical adsorption is based on weak physical intermolecular interactions including electrostatic, hydrophobic, van der Waals, and hydrogen bonding interactions. (B): The coupling via affinity, comprising bio-affinity, can occur on specified positions on a protein. Bio-affinity immobilization is based on strong interactions of two biomolecules, where one partner of the interaction is fused to the protein (square), and one partner is coated on the support material (circle). (C): The coupling via covalent bond (bar-bell) can occur via specific reactive residues on a protein. A covalent bond is a strong chemical bond. Reactive residues on the protein and reactive groups on the support material have to be present to form covalent bonds.

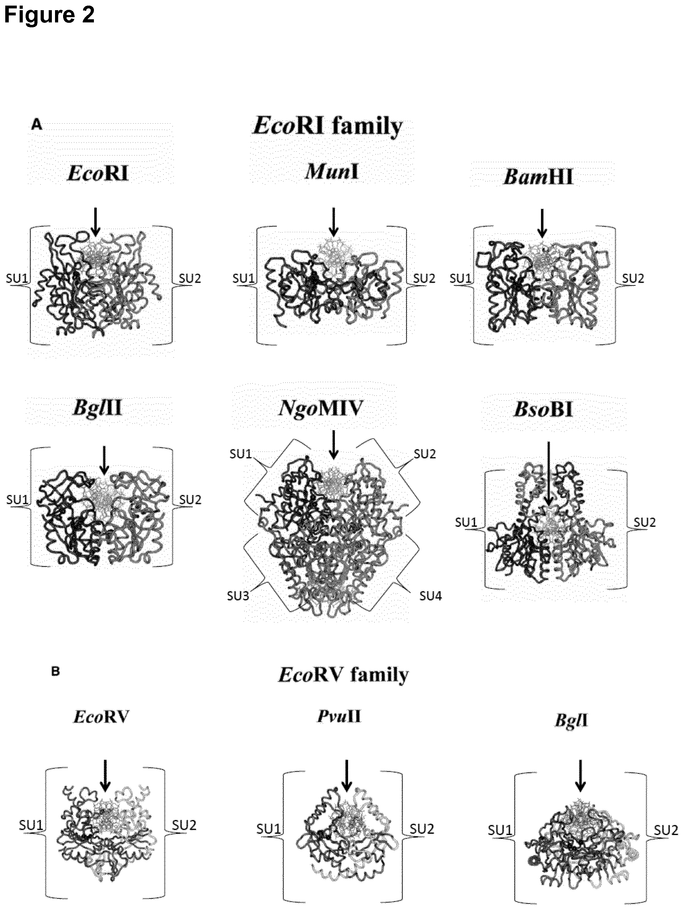

**Figure 2** **Crystal structures of specific restriction endonuclease-DNA complexes**
   The arrow indicates the spatial position of the double-stranded DNA substrate in the indicated restriction endonuclease. The homomers of the respective restriction endonucleases are indicated (brackets, sub units (SU)) and shown in different grey scales; figure adapted from (Pingoud and Jeltsch (2001) Nucl. Acids Res. 29.18: 3705-3727).

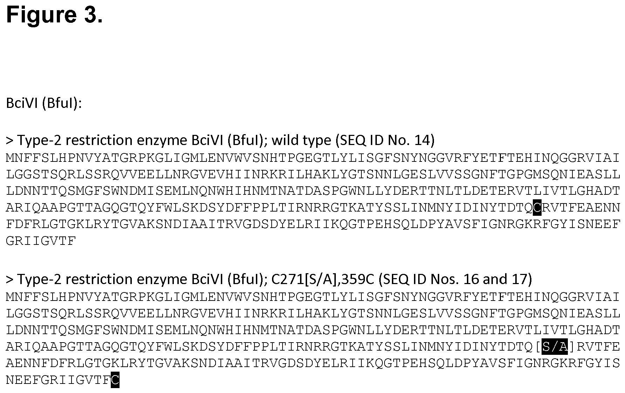

**Figure 3** **Amino acid sequence of the wild-type type II restriction endonuclease BciVI (BfuI) and of some exemplified mutants thereof**
   The amino acid sequence of BciVI wild type and two exemplified mutants (C271S, 359C) and (C271A, 359C) is shown in FASTA-format. The position 271 (native cysteine or substituted residues) and the C-terminal cysteine extension are highlighted (blackened). The wild type BciVI sequence according to SEQ ID No. 3 was retrieved from GenBank accession number: CAC12783.

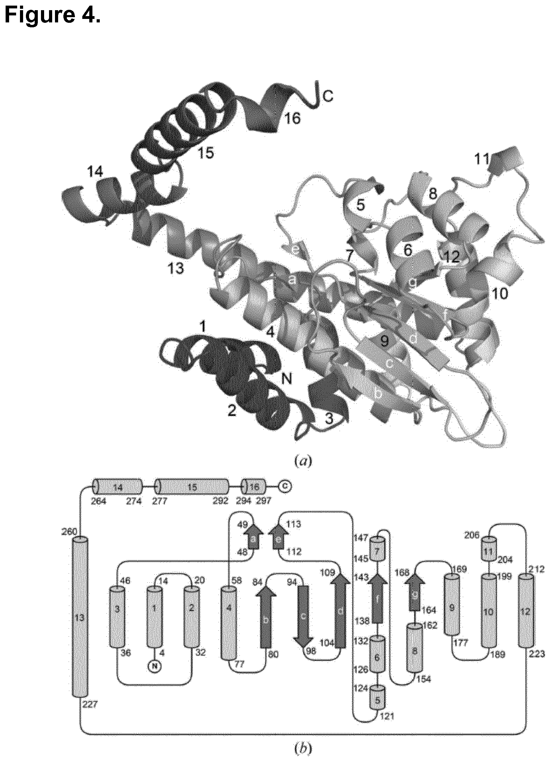

**Figure 4** **Structure of the HindIII monomer**
   (a) HindIII monomer with labelled helices (1-16) and beta-strands (a-g). (b) Topology diagram of HindIII. Beta-sheets: arrows; helices: zylinder. Both panels show that the C-terminus of the enzyme is free and accessible for immobilization with any support material. Figure taken from Watanabe, Nobuhisa, et al. (2009) Acta Crystallographica Section D: Biological Crystallography 65.12: 1326-1333.
**Figure 5** **Amino acid sequence of the wild-type type II restriction endonuclease HindIII and of some exemplified mutants thereof**
   The amino acid sequence of HindIII is displayed in FASTA-format. The introduced cysteine residues are highlighted (blackened). The wild-type HindIII sequence according to SEQ ID No. 2 was retrieved from GenBank (Accession number: P43870).
**Figure 6** **Topology of the restriction endonucleases EcoRI.**
   Catalytically important amino acid residues are marked with a cross (X), broadly defining the catalytic core of EcoRI. Amino acid residues involved in contacts to the bases of the recognition sequence on the DNA are marked with filled circles (•), broadly defining the DNA binding surface. Regions involved in dimerization contacts are shaded gray; figure taken from (Pingoud and Jeltsch (1997) Europ. J. Biochem. 246.1: 1-22).

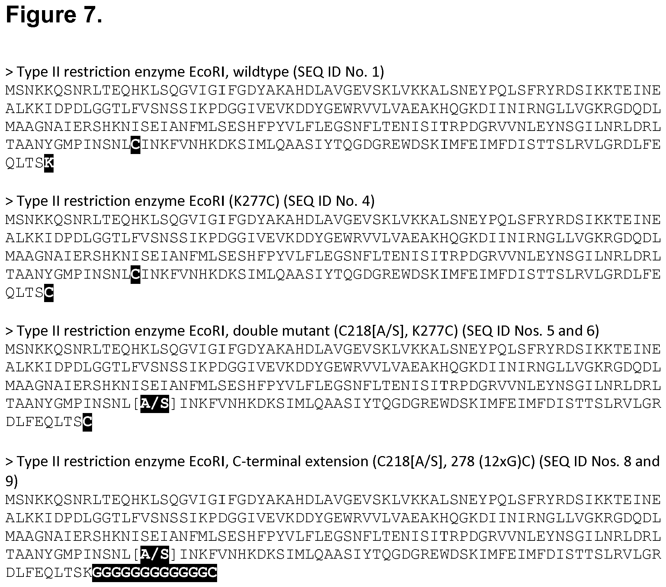

**Figure 7** **Amino acid sequence of the wild-type type II restriction endonuclease EcoRI and some exemplified mutants thereof**
   The amino acid sequence of EcoRI is displayed in FASTA-format. The native cysteine residue at position 218 and the lysine residue at position 277 are highlighted (blackened). The wild-type EcoRI sequence according to SEQ ID No. 1 was retrieved from GenBank (Accession number: P00642). EcoRI sequences suitable in the context of the present invention are also provided in the sequence listing (SEQ ID Nos. 4-9, 15-17, 20-34).

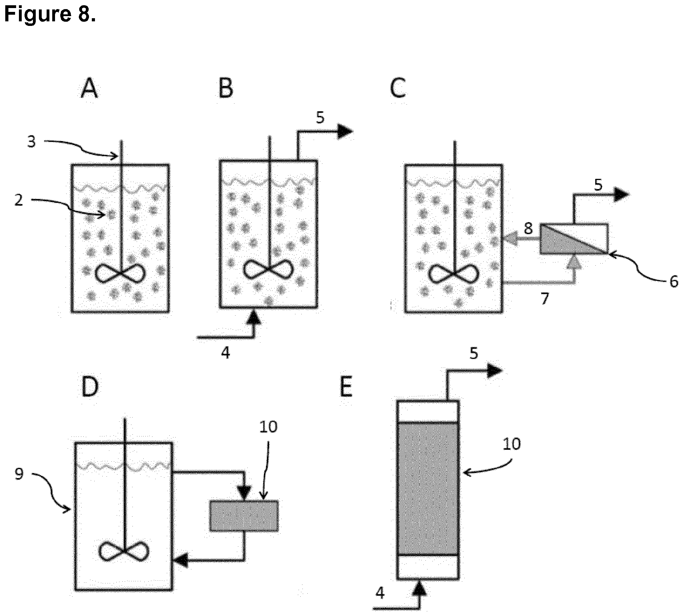

**Figure 8** **Examples of different configurations for enzyme reactors containing immobilized restriction endonucleases**
   (A) Stirred-tank batch reactor, (B) Continuous (stirred-tank) batch reactor, (C) stirred tank-ultrafiltration reactor, (D) Recirculation batch reactor and (E) Continuous packed bed reactor. Different components of the reactor types are indicated: (1) reactor vessel, (2) immobilized enzyme, (3) stirrer, (4) inlet, (5) outlet, (6) ultrafiltration device (diagonal line: ultrafiltration membrane), (7) feed tube for ultrafiltration device, (8) recirculation tube, (9) substrate/buffer tank, (10) packed bed tank containing enzymes. Figure adapted from Illanes, Andrés, ed. Enzyme biocatalysis: principles and applications. Springer Science & Business Media, 2008.

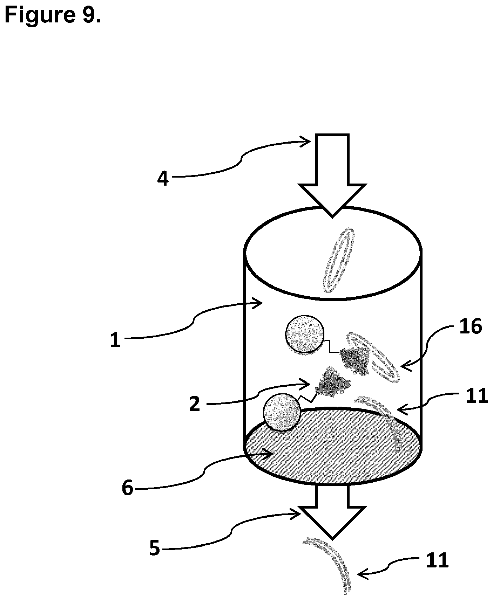

**Figure 9** **Schematic drawing of a continuous batch reactor of the present invention**
   The linearization reactor comprises a reactor vessel (1) and an ultrafiltration device comprising a cellulose membrane with a molecular weight cut-off of 100 to 300 kDa. (6), an inlet (4) and an outlet (5). Immobilized restriction enzymes (2) cleave circular plasmid DNA (16) to yield linearized template DNA (11) for subsequent RNA *in vitro* transcription.

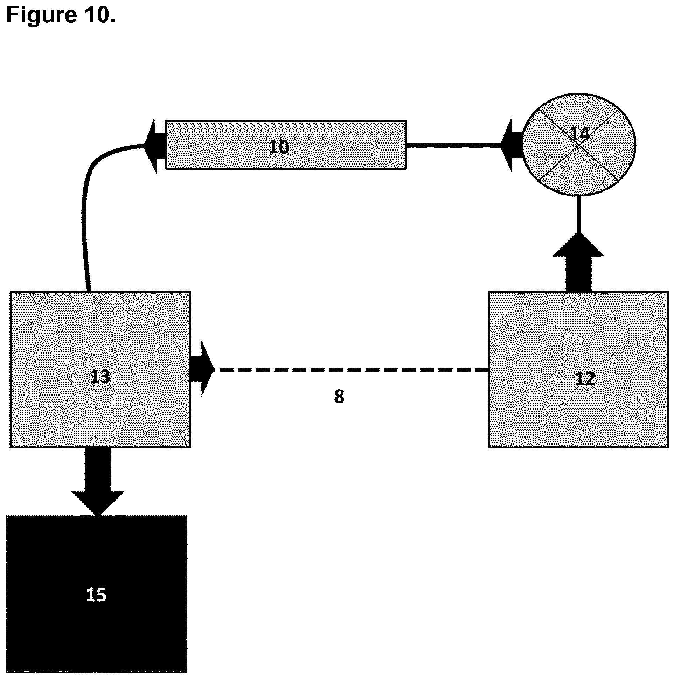

**Figure 10** **Schematic drawing of a continuous packed bed reactor of the present invention**
   A packed bed tank comprising immobilized restriction endonucleases (e.g., Thiol Sepharose 4B HiTrap column) (10) is connected to an input tank (e.g., a feed module) (12) and an output tank (e.g., a capture module) (13). The flow is adjusted using a peristaltic pump (14). Moreover, the output-tank is connected to the input-tank to optionally facilitate continuous flow in a closed system (optional re-circulation pipeline (8), dashed line, only opened if quality control is not complying with the predefined standards, that is e.g. DNA linearization efficiency of >99%). Linearized template DNA product (11) is subjected to quality controls and/or ultrafiltration methods, and then used for RNA *in vitro* transcription in an RNA *in vitro* transcription module (15).

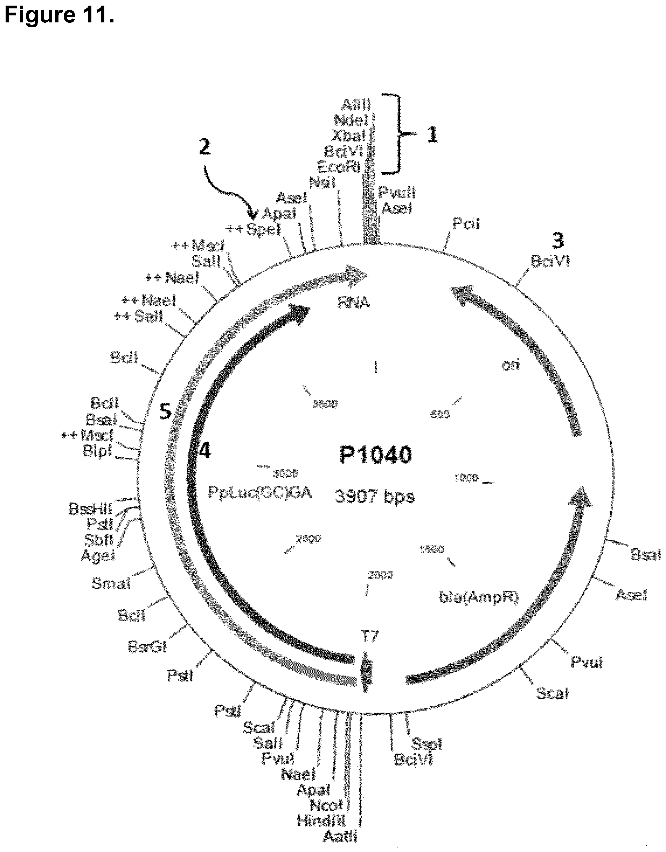

**Figure 11** **Plasmid map of the vector linearized in the examples**
   Plasmid map of P1040 (derived from puc19 plasmid) according to SEQ ID No. 18, which encodes PpLuc. Restriction sites are indicated. Important elements of the pDNA are indicated. (1) Enzymes that can potentially be used to linearize the plasmid, generating a linear template DNA for *in vitro* transcription, resulting in an RNA product with Poly-A, Poly-C and histone stem loop. (2) SpeI can also be used to generate a linear template DNA for *in vitro* transcription, resulting in an RNA product without Poly-A, Poly-C and histone stem loop. (3) BciVI can also be used for linearization, even though that enzyme is not a single cutter. (4) Insert PpLuc, cloned via SpeI and HindIII sites into the vector. (5) PpLuc (GC)GA-A64-C30-histone-stem-loop template for *in vitro* transcription (SEQ ID No. 19).

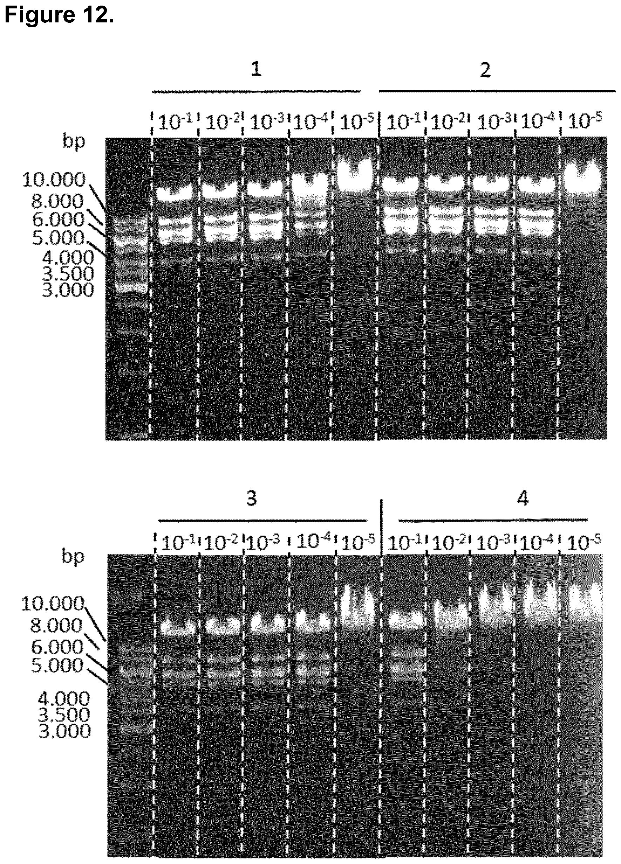

**Figure 12** **Enzymatic activity of EcoRI mutant** enzymes
   The figure shows an enzymatic digestion of lambda DNA using three mutant EcoRI proteins (mut1: 1, mut2: 2; mut3: 3) and a commercially available EcoRI enzyme (4) as control. For each enzyme, 5 different dilution factors are shown (10⁻¹, 10⁻², 10⁻¹, 10⁻⁴, 10⁻⁵). A detailed description of the experiment is provided in the example section, Example 1.

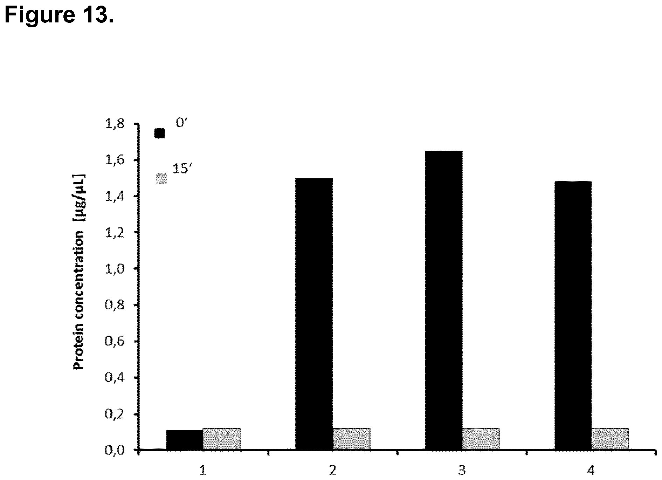

**Figure 13** **Immobilization of EcoRI mutant** enzymes
   The figure shows that recombinant EcoRI mutants (mut1, mut2, and mut3) were efficiently immobilized using Thiopropyl Sepharose 6B (TS) solid supports. Protein concentrations in the supernatant of the immobilization reaction after 0 minutes (black columns) and after 15 minutes (grey columns) are shown. 1: empty TS beads, 2: EcoRI-TS mut1, 3: EcoRI-TS mut2, 4: EcoRI-TS mut3. The figure shows that after 15 minutes, no relevant protein levels could be detected, indicating that the immobilization was successful. A detailed description of the experiment is provided in Example 1.

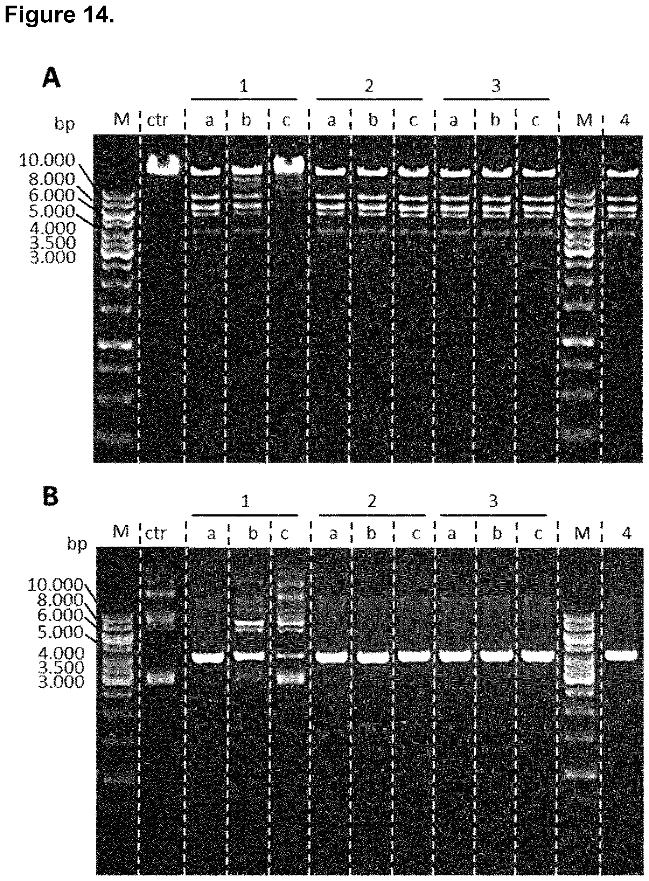

**Figure 14** **Enzymatic activity of immobilized of EcoRI mutant enzymes**
   The figure shows that all three immobilized EcoRI mutants (EcoRI-TS mut1, EcoRI-TS mut2, EcoRI-TS mut3) are functional and enzymatically active. Panel (A) shows a digestion of lambda DNA using three mutant EcoRI-TS mutants (mut1: 1, mut2: 2; mut3: 3) and a commercially available soluble EcoRI enzyme (4) as control. The occurrence of five discrete bands indicates a successful digestion. For each EcoRI-TS mutant, 3 different dilution factors are shown (1 (a), 10⁻¹ (b), 10⁻² (c)). Moreover, a reaction without enzyme (ctr) and respective DNA ladders (M) are shown. Panel (B) shows a digestion of plasmid DNA using three mutant EcoRI-TS mutants (mut1: 1, mut2: 2; mut3: 3) and a commercially available soluble EcoRI enzyme (4) as control. The occurrence of one discrete band indicates a successful linearization. For each EcoRI-TS mutant, 3 different dilution factors are shown (1 (a), 10⁻¹ (b), 10⁻² (c)). Moreover, a reaction without enzyme (ctr) and respective DNA ladders (M) are shown. A detailed description of the experiment is provided in Example 1.

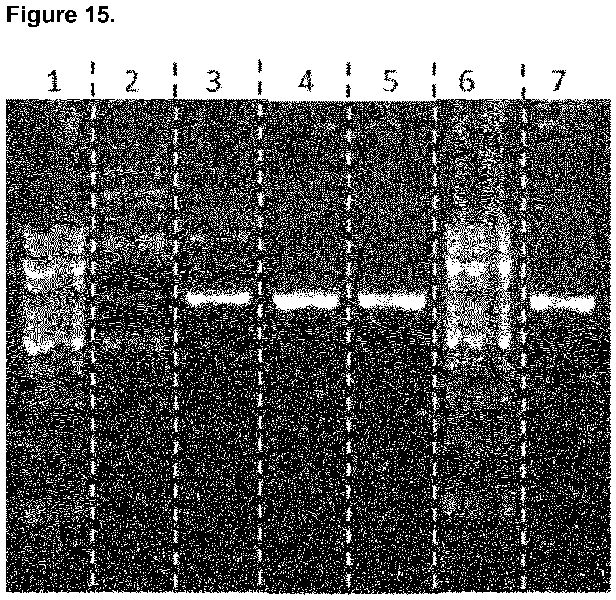

**Figure 15** **Plasmid digestions using a prototype linearization reactor**
   EcoRI-TS beads were packed into the prototype linearization reactor and circular plasmid DNA was introduced in the reactor via a pump. The flow-through was collected and plasmid DNA linearization was analyzed on an agarose gel. The occurrence of one discrete band indicates a successful linearization. Lane 1: 1kb DNA ladder; lane 2: undigested plasmid DNA; lane 3: EcoRI-TS mut1; lane 4: EcoRI-TS mut2; lane 5: EcoRI-TS mut3; lane 6: 1kb DNA ladder; lane 7: soluble EcoRI positive control. A detailed description of the experiment is provided in Example 2.

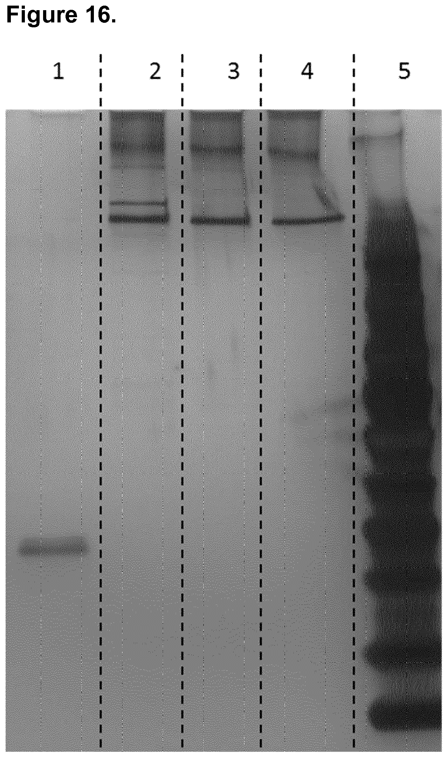

**Figure 16** **Analysis of flow-through samples of the linearization reactor**
   To assess the stability of EcoRI-TS beads, the flow-through samples of the linearization reactor were analysed via SDS-PAGE and subsequent silver staining to detect potential protein leakage and DNA. (1) position of the EcoRI band (positive control); (2) flow-through sample of EcoRI-TS mut1; (3) flow-through sample of EcoRI-TS mut2; (4) flow-through sample of EcoRI-TS mut3; (5) pre-stained protein ladder. The results show no detectable protein leakage in the reactor flow-through samples (compare band in lane 1 with the other lanes). Only DNA bands are visible in lanes 2-4 (bands on the top). A detailed description of the experiment is provided in the example section, Example 2.

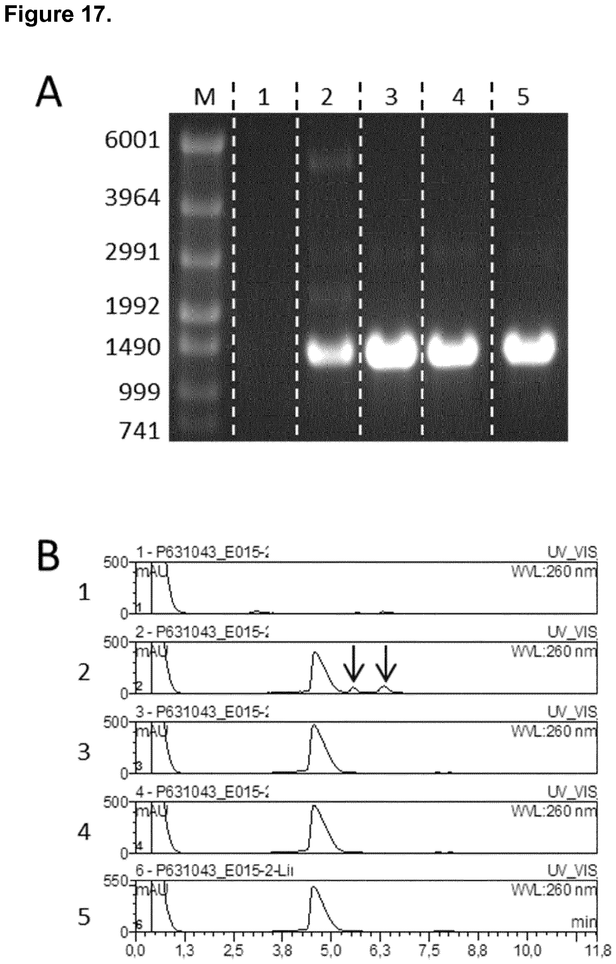

**Figure 17** **Analysis *of in vitro* transcribed RNA**
   The linearized DNA obtained from the linearization reactor was used as a template for RNA *in vitro* transcription. (A) shows an agarose gel electrophoresis of the *in vitro* transcribed RNA. (B) shows an analytic HPLC profile of the produced RNA samples. DNA obtained from the linearization reactor and linearized with EcoRI-TS mut1 (2), EcoRI-TS mut2 (3), or EcoRI-TS mut3 (4) was used for RNA *in vitro* transcription. (1) shows a negative control, (5) shows a positive control. A detailed description of the experiment is provided in the example section, Example 2.

### Definitions

For the sake of clarity and readability the following definitions are provided. Any technical feature mentioned in these definitions may be read on each and every embodiment of the invention. Additional definitions and explanations may be specifically provided in the context of these embodiments.

Enzyme: Enzymes are catalytically active biomolecules that perform biochemical reactions such as DNA-dependent RNA transcription (e.g., RNA polymerases), or double-stranded DNA digestion (e.g., restriction endonucleases). Enzymes are typically composed of amino acids and/or RNA (ribozymes, snRNA).

Restriction endonucleases: Restriction endonucleases are a class of enzymes that occur naturally in bacteria and in some viruses. Restriction endonucleases can be used in the laboratory e.g. to cleave DNA molecules into smaller fragments for molecular cloning and gene characterization.

A restriction enzyme (or restriction endonuclease) is an enzyme that cuts DNA at or near specific recognition nucleotide sequences known as restriction sites. Restriction endonucleases are commonly classified into four types, which differ in their structure and whether they cut their DNA substrate at their recognition site, or if the recognition and cleavage sites are separate from one another. To cut DNA, all restriction enzymes make two incisions, one per each sugar-phosphate backbone (i.e. each strand) of the DNA double helix.

The restriction endonucleases are categorized into four groups (Types I, II, III, and IV) based on their composition and enzyme cofactor requirements, the nature of their target sequence, and the position of their DNA cleavage site relative to the target sequence. All types of enzymes recognize specific short DNA sequences and carry out the cleavage of DNA, yielding specific fragments with terminal 5'-phosphates. Restriction endonucleases recognize and bind particular sequences of nucleotides (the 'recognition site') on DNA molecules. Once bound, they cleave the molecule within the recognition site (e.g., BamHI), at one side of (e.g., SapI), or at both sides (e.g., TspRI) of the recognition sequence.

Type I enzymes (EC 3.1.21.3) cleave at sites remote from their recognition site, require both ATP and S-adenosyl-L-methionine to function and are multifunctional proteins with both restriction and methylase (EC 2.1.1.72) activities.

Type II enzymes (EC 3.1.21.4) cleave within or at short specific distances from their recognition site. Most of them require magnesium as a cofactor. They function only as restriction enzymes and do not have methylase activity.

Type III enzymes (EC 3.1.21.5) cleave at sites a short distance from their recognition site, require ATP (but do not hydrolyse it) and exist as part of a complex with a modification methylase (EC 2.1.1.72). S-adenosyl-L-methionine stimulates the reaction, but is not required for enzyme activity.

Type IV enzymes target modified DNA, e.g. methylated, hydroxymethylated and glucosyl-hydroxymethylated DNA.

Different restriction endonucleases have affinity for different recognition sequences. The commonly used type II enzymes are homodimers which recognize palindromic sites. Depending on particular features several subtypes have been classified. All structures of type II restriction enzymes show a common structural core comprising four β-strands and one α-helix. Furthermore, two families of enzymes can be distinguished which are structurally very similar (EcoRI-like enzymes and EcoRV-like enzymes). With the determination of protein crystal structures it became clear that all restriction endonuclease structures known so far, including orthodox restriction enzymes producing sticky ends with a 5'-overhang (e.g. BamHI, BglII, EcoRI, MunI, BsoBI), sticky ends with a 3'-overhang (e.g. BglI) or blunt ends (e.g. EcoRV, PvuII), as well as members of the type IIS (e.g. FokI), type IIE (e.g. NaeI) and type IIF (e.g. Cfr10I, NgoMIV) subdivisions, have a very similar core. Moreover, the topologies of selected type II restriction endonucleases show similarities of architecture and of functionally important regions such as catalytic core, DNA binding domains and dimerization/multimerization domains (Pingoud and Jeltsch (2001) Nucl. Acids Res. 29(18): 3705-3727).

Typical type II restriction enzymes differ from type I restriction enzymes in several ways. They are usually a homodimer, with recognition sites are usually undivided and palindromic and 4-8 nucleotides in length. They recognize and cleave DNA at the same site, and they do not use ATP or S-Adenosyl methionine for their activity, but usually require only Mg²⁺ as a cofactor. These are the most commonly available and used restriction enzymes. In the 1990s and early 2000s, new enzymes from this family were discovered that did not follow all the classical criteria of this enzyme class, and new subfamily nomenclature was developed to divide this large family into subcategories based on deviations from typical characteristics of type II enzymes. These subgroups are defined using a letter suffix (Pingoud and Jeltsch (2001) Nucl. Acids Res. 29(18): 3705-3727).

Type IIB restriction enzymes (e.g. BcgI and BplI) are multimers, containing more than one subunit. They cleave DNA on both sides of their recognition site to cut out the recognition site. They require both S-Adenosyl methionine and Mg²⁺ cofactors. Type IIE restriction endonucleases (e.g. NaeI) cleave DNA following interaction with two copies of their recognition sequence. One recognition site acts as the target for cleavage, while the other acts as an allosteric effector that speeds up or improves the efficiency of enzyme cleavage. Similar to type IIE enzymes, type IIF restriction endonucleases (e.g. NgoMIV) interact with two copies of their recognition sequence but cleave both sequences at the same time. Type IIG restriction endonucleases (Eco57I) do have a single subunit, like classical Type II restriction enzymes, but require the cofactor S-Adenosyl methionine to be active. Type IIM restriction endonucleases, such as DpnI, are able to recognize and cut methylated DNA. Type IIP restriction endonucleases such as EcoRI and HindIII recognize, and cleave within, symmetric DNA sequences, thereby generating palindromic ends. Type IIS restriction endonucleases (e.g. FokI, BciVI) cleave DNA at a defined distance from their non-palindromic asymmetric recognition sites. These enzymes may function as dimers but some may also function as monomers (Armalyte, Elena, et al. Journal of Biological Chemistry 280.50 (2005): 41584-41594). Similarly, Type IIT restriction enzymes (e.g., Bpu10I and BslI) are composed of two different subunits. Some recognize palindromic sequences while others have asymmetric recognition sites (Pingoud and Jeltsch (2001) Nucl. Acids Res. 29(18): 3705-3727). The use of Type IIP and Type IIS restriction endonucleases is particularly preferred in the present invention.

Restriction site: A restriction site, also termed restriction enzyme recognition site or recognition sequence, is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising its specific restriction site within the recognition site or close to it. In a double-stranded nucleotide sequence, such as a double-stranded DNA sequence, the restriction enzyme typically cuts both strands of the nucleotide sequence.

Protein: A protein typically comprises one or more peptides or polypeptides. A protein is typically folded into 3-dimensional form, which may be required for the protein to exert its biological function. The sequence of a protein or peptide is typically understood to be the order, i.e. the succession of its amino acids.

Recombinant protein: The term 'recombinant protein' refers to proteins that have been produced in a heterologous system, that is, in an organism that naturally does not produce such a protein, or a variant of such a protein. It also refers to a protein which is expressed from a typical expression vector in an expression host which also naturally expresses this protein, but naturally expresses it in smaller amounts. Said recombinant protein may also comprise elements necessary for the purification of the protein, e.g. purification tags, e.g. oligo histidine tags (HIS-tags). Some other examples of purification tags are SEQ ID Nos. 60-80.

Expression host: An expression host denotes an organism which is used for recombinant protein production, e.g., for the production of recombinant restriction endonucleases. General expression hosts are bacteria, such as *E. coli*, yeasts, such as *Saccharomyces cerevisiae* or *Pichia pastoris,* insect cells or also mammalian cells, such as human cells.

Functional group: The term is to be understood according to the skilled person's general understanding in the art and denotes a chemical moiety which is present on a molecule, in particular on the solid support, and which may participate in a covalent or non-covalent bond to another chemical molecule, such as a restriction endonuclease. Exemplary functional groups in the context of the invention include thiol, haloacetyl, pyridyl disulfide, epoxy and a maleimide group.

Plasmid DNA: The term 'plasmid DNA' refers to a circular nucleic acid molecule, preferably to an artificial nucleic acid molecule. A plasmid DNA in the context of the present invention is suitable for incorporating or harboring a desired nucleic acid sequence, such as a nucleic acid sequence comprising an RNA coding sequence and/or an open reading frame encoding at least one peptide or polypeptide. Such plasmid DNA constructs may be storage vectors, expression vectors, cloning vectors, transfer vectors etc. A storage vector is a vector which allows the convenient storage of a nucleic acid molecule, for example, of an RNA molecule. Thus, the plasmid DNA may comprise a sequence corresponding to (coding for), e.g., a desired RNA sequence or a part thereof, such as a sequence corresponding to the open reading frame and the 5'-and/or 3'UTR of an mRNA. An expression vector may be used for production of expression products such as RNA, e.g. mRNA in a process called *in vitro* transcription. For example, an expression vector may comprise sequences needed for *in vitro* transcription of a sequence stretch of the vector, such as a promoter sequence, e.g. an RNA promoter sequence, preferably T3, T7 or SP6 RNA promotor sequences. A cloning vector is typically a vector that contains a cloning site, which may be used to incorporate nucleic acid sequences (insert) into the vector. A cloning vector may be, e.g., a plasmid vector or a bacteriophage vector. A transfer vector may be a vector which is suitable for transferring nucleic acid molecules into cells or organisms, for example, viral vectors. Preferably, a plasmid DNA vector within the meaning of the present invention comprises a multiple cloning site, an RNA promoter sequence, optionally a selection marker, such as an antibiotic resistance factor, and a sequence suitable for multiplication of the vector, such as an origin of replication. The DNA, which forms the template of the RNA to be transcribed *in vitro,* may be prepared by fermentative proliferation and subsequent isolation as part of a plasmid which can be replicated in bacteria. Particularly preferred in the context of the present invention are plasmid DNA vectors, or expression vectors, comprising promoters for DNA-dependent RNA polymerases such as T3, T7 and Sp6. As plasmid backbone, particularly preferred are pUC19 and pBR322.

Linear template DNA: The linear template DNA is obtained by contacting the plasmid DNA with a restriction enzyme under suitable conditions so that the restriction enzyme cuts the plasmid DNA at its recognition site(s) and disrupts the circular plasmid structure. Hence, the linear template DNA comprises a free 5' end and a free 3' end which are not linked to each other. If the plasmid DNA contains only one recognition site for the restriction enzyme, the linear template DNA has the same number of nucleotides as the plasmid DNA. If the plasmid DNA contains more than one recognition site for the restriction enzyme, the linear template DNA has a smaller number of nucleotides than the plasmid DNA. The linear template DNA is then the fragment of the plasmid DNA which contains the elements necessary for *in vitro* transcription, that is a promotor element for RNA transcription and the template DNA element. The open reading frame of the linear template DNA determines the sequence of the transcribed RNA by the rules of base-pairing.

Modified nucleoside triphosphate: The term "modified nucleoside triphosphate" as used herein refers to chemical modifications comprising backbone modifications as well as sugar modifications or base modifications. These modified nucleoside triphosphates are also termed herein as (nucleotide) analogs, modified nucleosides/nucleotides or nucleotide/nucleoside modifications.

In this context, the modified nucleoside triphosphates as defined herein are nucleotide analogs/modifications, e.g. backbone modifications, sugar modifications or base modifications. A backbone modification in connection with the present invention is a modification, in which phosphates of the backbone of the nucleotides are chemically modified. A sugar modification in connection with the present invention is a chemical modification of the sugar of the nucleotides. Furthermore, a base modification in connection with the present invention is a chemical modification of the base moiety of the nucleotides. In this context nucleotide analogs or modifications are preferably selected from nucleotide analogs which are applicable for transcription and/or translation. In the context of the present invention, modified nucleotides as defined herein may be used in RNA *in vitro* transcription reactions.

Sugar Modifications: The modified nucleosides and nucleotides, which may be used in the context of the present invention, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), - 0(CH2CH2o)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified nucleotide can include nucleotides containing, for instance, arabinose as the sugar.

Backbone Modifications: The phosphate backbone may further be modified in the modified nucleosides and nucleotides. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

Base Modifications: The modified nucleosides and nucleotides, which may be used in the present invention, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogs/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5 '-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-Iodo-2'-deoxycytidine-5'-triphosphate, 5-iodouridine-5'-triphosphate, 5-Iodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio- 1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio- 1-methyl-pseudoisocytidine, 4-thio- 1 -methyl- 1 -deaza-pseudoisocytidine, 1 -methyl- 1 -deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group.

In specific embodiments, a modified nucleoside is 5'-0-(1-Thiophosphate)-Adenosine, 5'-0-(1-Thiophosphate)-Cytidine, 5'-0-(1-Thiophosphate)-Guanosine, 5'-0-(1-Thiophosphate)-Uridine or 5'-0-(1-Thiophosphate)-Pseudouridine.

In further specific embodiments the modified nucleotides include nucleoside modifications selected from 6-aza-cytidine, 2-thio-cytidine, α-thio-cytidine, Pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, Pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, α-thio-adenosine, 8-azido-adenosine, 7-deaza-adenosine.

Further modified nucleotides have been described previously (see, e.g., WO 2013/052523).

5'-cap: A 5 '-cap is an entity, typically a modified nucleotide entity, which generally "caps" the 5'-end of a mature mRNA and increases its stability. A 5'-cap may typically be formed by a modified nucleotide (cap analog), particularly by a derivative of a guanine nucleotide. Preferably, the 5'-cap is linked to the 5'-terminus of the RNA via a 5'-5'-triphosphate linkage. A 5'-cap may be methylated, e.g. m7GpppN (e.g. m7G(5')ppp(5')G (m7G)), wherein N is the terminal 5' nucleotide of the nucleic acid carrying the 5'-cap, typically the 5'-end of an RNA, m7 is a methyl group attached to position 7 of the guanine (G) and ppp is the triphosphate. Further examples of 5'cap structures include glyceryl, inverted deoxy abasic residue (moiety), 4',5' methylene nucleotide, 1 -(beta-D- erythrofuranosyl) nucleotide, 4'-thio nucleotide, carbocyclic nucleotide, 1 ,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3 '-3 '-inverted nucleotide moiety, 3'-3'-inverted abasic moiety, 3'-2'-inverted nucleotide moiety, 3'-2 '-inverted abasic moiety, 1 ,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. Further modified 5'-CAP structures which may be used in the context of the present invention are CAP1 (additional methylation of the ribose of the adjacent nucleotide of m7GpppN), CAP2 (additional methylation of the ribose of the 2nd nucleotide downstream of the m7GpppN), CAP3 (additional methylation of the ribose of the 3rd nucleotide downstream of the m7GpppN), CAP4 (additional methylation of the ribose of the 4th nucleotide downstream of the m7GpppN), ARCA (anti-reverse CAP analogue), modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

Poly(A) sequence: A poly(A) sequence, also called poly(A) tail or 3'-poly(A) tail, is typically understood to be a sequence of adenine nucleotides, e.g., of up to about 400 adenine nucleotides, e.g. from about 20 to about 400, preferably from about 50 to about 400, more preferably from about 50 to about 300, even more preferably from about 50 to about 250, most preferably from about 60 to about 250 adenine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription from the vector or from the linear template DNA generated from this vector. Additionally, a poly(A)sequence may be generated enzymatically using a poly(A)polymerase.

RNA, mRNA: RNA is the usual abbreviation for ribonucleic acid. It is a nucleic acid molecule, i.e. a polymer consisting of nucleotide monomers. These nucleotides are usually adenosine-monophosphate, uridine-monophosphate, guanosine-monophosphate and cytidine-monophosphate monomers or analogs thereof, which are connected to each other along a so-called backbone. The backbone is formed by phosphodiester bonds between the sugar, i.e. ribose, of a first and a phosphate moiety of a second, adjacent monomer. The specific order of the monomers, i.e. the order of the bases linked to the sugar/phosphate-backbone, is called the RNA-sequence. Usually RNA may be obtainable by transcription of a DNA-sequence, e.g., inside a cell. In eukaryotic cells, transcription is typically performed inside the nucleus or the mitochondria. *In vivo*, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, optionally a 5'UTR, an open reading frame, optionally a 3'UTR and a poly(A) sequence.

In addition to messenger RNA, several non-coding types of RNA exist which may be involved in regulation of transcription and/or translation, and immunostimulation. The term "RNA" further encompasses RNA molecules, such as viral RNA, retroviral RNA and replicon RNA, small interfering RNA (siRNA), antisense RNA, CRISPR/Cas9 guide RNA, ribozymes, aptamers, riboswitches, immunostimulating RNA (isRNA), transfer RNA (tRNA), ribosomal RNA (rRNA), small nuclear RNA (snRNA), small nucleolar RNA (snoRNA), microRNA (miRNA), and Piwi-interacting RNA (piRNA) etc.

*In vitro* transcribed RNA: An *in vitro* transcribed RNA is an RNA molecule that has been synthesized from a DNA template, commonly a linearized and purified plasmid DNA template, a PCR product, or an oligonucleotide. RNA synthesis occurs in a cell free (*"in vitro"*) assay catalyzed by DNA-dependent RNA polymerases. Particular examples of DNA-dependent RNA polymerases are the T7, T3, and SP6 RNA polymerases. All RNA molecules as defined herein may be synthesized by *in vitro* transcription.

Native amino acid sequence: The term is to be understood according to the skilled person's general understanding in the art and denotes the amino acid sequence in the form of its occurrence in nature without any mutation or amino acid amendment by man, also called "wild-type sequence". "Native restriction endonuclease" denotes a restriction endonuclease having the amino acid sequence as it occurs in nature. The presence or absence of an N-terminal methionine, which depends on the expression host used, usually does not change the status of a protein being considered as having its natural or native sequence.

Mutated, mutant: The term is to be understood according to the skilled person's general understanding in the art. An amino acid sequence is called "mutated" if it contains at least one additional, deleted or exchanged amino acid in its amino acid sequence in comparison to its natural or native amino acid sequence, i.e. if it contains an amino acid mutation. Mutated proteins are also called mutants. "Mutated to comprise only one cysteine residue" denotes that the amino acid sequence has been changed on the amino acid level so that the amino acid sequence contains only one cysteine residue. This may include that a cysteine residue was introduced via site-directed mutagenesis into a protein not naturally comprising a cysteine residue or that one or more cysteine residues occurring in the native protein were removed, leaving only one cysteine residue in the amino acid sequence. The term "mutant" has to be understood as a protein that differs from their corresponding wild-type sequence in at least one amino acid. The term "mutant" is also used herein if wild type proteins are C-terminally and/or N-terminally extended (e.g., via a linker element (e.g. according to SEQ ID Nos. 35-59) and/or via a purification tag (e.g. according to SEQ ID Nos. 60-80). In the context of the invention, restriction endonucleases according to e.g. SEQ ID Nos. 4-17 and 20-34 are considered as mutant proteins.

Sequence of a nucleic acid molecule/nucleic acid sequence: The sequence of a nucleic acid molecule is typically understood to be the particular and individual order, i.e. the succession, of its nucleotides.

Sequence of a protein or peptide/amino acid sequence: The sequence of a protein or peptide is typically understood to be the order, i.e. the succession, of the amino acids constituting the protein.

Sequence identity: Two or more sequences are identical, if they exhibit the same length and order of nucleotides or amino acids. The percentage of identity typically describes the extent to which two sequences are identical, i.e. it typically describes the percentage of nucleotides that correspond in their sequence position to identical nucleotides of a reference sequence. For the determination of the degree of identity, the sequences to be compared are considered to have the same length, i.e. the length of the longest sequence of the sequences to be compared. This means that a first sequence consisting of 8 nucleotides/amino acids is 80% identical to a second sequence consisting of 10 nucleotides/amino acids comprising the first sequence. In other words, in the context of the present invention, identity of sequences preferably relates to the percentage of nucleotides/amino acids of a sequence, which have the same position in two or more sequences having the same length. Gaps are usually regarded as non-identical positions, irrespective of their actual position in an alignment.

The sequence identity may be determined using a series of programs, which are based on various algorithms, such as BLASTN, ScanProsite, the laser gene software, etc. As an alternative, the BLAST program package of the National Center for Biotechnology Information may be used with the default parameters. In addition, the program Sequencher (Gene Codes Corp., Ann Arbor, MI, USA) using the "dirtydata"-algorithm for sequence comparisons may be employed.

The identity between two protein or nucleic acid sequences is defined as the identity calculated with the program *Needle* in the version available in April 2011. *Needle* is part of the freely available program package EMBOSS, which can be downloaded from the corresponding website. The standard parameters used are gapopen 10.0 ("gap open penalty"), gapextend 0.5 ("gap extension penalty"), datafile EONAFULL (matrix) in the case of nucleic acids.

Newly introduced amino acids: "Newly introduced amino acids" denote amino acids which are newly introduced into an amino acid sequence in comparison to a native amino acid sequence. The native amino acid sequence is usually changed by mutagenesis in order to have a certain amino acid side chain at a desired position within the amino acid sequence. In the present invention, in particular the amino acid cysteine is newly introduced into the amino acid sequence at one or more desired positions since the side chain of cysteine being a thiol group allows for easy and straightforward immobilization of the restriction endonuclease onto a solid support via formation of a disulfide bridge or thioether bond, depending on the functional group of the solid support. The newly introduced amino acid may be introduced into the native or a mutated amino acid sequence between two amino acid residues already existing in the native or mutated amino acid sequence or may be introduced instead of an amino acid residue already existing in the native or mutated amino acid sequence, i.e. an existing amino acid is exchanged for the newly introduced amino acid sequence. In addition, certain amino acids or amino acid stretches (or tags) suitable for the purification of the recombinant enzyme may be introduced, e.g. oligo-histidine tags (HIS tag), FLAG tags, etc.

DNA: DNA is the usual abbreviation for deoxyribonucleic acid. It is a nucleic acid molecule, i.e. a polymer consisting of nucleotide monomers. These nucleotides are usually deoxy-adenosine-monophosphate, deoxy-thymidine-monophosphate, deoxy-guanosine-monophosphate and deoxy-cytidine-monophosphate monomers or analogs thereof which are - by themselves - composed of a sugar moiety (deoxyribose), a base moiety and a phosphate moiety, and polymerize by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single-stranded or double-stranded. In the double stranded form, the nucleotides of the first strand typically hybridize with the nucleotides of the second strand, e.g. by A/T-base-pairing and G/C-base-pairing.

Cloning site: A cloning site is typically understood to be a segment of a nucleic acid molecule, which is suitable for insertion of a nucleic acid molecule, e.g., a nucleic acid sequence molecule comprising an open reading frame. The nucleic acid molecule may be inserted by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG), at its 5'-end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "protein coding region".

*In vitro* transcription: The term *"in vitro* transcription" or "RNA *in vitro* transcription" relates to a process wherein RNA is synthesized in a cell-free system (*in vitro*). DNA, particularly plasmid DNA, is used as template for the generation of RNA transcripts. RNA may be obtained by DNA-dependent *in vitro* transcription of an appropriate DNA template, which according to the present invention is preferably a linearized plasmid DNA template. The promoter for controlling *in vitro* transcription can be any promoter for any DNA dependent RNA polymerase. Particular examples of DNA dependent RNA polymerases are the T7, T3, and SP6 RNA polymerases. A DNA template for *in vitro* RNA transcription may be obtained by cloning of a nucleic acid, in particular cDNA corresponding to the respective RNA to be *in vitro* transcribed, and introducing it into an appropriate vector for *in vitro* transcription, for example into plasmid DNA. Within the meaning of the present invention the DNA template is linearized by the method of the invention, before it is transcribed *in vitro.* The cDNA may be obtained by reverse transcription of mRNA or chemical synthesis. Moreover, the DNA template for *in vitro* RNA synthesis may also be obtained by gene synthesis.

Methods for *in vitro* transcription are known in the art (Geall et al. (2013) Semin. Immunol. 25(2): 152-159; Brunelle et al. (2013) Methods Enzymol. 530:101-14).

Reagents used in said method typically include:
1) a linearized DNA template (as defined above) with a promoter sequence that has a high binding affinity for its respective RNA polymerase such as bacteriophage-encoded RNA polymerases;
2) ribonucleoside triphosphates (NTPs) for the four bases (adenine, cytosine, guanine and uracil);
3) optionally a cap analog as defined above (e.g. m7G(5')ppp(5')G (m7G));
4) a DNA-dependent RNA polymerase capable of binding to the promoter sequence within the linearized DNA template (e.g. T7, T3 or SP6 RNA polymerase);
5) optionally a ribonuclease (RNase) inhibitor to inactivate any contaminating RNase;
6) optionally a pyrophosphatase to degrade pyrophosphate, which may inhibit transcription;
7) MgCl₂, which supplies Mg²⁺ ions as a co-factor for the polymerase;
8) a buffer to maintain a suitable pH value, which can also contain antioxidants (e.g. DTT) and polyamines such as spermidine at optimal concentrations.

According to a preferred embodiment, the (transcription) buffer is selected from the group consisting of 4-(2-hydroxyethyl)-1-piperazineethanesulfonic acid (HEPES) and tris(hydroxymethyl)aminomethane (Tris). Preferably the buffer is used at a concentration from 10 to 100 mM, 10 to 75 mM, 10 to 50 mM, 10 to 40 mM, 10 to 30 mM or 10 to 20 mM. The pH value of the buffer can be adjusted with, for example, NaOH, KOH or HCl. Preferably the buffer has a pH value from 6 to 8.5, from 6.5 to 8.0, from 7.0 to 7.5, even more preferred 7.5. Most preferred is a buffer selected from the group consisting of 80 mM HEPES/KOH, pH 7.5 and 40 mM Tris/HCl, pH 7.5.

According to a preferred embodiment of the invention, the RNA polymerase is selected from the group consisting of T3, T7 and SP6 RNA polymerase. Preferably, the concentration of the RNA polymerase is from about 1 to 100 nM, 1 to 90 nM, 1 to 80 nM, 1 to 70 nM, 1 to 60 nM, 1 to 50 nM, 1 to 40 nM, 1 to 30 nM, 1 to 20 nM, or about 1 to 10 nM. Even more preferred, the concentration of the RNA polymerase is from about 10 to 50 nM, 20 to 50 nM, or 30 to 50 nM. Most preferred is a RNA polymerase concentration of about 40 nM. The person skilled in the art will understand that the choice of the RNA polymerase concentration is influenced by the concentration of the DNA template. Therefore, in specific embodiments the concentration of the RNA polymerase is between 1 and 1000 U/µg template DNA, preferably between 10 and 100 U/µg DNA, particularly if plasmid DNA is used as template DNA.

According to a preferred embodiment of the invention, the concentration of the linear DNA template is in a range from about 1 to 50 nM, 1 to 40 nM, 1 to 30 nM, 1 to 20 nM, or about 1 to 10 nM. Even more preferred the concentration of the DNA template is from about 10 to 30 nM. Most preferred the concentration of the DNA template is about 20 nM. In case plasmid DNA is used as DNA template, the concentration of the DNA template is preferably between 1 to 100 µg/ml, particularly in a concentration of about 50 µg/ml.

According to a preferred embodiment of the invention, the *in vitro* transcription is performed in the presence of pyrophosphatase. Preferably, the concentration of the pyrophosphatase is from about 1 to 20 units/ml, 1 to 15 units/ml, 1 to 10 units/ml, 1 to 5 units/ml, or 1 to 2.5 units/ml. Even more preferred the concentration of the pyrophosphatase is about 5 unit/ml.

According to a preferred embodiment of the invention, the *in vitro* transcription reaction mixture comprises Mg²⁺ ions. Preferably, the Mg²⁺ ions are provided in the form of MgCl₂ or Mg(OAc)₂. Preferably, the initial free Mg²⁺ concentration is from about 1 to 100 mM, 1 to 75 mM, 1 to 50 mM, 1 to 25 mM, or 1 to 10 mM. Even more preferred the initial free Mg²⁺ concentration is from about 10 to 30 mM or about 15 to 25 mM. Most preferred is an initial free Mg²⁺ concentration of about 24 mM. The person skilled in the art will understand that the choice of the Mg²⁺ concentration is influenced by the initial total NTP concentration.

According to a preferred embodiment of the invention, the *in vitro* transcription reaction mixture comprises a reducing agent (antioxidant) to keep the RNA polymerase in its active state. Preferably, the reducing agent is selected from the group consisting of dithiothreitol (DTT), dithioerythritol (DTE), Tris(2-carboxyethyl)phosphine (TCEP) and β-mercaptoethanol. Preferably the concentration of the reducing reagent is from about 1 to 50 mM, 1 to 40 mM, 1 to 30 mM, or 1 to 20 mM, or 1 to 10 mM. Even more preferred the concentration of the reducing reagent is from 10 to 50 mM or 20 to 40 mM. Most preferred is a concentration of 40 mM of DTT.

According to a preferred embodiment of the invention, the *in vitro* transcription reaction mixture comprises a polyamine. Preferably, the polyamine is selected from the group consisting of spermine and spermidine. Preferably the concentration of the polyamine is from about 1 to 25 mM, 1 to 20 mM, 1 to 15 mM, 1 to 10 mM, 1 to 5 mM, or about 1 to 2.5 mM. Even more preferred the concentration of the polyamine is about 2 mM. Most preferred is a concentration of 2 mM of spermidine.

According to a preferred embodiment of the invention, the *in vitro* transcription reaction mixture comprises a ribonuclease inhibitor. Preferably, the concentration of the ribonuclease inhibitor is from about 1 to 500 units/ml, 1 to 400 units/ml, 1 to 300 units/ml, 1 to 200 units/ml, or 1 to 100 units/ml. Even more preferred the concentration of the ribonuclease inhibitor is about 200 units/ml.

According to a preferred embodiment of the invention, the total NTP concentration in the *in vitro* transcription reaction mixture is between 1 and 100 mM, preferably between 10 and 50 mM, and most preferably between 10 and 20 mM.

According to the invention, the term total nucleotide concentration means the total concentration of NTPs, e.g. the sum of the concentrations of ATP, GTP, CTP, UTP, and/or cap analog present initially in the *in vitro* transcription when the various components of the reaction have been assembled in the final volume for carrying out the *in vitro* transcription reaction. Naturally, as the reaction proceeds, the nucleotides will be incorporated into the RNA molecule and consequently the total nucleotide concentration will be progressively reduced from its initial value.

In this context it is particularly preferred that the single nucleotides are provided in a concentration between 0.1 and 10 mM, preferably between 1 and 5 mM and most preferably in a concentration of 4 mM.

In case a 5' cap as defined above has to be generated at the 5'-end of the RNA, the in vitro transcription reaction mixture further comprises a cap analog. In this context the concentration of GTP is preferably reduced compared to the other nucleotides (ATP, CTP and UTP). Preferably the cap analog is added with an initial concentration in the range of about 1 to 20 mM, 1 to 17.5 mM, 1 to 15 mM, 1 to 12.5 mM, 1 to 10 mM, 1 to 7.5 mM. Most preferably the cap analog is added in a concentration of 5.8 mM and the GTP concentration is reduced to a concentration of 1.45 mM whereas ATP, CTP and UTP are comprised in the reaction in a concentration of 4 mM each.

The ribonucleoside triphosphates (NTPs) GTP, ATP, CTP and UTP or analogs thereof may be provided with a monovalent or divalent cation as counterion. Preferably the monovalent cation is selected from the group consisting of Li⁺, Na⁺ , K⁺ , NH4⁺ or tris(hydroxymethyl)-aminomethane (Tris). Preferably, the divalent cation is selected from the group consisting of Mg²⁺, Ba²⁺ and Mn²⁺.

According to a preferred embodiment of the invention, a part or all of at least one ribonucleoside triphosphate in the *in vitro* transcription reaction mixture is replaced with a modified nucleoside triphosphate (as defined herein). In a preferred embodiment of the invention, said modified nucleoside triphosphate is selected from the group consisting of pseudouridine-5'-triphosphate, 1-methylpseudouridine-5'-triphosphate, 2-thiouridine-5'-triphosphate, 4-thiouridine-5'-triphosphate and 5-methylcytidine-5 '-triphosphate.

After *in vitro* transcription has occurred, the RNA product is subjected to purification methods. In this context any purification method may be used (e.g. DNA template digest, phenol-chloroform extraction, LiCl precipitation, HPLC, etc.).

Immunostimulatory RNA: An immunostimulatory RNA (isRNA) in the context of the invention may typically be a RNA that is able to induce an innate immune response. An isRNA usually does not have an open reading frame and thus does not provide a peptide-antigen, but elicits an innate immune response, e.g. by binding to pathogen-associated molecular patterns (PAMP) receptors (e.g. Toll-like-receptor (TLR) or other intracellular RNA sensors (e.g. RIG-I, MDA-5 or PKR).

Immobilization: The term immobilization relates to the attachment of a molecule such as a protein and preferably a restriction endonuclease to an inert, insoluble material which is also called solid support.

Linker: A linker is typically a short amino acid sequence which links two domains or amino acids, but does not have a biological function itself. Linkers which can be used in the invention may comprise between 1 and 20 amino acids, preferably between 3 and 18 amino acids, more preferably between 5 and 15 amino acids and most preferably between 8 and 12 amino acids. The linker molecule typically consists of small amino acid residues such as glycine and serine. Examples of suitable linker sequences in the context of the invention are SEQ ID Nos. 35-59. A particularly preferred linker sequence has the sequence according to SEQ ID No. 49.

Enzyme reactor: An "enzyme reactor", also called "linearization reactor", may be any enzyme reactor comprising a vessel comprising a restriction endonuclease of the present disclosure immobilized onto a solid support. The enzyme reactor further comprises the other components of the linearization reaction, such as water, buffer components and salts and is suitable for performing the linearization reaction in that the desired reaction conditions, e.g., temperature, reaction component concentration, salt and buffer concentration, pressure and pH value for the linearization reaction can be adjusted. The enzyme reactor further allows for the introduction and removal of the reaction components.

### Detailed Description of the Invention

To solve the above-mentioned problem, the method of the present invention uses immobilized restriction endonucleases or restriction enzymes (as defined above) for the linearization of plasmid DNA (as defined above).

In a first aspect, the present invention uses an immobilized restriction enzyme for linearization of plasmid DNA to generate linear template DNA in the production process of *in vitro* transcribed RNA.

In this context, any restriction endonuclease (see e.g. Roberts et al. (2015) Nucl. Acids Res. 43.D1: D298-D299.) may be used wherein type II restriction endonucleases are preferred, type IIP and type IIS restriction endonucleases are more preferred and the restriction endonucleases EcoRI, BciVI, SpeI, XbaI, NdeI, AflII, SacI, KpnI, SmaI, BamHI, SalI, Sbfl, PstI and HindIII are particularly preferred. Among those, the restriction endonucleases EcoRI, XbaI, BciVI, NdeI and AflII are even more preferred and the restriction endonuclease EcoRI is most preferred.

For immobilization of said restriction endonucleases any coupling or attachment strategy may be used.

In general, it is known in the art that immobilization of enzymes should avoid steric hindrances, enzyme aggregation and denaturation (Mateo et al. (2007) Enzyme and Microbial Technology 40.6: 1451-1463.).

In general, immobilization of an enzyme can be performed in manifold ways, as exemplified in various reviews, including Datta et al. (2013) 3 Biotech 3.1: 1-9 and Kim and Herr (2013) Biomicrofluidics 7.4: 041501.

An immobilization procedure for a restriction endonuclease has to consider aspects of how the enzyme may be coupled and on which support material the coupling may occur. Immobilization of a restriction enzyme comprises two technical aspects: support material and coupling/attachment of the enzymes to the support material **(****Figure 1****)**.

Restriction endonucleases have, besides other important structural features, dimerization or multimerization domains, catalytic cores where digestion of the DNA occurs, and DNA binding surfaces. All those key structural features have to be intact for proper enzyme functionality, that is, DNA digestion at or adjacent to respective DNA recognition motifs. Therefore, any coupling strategy should fulfill prerequisites for successful restriction endonuclease immobilization as exemplified below.
(I) Restriction endonucleases should retain or enhance their biological activity after coupling.
(II) The immobilized restriction endonuclease should have similar or even a better long-term and thermal stability, leading to a longer shelf life.
(III) The sensitivity and reactivity of the restriction endonuclease should be preserved after immobilization.
(IV) The immobilization procedure should be strong enough and stable enough to minimize enzyme leakage or leakage of the support material or leakage of other chemicals involved in the immobilization process.

In principle, coupling strategies mainly comprise, but are not limited to, entrapment/encapsulation, physical adsorption, bio-affinity interactions, and covalent bond **(cf.** **Figure 1****).**

An immobilization support may comprise metals, silicon, glass, polydimethylsiloxane (PDMS), plastic materials, porous membranes, papers, alkoxysilane-based sol gels, agarose, sepharose, polymethylacrylate, polyacrylamide, cellulose, and silica, monolithic supports, and expanded-bed adsorbents. The choice of a suitable support material largely depends on the coupling strategy. Therefore, potential support materials are mentioned in the context of the respective coupling strategy.

The basic principle of protein entrapment/encapsulation is that the restriction endonuclease may be encapsulated in the interior of the respective support material, which may prevent enzyme aggregation and enzyme denaturation. Possible support materials for protein entrapment/encapsulation comprise polyacrylamide gels, sol-gels, lipid vesicles and polymers such as poly (lactic acid) and poly (lactic-co-glycolic acid).

Physical adsorption, where the restriction endonuclease may bind passively on a particular support material, is based on physical forces such as electrostatic, hydrophobic, van der Waals, and hydrogen bonding interactions. Physical adsorption is based on random binding of the restriction endonuclease on multiple anchoring points to the support material. Possible support materials for physical adsorption comprise metal, silicon, glass, PDMS, and various adhesive plastic materials.

Bio-affinity immobilization strategies exploit the affinity interactions of different biological systems comprising the avidin-biotin system and affinity capture ligands (His/GST tags).

In the widely employed avidin-biotin strategy, partners for biomolecules are avidin (tetrameric glycoprotein from chicken eggs), or neutravidin (deglycosylated version of avidin), or streptavidin (a protein form *Streptomyces avidinii* with higher affinity than avidin) and biotin (water soluble vitamin-B) that form strong non-covalent interactions. Biotinylated moieties strongly bind avidin/streptavidin. Biotinylation, that is the conjugation of biotin on molecules particularly proteins, usually does not affect functionality or conformation of proteins due to its small size. Restriction endonucleases may be biotinylated chemically or enzymatically. Most chemical biotinylation reagents consist of a reactive group attached via a linker to the valeric acid side chain of biotin. As the biotin binding pocket in avidin/streptavidin is buried beneath the protein surface, biotinylation reagents possessing a longer linker are desirable, as they enable the biotin molecule to be more accessible to binding avidin/streptavidin protein. Chemical biotinylation may occur on several moieties in the restriction endonuclease including primary amines (-NH2), thiols (-SH, located on cysteines) and carboxyls (-COOH, a group located at the C-terminus of each polypeptide chain and in the side chains of aspartic acid and glutamic acid). All these above mentioned biotinylation targets in a protein can be used, depending on the respective buffer and pH conditions. For example, free thiol groups (sulfhydryl groups, -SH, located on cysteine side chains) are less prevalent on most proteins. Biotinylation of thiol groups is useful when primary amines are located in the regulatory domain(s) of the target protein or when a reduced level of biotinylation is required. Thiol-reactive groups such as maleimeides, haloacetyls and pyridyl disulfides require free thiol groups for conjugation; disulfide bonds must first be reduced to free up the thiol groups for biotinylation. If no free thiol groups are available, lysines can be modified with various thiolation reagents (Traut's Reagent, SAT (PEG4), SATA and SATP), resulting in the addition of a free sulfhydryl. Thiol biotinylation is performed in a pH range of 6.5-7.5. The biotin-avidin strategy has been used in the art to couple the restriction endonuclease EcoRI to fluorescent beads. After EcoRI biotinylation, the enzyme was coupled to an avidin-coated fluorescent bead. Both gel-shift assay and functional assays could demonstrate that this tagged EcoRI protein was functional (Dylla-Spears, Rebecca, et al. Analytical chemistry 81.24 (2009): 10049-10054).

Possible support materials for immobilizing restriction endonucleases using the biotin-avidin strategy comprise, but are not limited to, agarose, sepharose, glass beads, which are coated with avidin or streptavidin. Particularly preferred is agarose and sepharose as support material.

Affinity capture ligands comprise, but are not limited to, oligohistidine-tag (His) and (glutathione-S-transferase) GST tags.

The C- or N- terminus of restriction endonucleases may be genetically engineered to have a His segment that specifically chelates with metal ions (e.g., Ni2Þ). Ni2Þ is then bound to another chelating agent such as NTA (nitriloacetic acid), which is typically covalently bound to an immobilization support material. The controlled orientation of immobilized restriction endonucleases may be facilitated, as the His tags can in principal be placed to the C- or N-terminus of each protein. Possible support materials comprise, but are not limited to, various nickel or cobalt chelated complexes, particularly preferred are nickel-chelated agarose or sepharose beads.

GST (glutathione S-transferase) may be tagged onto the C- or N-terminus (commonly the N-terminus is used) of restriction endonucleases by genetic engineering. The result would be a GST-tagged fusion protein. GST strongly binds to its substrate glutathione. Glutathione is a tripeptide (Glu-Cys-Gly) that is the specific substrate for glutathione S-transferase (GST). When reduced glutathione (G233SH) is immobilized through its thiol group to a solid support matreial, such as cross-linked beaded agarose or sepharose, it can be used to capture GST-tagged restriction endonucleases via the enzyme-substrate binding reaction. Possible support materials comprise, but are not limited to, glutathione (GSH) functionalized support materials, particularly GSH-coated beads, particularly preferred GSH-coated agarose or sepharose.

Covalent immobilization is generally considered to have the advantage that the protein to be immobilized and the corresponding support material have the strongest binding, which is supposed to minimize the risk of proteins to dissociate from the support material, also referred to as enzyme leakage.

To achieve covalent binding of the restriction endonuclease to the support material, the respective support material has to be chemically activated via reactive reagents. Then, the activated support material reacts with functional groups on amino acid residues and side chains on the restriction endonuclease to form covalent bonds.

Functional groups on the restriction endonuclease suitable for covalent binding comprise, but are not limited to, primary amines (-NH2) existing at the N-terminus of each polypeptide chain and in the side-chain of lysine (Lys, K), α-carboxyl groups and the β- and γ-carboxyl groups of aspartic and glutamic acid, and sulfhydryl or thiol groups of cysteines.

Primary amines (-NH2) provide a simple target for various immobilization strategies. This involves the use of chemical groups that react with primary amines. Primary amines are positively charged at physiological pH; therefore, they occur predominantly on the outer surfaces of the protein and are mostly accessible to immobilization procedures. Suitable support materials for immobilization via primary amines comprise, but are not limited to, formaldehyde and glutaraldehyde activated support materials, 3-aminopropyltriethoxysilane (APTES) activated support materials, cyanogen bromide (CnBr) activated support materials, N-hydroxysuccinimide (NHS) esters and imidoesters activated support materials, azlactone activated support materials, and carbonyl diimidazole (CDI) activated support materials.

There are several reports in the art that use amines (NH2) for the immobilization of restriction endonucleases.

The restriction endonucleases HaeIII and HindIII were immobilized on a glutaraldehyde-activated silica support and the activity of immobilized HaeIII and HindIII restriction endonucleases was tested by digesting viral double-stranded DNA (SV40) (Davidson et al. (2001) Journal of separation science 24(1): 10-16).

The immobilization of restriction endonucleases BamHI and EcoRI to CNBr - activated Sepharose improved the thermal stability and shelf-life of the enzymes (Lee et al. (1978) Nucl. Acids Res. 5(3): 679-689).

The carboxyl group is a frequent moiety (-COOH) at the C-terminus of each polypeptide chain and in the side chains of aspartic acid (Asp, D) and glutamic acid (Glu, E), usually located on the surface of the protein structure. Carboxylic acids may be used to immobilize restriction endonucleases through the use of a carbodiimide-mediated reaction.

1-ethyl-3-(3-dimethylaminoipropyl) carbodiimide (EDC) and other carbodiimides cause direct conjugation of carboxylates (-COOH) to primary amines (-NH2).

Possible support materials comprise, but are not limited to, diaminodipropylamine (DADPA) agarose resin that allows direct EDC-mediated crosslinking, which usually causes random polymerization of proteins.

Site-specific covalent immobilization allows that the enzymes are immobilized in a definite, oriented fashion. Therefore the presence of unique and mutually reactive groups on the protein (e.g., thiol group of cysteine) and the support (e.g., thiol activated sepharose) is particularly preferred.

Sulfhydryl groups, also called thiol groups, which have the structure R-SH, typically allow a selective immobilization of proteins and peptides as they commonly occur in lower frequencies (Hansen et al. (2009) Proc. Natl. Acad. Sci. USA 106.2: 422-427). These thiol groups may be used for direct immobilization reactions of restriction endonucleases to activated support materials, forming either thioether linkages (R-S-R) prepared by the alkylation of thiols or disulfide bonds (R-S-S-R) derived from coupling two thiol groups. The thiol groups necessary for those reactions may have different sources:
a) Thiol groups of inherent or native free cysteine residues of the protein to be immobilized.
b) Often, as part of a protein's secondary or tertiary structure, cysteine residues are joined together between their side chains via disulfide bonds. Thiol groups can be generated from existing disulfide bridges using reducing agents.
c) Thiol groups can be generated through the use of thiolation reagents, which add thiol groups to primary amines.
d) Thiol groups can be genetically introduced by adding a cysteine residue at the C- or N-terminus, by introducing a cysteine residue into the protein sequence or by substituting an amino acid residue within the protein sequence with a cysteine residue.

In a preferred embodiment, restriction endonucleases are covalently coupled via the thiol groups of cysteine (native or introduced) to a suitable support material, more preferably they are coupled via disulfide bonds to a thiol-activated support material or via a thioether bond to a maleimide-activated solid support or to a pyridyl disulfide-functionalized solid support or to an epoxy activated support. Thiol-activated support material contains chemical groups which are capable of reacting with the thiol group of the restriction endonuclease, such as maleimides, epoxy, haloacetyls and pyridyl disulfides. Suitable solid supports include thiol sepharose, thiopropyl-sepharose, thiol-activated sephadex, thiol-activated agarose, silica-based thiol-activated matrix, silica-based thiol-activated magnetic beads and epoxy methacrylate beads. Specific examples of thiol-activated sepharose are thiol-activated sepharose such as Thiol Sepharose™ 4B HiTrap or Thiol Sepharose™ 4B available, for example, from GE Healthcare Life Sciences. Suitable pyridyl disulfide-functionalized supports include nanoparticles such as Nanosprings® of STREM chemicals or any amine-containing support thiolated by an N-Hydroxysuccinimide-pyridyl disulfide like NHS-PEG₄-pyridyl disulfide. In further examples, the solid support comprises pyridyl disulfide-functionalized nanoparticles and/or maleimide-activated agarose.

The inventors consider the immobilization via thiol groups to be generally advantageous because, commonly, only a low number of free existing thiol groups exist in the protein primary structure of enzymes (Hansen et al. (2009) Proc. Natl. Acad. Sci. USA 106.2: 422-427).

This may allow a more controlled and efficient way of immobilization, that is oriented immobilization which means that the site within the enzyme with which it is immobilized is defined and not randomly selected. Such an oriented immobilization is particularly preferred in the context of the present invention. Additionally, this immobilization strategy may avoid multiple coupling events to the support material which may lead to steric hindrances or other disturbances which may eventually decrease enzyme stability, reactivity and specificity. Moreover, the covalent coupling via thiol groups of the respective restriction endonuclease may have the advantage of a very strong bond that, most importantly, minimizes the danger of an uncontrolled dissociation of support material and enzyme.

If a restriction endonuclease may be covalently coupled via the thiol group of cysteine to the support material, several aspects should be considered by a person skilled in the art:
I) If several cysteine residues are present in the primary protein structure, free thiol groups, meaning cysteine residues not linked to other cysteine residues via disulfide bridges, may be identified using disulfide bridge prediction algorithms (Yaseen, Ashraf, and Yaohang Li. BMC bioinformatics 14.Suppl 13 (2013): S9.).
II) The free existing thiol groups should not be present in the catalytic core, the dimerization or multimerization surface, the DNA-binding domain or other functional relevant parts of the restriction endonuclease due to the potential problems caused, as explained above. A person skilled in the art may first conduct the present literature on the structure of restriction endonucleases (see, e.g., **Figure 2**) or literature on structure-function relationships to identify such potential cysteine residues (e.g., Pingoud and Jeltsch (2001) Nucl. Acids Res. 29(18): 3705-3727)).
III) If several free thiol groups from cysteines are present in the primary sequence of the protein, that are not located in the catalytic core, the dimerization or multimerization surface, the DNA-binding domain or other functional relevant parts of the restriction endonuclease, these cysteines may be substituted with a different amino acid, preferably serine (similar size) or alanine (similar charge) or valine, preferably by genetic means. This may help to avoid multiple coupling events to the support which might lead to steric hindrances or other disturbances that could eventually decrease enzyme stability, reactivity and specificity. Protein visualization tools (e.g., PDB viewer, Guex and Peitsch (1997) Electrophoresis 18: 2714-2723) may help a person skilled in the art to decide whether respective cysteine residues should be substituted in the restriction endonuclease. Moreover, the effect of certain cysteine substitutions / point mutations can also be estimated, even without structural knowledge, using machine-learning based prediction tools (Rost et al. (2004) Nucl. Acids Res. 32.suppl 2: W321-W326).
IV) If free thiol groups are present in the primary structure of the respective restriction endonuclease, a person skilled in the art may also use recent literature on the respective protein structure, if available, to assess if these cysteine residues are accessible for chemical interactions (i.e., covalent bond to a support material), or if these cysteine residues are buried in the interior of the protein 3-D structure. A person skilled in the art may use algorithms to predict if a respective cysteine is buried or freely accessible by performing calculations comprising residue depth calculations or solvent-accessible surface area calculations (Xu, Dong, Hua Li, and Yang Zhang. Journal of Computational Biology 20.10 (2013): 805-816).
V) If no freely accessible cysteine residues are present in the primary structure of the respective restriction endonuclease, cysteine residues may be introduced by various means. For example, cysteine residues may be introduced at the N-terminus or C-terminus of the restriction endonuclease by methods comprising genetic engineering, either by extending the N-terminus or the C-terminus or by substitution of the N-terminal-most or C-terminal-most amino acid. Moreover, a person skilled in the art may introduce flexible linkers, in particular, if the N- or C- terminus of the restriction endonuclease displays important functional or structural features (Chen et al. (2013) Advanced drug delivery reviews 65.10: 1357-1369). Again, cysteine residues may also be introduced into any other suitable regions of the protein by substitution of amino acids within these regions. Ideally, such residues should be located at the protein surface. Moreover, such residues should not be located in relevant parts of the restriction endonuclease (as explained above). Preferably, an amino acid that occupies a similar space in a protein 3-D structure such as serine may be considered for an S to C substitution.
VI) If the respective restriction enzyme is composed of several identical subunits (e.g., homodimer, homo-multimer), it should be considered that coupling of support material to one monomer may lead to a multimer with several covalently bound support particles. For example, coupling of one support particle to an EcoRI monomer would lead to the formation of a homodimer with two coupled support particles. Some restriction endonucleases (e.g., type IIS), hovewer, can also act as monomers (Armalyte, Elena, et al. Journal of Biological Chemistry 280.50 (2005): 41584-41594). In such a case, only one support particle would be coupled to the restriction endonuclease.

Alternatively, or in addition to that, cysteine residues may be substituted with any other amino acid. For example, the wild type restriction endonuclease sequence may be aligned with multiple related sequences from other organisms to identify suitable amino acid substitutions that do not influence enzymatic activity. A person skilled in the art will know how to find similar sequences and how to align those sequences and how to identify suitable amino acids for substituting naturally occurring cysteines in a restriction endonuclease.

In a more preferred embodiment, a type II restriction endonuclease is covalently coupled via a thiol group of a cysteine residue to a suitable activated support material such as thiol sepharose. The cysteine residue should be located outside the catalytic core, the DNA binding domains and the dimerization/multimerization domains of the enzyme. These domains are known to the expert and for EcoRI and HindIII are also shown in the figures of this application.

The cysteine residue used for coupling may be present in the wild-type enzyme, if it is in a position suitable for coupling, or it may be introduced into the enzyme at a suitable position, preferably at the N- or the C-terminus of the enzyme. The cysteine residue can be coupled to the N- or C-terminus directly, i.e. by forming a peptide bond with the N- or C-terminal amino acid of the wild-type enzyme, or via a linker as defined herein. Alternatively, N- or C-terminal amino acid of the wild-type enzyme may be substituted with a cysteine residue.

Additionally, any cysteine residue present in the wild-type enzyme which is not suitable for coupling to a solid support may be substituted with another amino acid to avoid any residual, but uncontrollable coupling by this cysteine residue.

In a preferred embodiment, the type II restriction endonuclease BciVI (BfuI) (**Figure 3**) is covalently coupled via a thiol group of an introduced cysteine residue to a suitable thiol-activated support material.

Structural information on BciVI (BfuI) is currently not available in the art. The sequence of wild-type BciVI as depicted in SEQ ID No. 3 harbors one native cysteine residue at position 271 (C271) **(****Figure 3****).** Based on solvent accessibility predictions, that respective C271 residue is not in direct contact with the solvent, which may lead to a reduced coupling efficiency. Therefore, that native cysteine residue may not be suitable for immobilization strategies. Moreover, substitution of that C271 residue to alanine, isoleucine, serine, or valine is predicted to be without effect for the restriction endonuclease (predictions performed using Rost et al. (2004) Nucl. Acids Res. 32.suppl 2: W321-W326)). A first substitution would therefore be at position 271, where the native cysteine residue is substituted with a different amino acid, preferably serine (C271S) or alanine (C271A). Moreover, a Cysteine residue is introduced at the C-terminus of the restriction endonuclease (359C; see SEQ ID No. 12) **(****Figure 3****).** The use of such mutants (C271A, 359C; SEQ ID No. 13) or (C271S, 359C; SEQ ID No. 14) facilitates an oriented immobilization of BciVI, that is, the support material is only coupled to the C-terminal cysteine residue, and not to the native cysteine residue. BciVI acts as a homodimer. Therefore, a double-mutant as disclosed above guarantees that only two support particles (e.g., sepharose, agarose) are coupled to the enzyme, more precisely, one particle per monomer is coupled to the enzyme.

In another preferred embodiment, the type II restriction endonuclease HindIII **(****Figure 4****)** is covalently coupled via a thiol group of an introduced cysteine residue to a suitable thiol-activated support material.

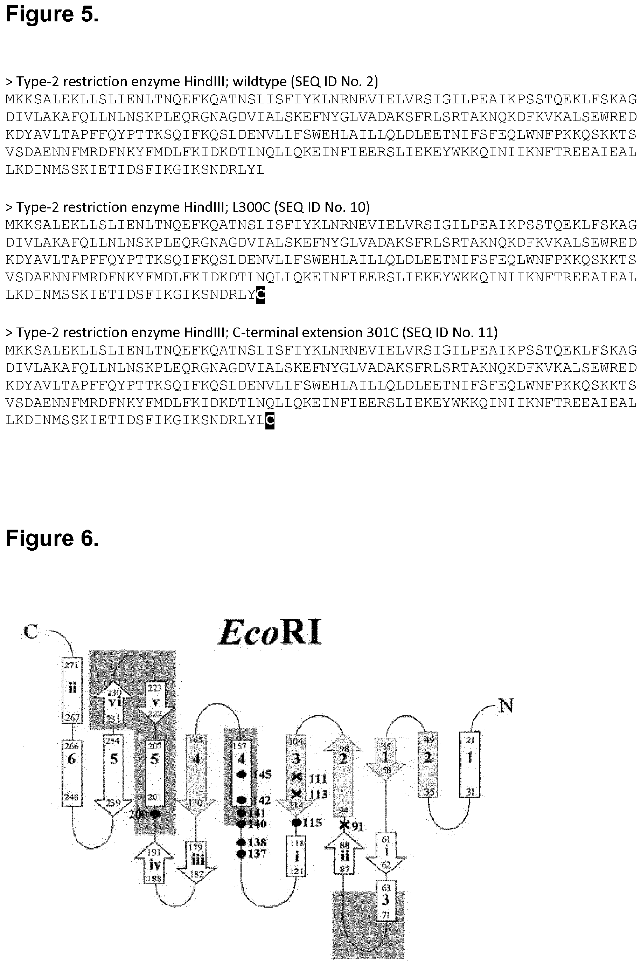

The amino acid sequence of wild-type HindIII as shown in SEQ ID No. 2 does not harbor native cysteine residues. The structural properties of HindIII (Watanabe et al. (2009) Acta Crystallographica Section D: Biological Crystallography 65.12: 1326-1333) suggest that the C-terminus may be an ideal target for a covalently bound support **(****Figure 4****).** Therefore, a recombinant mutant HindIII protein in which the C-terminal leucine is replaced with cysteine (L300C; SEQ ID No. 10) or a cysteine is added to the C-terminus (301C; SEQ ID No. 11) may be used for oriented immobilization **(****Figure 5****).** As HindIII also functions as a homodimer, two support particles (e.g., Sepharose) are coupled to the immobilized restriction endonuclease.

In a particularly preferred embodiment, the type II restriction endonuclease EcoRI **(****Figure 6****)** is covalently coupled via the thiol group of an introduced cysteine residue to a suitable thiol-activated support material.

The wild-type amino acid sequence of EcoRI, which is a type II restriction endonuclease, has only one native cysteine residue at position 218 (C218) (see SEQ ID No. 1) (**Figure 7**). In the art, this residue has been used to immobilize EcoRI by coupling the enzyme to thiol-activated Sephadex G-10 support material (Bircakova et al. (1996) Journal of Molecular Recognition 9.5-6: 683-690). However, more recent work has reported that cysteine C218 is a (slightly) buried residue not located at the surface of the protein, suggesting that the thiol group of that cysteine may not be amenable to modification (e.g., coupling to a support) at high yield (Dylla-Spears et al. (2009) Analytical chemistry 81.24: 10049-10054; Stone et al. (2008) Angewandte Chemie 120.52: 10346-10348). Hence, preferably a cysteine residue is introduced into the amino acid sequence of EcoRI to enable coupling to the solid support.

In principle, introduction of cysteine residues in EcoRI is possible via the substitution of amino acids with cysteine at any position of the protein primary sequence, by introducing an additional cysteine residue into the protein primary sequence or by extending the free N- or C-termini. However, several important aspects should be considered by a person skilled in the art if a cysteine residue may be introduced to EcoRI via substitution:
I) Amino acids that are particularly important for the dimerization of EcoRI, for the DNA binding (e.g., residues 115, 137, 138, 140, 141, 142, 145) or for catalysis (e.g., 91, 111, 113) (see **Figure 6**) should not be substituted with cysteine.
II) Amino acid residues that are located at the surface of EcoRI are potential targets for substitution with cysteine. Particularly, serine residues that have a similar size than cysteine residues may be preferred targets.
III) Amino acids that are not at the surface of EcoRI should not be changed to cysteine, as their thiol groups might not react with the respective support material. Moreover, a substitution of residues located in the interior of the protein may locally disrupt the protein structure.

In a particularly preferred embodiment, a recombinant EcoRI (K277C; SEQ ID No. 4) mutant protein may be used for immobilization **(****Figure 7****),** i.e. a mutant in which the C-terminal lysine residue is replaced with a cysteine residue.

Recombinant EcoRI enzyme has been described in the art, where an internal lysine residue (K249) was substituted to cysteine. (Dylla-Spears et al. (2009) Analytical chemistry 81.24: 10049-10054; Ghindilis, et al. Scientific reports 5 (2015)).

In a further preferred embodiment a recombinant EcoRI double-mutant protein is used. The first substitution is at position 277, where the C-terminal lysine residue is substituted with a cysteine residue (K277C). The second substitution is at position 218, where the native cysteine residue is substituted with a different amino acid, preferably serine (C218S; SEQ ID No. 6) or alanine (C218A; SEQ ID No. 5) **(****Figure 7**) or valine (C218V; SEQ ID No. 20). The use of such a double-mutant facilitates an oriented immobilization of EcoRI, that is, the support material is only coupled to the C-terminal cysteine residue, and not to the (buried) native cysteine residue. EcoRI acts as a homodimer. Therefore, a double-mutant as disclosed above guarantees that only two support particles (e.g., sepharose, agarose) are coupled to the enzyme, more precisely, one particle per monomer is coupled to the enzyme.

In another embodiment, a recombinant EcoRI mutant with a C-terminal cysteine addition (278C; SEQ ID No. 15) may be used for immobilization. This mutant may have an additional substitution at position 218, where the native cysteine residue is substituted to another amino acid such as serine (C218S; SEQ ID No. 17) or alanine (C218A; SEQ ID No. 16) **(****Figure 7****)** or valine (C218V; SEQ ID No. 22). The use of such an enzyme also facilitates an oriented immobilization of EcoRI, that is, the support material is merely coupled to the C-terminal cysteine residue, and not to the buried native cysteine residue. EcoRI acts as a homodimer. Therefore, a double-mutant as disclosed above guarantees that only two support particles (e.g., Sepharose) are coupled to the enzyme, more precisely, one particle per monomer is coupled to the enzyme.

In still another embodiment, a C-terminal cysteine may be added to a recombinant EcoRI via an amino acid linker attached to the C-terminal lysine residue of wild-type EcoRI. Such an amino acid linker may comprise between 1 and 20 amino acids, preferably between 3 and 18 amino acids, more preferably between 5 and 15 amino acids and most preferably between 8 and 12 amino acids. The linker molecule typically consists of small amino acid residues such as glycine and serine. In particular, a cysteine is added to the C-terminus of recombinant EcoRI via a linker comprising 12 glycine residues. The amino acid sequence of such an enzyme is shown in SEQ ID No. 7. Preferably, the recombinant EcoRI has an additional substitution at position 218, where the native cysteine residue is substituted to another amino acid such as serine (C218S; SEQ ID No. 9) or alanine (C218A; SEQ ID No. 8) or valine (C218V; SEQ ID No. 21).

In other preferred embodiments, the mutant EcoRI enzyme comprises the linker element GGGGSGGGGS to which an additional cysteine residue is attached and has the sequence according to SEQ ID No. 27. Preferably, the recombinant EcoRI has an additional substitution at position 218, where the native cysteine residue is substituted to another amino acid such as serine (C218S; SEQ ID No. 29) or alanine (C218A; SEQ ID No. 28) or valine (C218V; SEQ ID No. 30).

In other preferred embodiments, the mutant EcoRI enzyme comprises at its C-terminus in 5' to 3' order the linker element GGGGSGGGGS, a purification tag comprising six histidine residues and an additional cysteine. This mutant EcoRI enzyme has the sequence according to SEQ ID No. 31. Preferably, the recombinant EcoRI has an additional substitution at position 218, where the native cysteine residue is substituted to another amino acid such as serine (C218S; SEQ ID No. 33) or alanine (C218A; SEQ ID No. 32) or valine (C218V; SEQ ID No. 34).

In some embodiments, the linker element can be selected from the group comprising the sequences according to any of SEQ ID Nos. 35 - 59.

In some embodiments, the purification tag can be selected from the group comprising the sequences according to any of SEQ ID Nos. 60 - 80.

In some embodiments, the mutant EcoRI enzyme comprises at its C-terminus in 5' to 3' order a purification tag comprising six histidine residues and an additional cysteine. This mutant enzyme has the sequence according to SEQ ID No. 23. Preferably, the recombinant EcoRI has an additional substitution at position 218, where the native cysteine residue is substituted to another amino acid such as serine (C218S; SEQ ID No. 25) or alanine (C218A; SEQ ID No. 24) or valine (C218V; SEQ ID No. 26).

The use of such a double mutant also facilitates an oriented immobilization of EcoRI, that is, the support material is merely coupled to the C-terminal cysteine residue, and not at small but nonetheless uncontrollable amounts to the buried native cysteine residue. EcoRI acts as a homodimer. Therefore, a double-mutant as disclosed above guarantees that only two support particles (e.g., Sepharose) are coupled to the enzyme, more precisely, one particle per monomer is coupled to the enzyme.

Within the scope of the present invention not only the wild-type restriction endonucleases can be used, but also functional variants thereof. Variants of the restriction endonucleases have a sequence which differs from that of the wild-type restriction endonucleases by one or more amino acid substitutions, deletions or additions, resulting in a sequence identity to the wild-type restriction endonuclease such as EcoRI as defined by SEQ ID No. 1, HindIII as defined by SEQ ID No. 2 or BciVI as defined by SEQ ID No. 3 of at least 80%, preferably of at least 81%, 82%, 83%, 84% or 85%, more preferably of at least 86%, 87%, 88%, 89%, 90%, 91 %, 92%, 93% or 94%, even more preferably of at least 95%, 96% and most preferably of at least 97%, 98% or 99%. The sequence identity can for example be determined using the BLAST algorithm known to the skilled person by aligning the two sequences to be compared using the default settings of this algorithm.

Variants defined as above are functional variants, if they retain the function of the wild-type enzyme, i.e. the ability of the enzyme to cleave a target DNA at the recognition site of the wild-type enzyme. The enzyme activity of the functional variant of the restriction enzyme is at least 50%, 60% or 70%, preferably at least 75%, 80% or 85%, more preferably at least 87%, 89%, 91% or 93% and most preferably at least 94%, 95%, 96%, 97%, 98% or 99% of the wild-type enzyme such as the EcoRI as defined by SEQ ID No. 1, HindIII as defined by SEQ ID No. 2 or BciVI as defined by SEQ ID No. 3. The enzyme activity of a variant of a restriction enzyme can be determined by incubating the variant with a suitable substrate DNA and determining the amount of cleaved substrate in comparison to the amount of substrate cleaved by the wild-type enzyme.

It is possible to identify the residues in the variant which correspond to those in the wild-type enzyme by aligning the amino acid sequences of the wild-type and variant enzymes using alignment software known to the skilled person.

Despite the low homology among type II, or type IIB restriction enzymes, they show a surprisingly high structural similarity (Pingoud and Jeltsch (2001) Nucl. Acids Res. 29(18): 3705-3727). Therefore, the immobilization strategy developed herein for EcoRI and HindIII is also transferable to other restriction enzymes, preferably members of the restriction class IIB EcoRI family. This immobilization strategy is also suitable for EcoRV family members which also comprise a C-terminus which does not take part in the common core elements, the catalytic reaction center or in dimerization.

Therefore, an immobilization strategy as disclosed herein is also applicable for immobilizing other restriction endonucleases, also including type I and type III restriction endonucleases (Roberts et al. (2015) Nucl. Acids Res. 43.D1: D298-D299.).

Several different approaches exist in the art to bind a support material to thiol group-containing proteins. Thiol-reactive chemical groups present on thiol-activated support materials include maleimides, epoxy, haloacetyls, pyridyl disulfides and other disulfide reducing agents. Most of these groups conjugate to thiols on the respective protein by either alkylation (usually the formation of a thioether bond) or disulfide exchange (formation of a disulfide bond). The terms "functionalized" and "activated" with respect to the solid support are used interchangeably and refer to the chemical group which is available on the surface of the solid support for immobilization of the restriction endonuclease.

Maleimide-activated reagents react specifically with thiol groups (-SH) at near neutral conditions (pH 6.5 - 7.5) to form stable thioether linkages. The maleimide chemistry is the basis for most crosslinkers and labeling reagents designed for conjugation of thiol groups. Thiol-containing compounds, such as dithiothreitol (DTT) and betamercaptoethanol (BME), must be excluded from reaction buffers used with maleimides because they will compete for coupling sites.

Haloacetyls react with thiol groups at physiological pH. The reaction of the iodoacetyl group proceeds by nucleophilic substitution of iodine with a sulfur atom from a thiol group, resulting in a stable thioether linkage. Using a slight excess of the iodoacetyl group over the number of thiol groups at pH 8.3 ensures thiol selectivity. Histidyl side chains and amino groups react in the unprotonated form with iodoacetyl groups above pH 5 and pH 7, respectively. To limit free iodine generation, which has the potential to react with tyrosine, histidine and tryptophan residues, the iodoacetyl reactions and preparations should be performed in the dark.

Pyridyl disulfides react with thiol groups over a broad pH range (the optimum is pH 4 to 5) to form disulfide bonds. During the reaction, a disulfide exchange occurs between the molecule's -SH group and the reagent's 2-pyridyldithiol group. As a result, pyridine-2-thione is released and can be measured spectrophotometrically (Amax = 343nm) to monitor the progress of the reaction. These reagents can be used as crosslinkers and to introduce thiol groups into proteins. The disulfide exchange can be performed at physiological pH, although the reaction rate is slower than under acidic conditions.

There are several commercially available thiol-activated support materials comprising thiol-activated sepharose such as Thiol Sepharose 4B (GE Healthcare) and thiol-activated agarose that are preferably used to immobilize thiol-group-containing restriction endonucleases. Particularly preferred support materials comprise thiol-activated sepharose. Most preferably, the solid support is thiol sepharose 4B and thiol sepharose 6B.

Another very potent solid support is an epoxy functionalized solid support. Epoxy comprises the functional group as depicted in Formula (I):

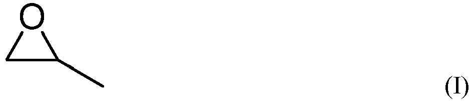

Epoxy-activated matrices can be used for coupling ligands stably through amino, thiol, phenolic or hydroxyl groups depending on the pH employed in the coupling reaction. Immobilization via epoxy groups is also described by Mateo et al. (2000) Biomacromolecules 1(4): 739-745. If the immobilization reaction takes place at a pH between 7.5 - 8.5, i.e. at physiological conditions, the attachment occurs at thiol groups, if the reaction takes place at a pH between 9 and 11, attachment occurs at amine residues and if the reaction takes place at a pH above 11, the attachment occurs at hydroxyl groups.

Examples of Epoxy-activated resins are Purolite® ECR8205 epoxy methacrylate and Purolite® ECR8214 epoxy methacrylate are e.g. obtainable from Purolite® Corp., Llantrisant, UK, which are produced via crosslinking in the presence of a porogenic agent that allows the control of porosity, or ECR8204F epoxy-methacrylate beads which are obtainable from Lifetech™, Thermo Fisher Scientific, Waltham, MA USA). ECR8204F beads are of 150 - 300 µm diameter (mean = 198) and pores of 300 - 600 Å.

Sepharose-immobilized restriction endonuclease may be re-solubilized using reducing agents such as DTT or mercaptoethanol, or low pH to potentially re-use the support material.

The plasmid DNA serving as a substrate for the restriction endonucleases has to comprise at least one recognition sequence for the restriction enzyme which is to be used for cutting the plasmid DNA. For example, if the immobilized enzyme to be used within the present invention is EcoRI, the plasmid DNA has to comprise at least one recognition sequence for EcoRI. More preferably, the plasmid DNA has only one recognition sequence for the restriction enzyme, so that the linear template DNA produced by the restriction enzyme has the same number of nucleotides as the plasmid DNA. To enable *in vitro* transcription of the linear template DNA, the recognition sequence(s) for the restriction enzyme should be located outside the template sequences to be *in vitro* transcribed and the control sequences, that is the promotor sequence, controlling the *in vitro* transcription of the RNA. To achieve desired length homogeneity of the transcribed RNA molecules as described above, one of the restriction sites within the plasmid DNA, preferably the only restriction site for the restriction enzyme used, is preferably located at the 3' end of the template sequence required for producing the RNA, preferably 3' of the polyadenylation sequence or any histone stem loop. As a result, this facilitates the controlled termination of the *in vitro* transcription reaction.

In another aspect the present invention provides an enzyme reactor comprising said immobilized restriction endonuclease (as defined above), which is particularly useful for linearization of plasmid DNA.

Any enzyme reactor known to a skilled person or in the art may be used according to the present invention.

In general, an enzyme reactor consists of a vessel or series of vessels used to perform the desired enzymatic reaction, for example the linearization of plasmid DNA by restriction endonucleases, particularly by immobilized restriction endonucleases (as defined above). Hence, the enzyme reactor contains all reagents necessary to perform the restriction reaction.

In a preferred embodiment, the enzyme reactor contains immobilized EcoRI, particularly preferred is sepharose-immobilized EcoRI. Preferably, the immobilized EcoRI is any of the EcoRI mutants disclosed herein, such as a mutant in which the C-terminal lysine is replaced with a cysteine residue (K277C, SEQ ID No. 4), optionally in combination with a substitution of the native cysteine at position 218 with another amino acid such as alanine (C218A, K277C; SEQ ID No. 5) or serine (C218S, K277C; SEQ ID No. 6) or valine (C218V, K277C; SEQ ID No. 20) or in which a cysteine residue is added to the C-terminus (278C; SEQ ID No. 15), optionally in combination with a substitution of the native cysteine at position 218 with another amino acid such as alanine (C218A, 278C; SEQ ID No. 16) or serine (C218S, 278C; SEQ ID No. 17) or valine (C218V, 278C; SEQ ID No. 22), or in which a cysteine residue is added to the C-terminus via an amino acid linker (SEQ ID No. 7), optionally in combination with a substitution of the native cysteine at position 218 with another amino acid such as alanine (SEQ ID No. 8) or serine (SEQ ID No. 9) or valine (SEQ ID No. 21), or in which a cysteine residue is added to the C-terminus via an amino acid flexible linker (SEQ ID No. 27), optionally in combination with a substitution of the native cysteine at position 218 with another amino acid such as alanine (SEQ ID No. 28) or serine (SEQ ID No. 29), or valine (SEQ ID No. 30), or in which a cysteine residue is added to the C-terminus via an amino acid flexible linker and a purification tag (SEQ ID No. 31), optionally in combination with a substitution of the native cysteine at position 218 with another amino acid such as alanine (SEQ ID No. 32) or serine (SEQ ID No. 33) or valine (SEQ ID No. 34).

Important reactor types that may be used for the present invention comprise, but are not limited to, variants of stirred-tank batch reactors, continuous stirred-tank batch reactors, recirculation batch reactors, stirred tank-ultrafiltration reactors, and continuous packed-bed reactors (Illanes, Andrés, ed. Enzyme biocatalysis: principles and applications. Springer Science & Business Media, 2008, chapter 5), **Figure 8****.** All these reactor types listed above may additionally contain sensors to measure parameters including the operation scale (e.g., inlet flow, outlet flow), pressure, pH, temperature, osmolality, and salinity. All reactor types may additionally have heating/cooling devices, pressure devices, and the stirred reactors may contain elements to control the stirring efficiency. Moreover, some reactors may be connected to a filtration setup, comprising e.g. an ultrafiltration device.

An enzyme reactor (of any kind), including tubes, vessels and other parts (sensors), for use in the present invention may be manufactured from materials with the following critical characteristics:
- No binding of DNA, RNA or protein
- No contamination of chemicals, especially no leaking of hazardous chemicals (e.g., bisphenol A) or allergens (e.g., heavy metals).
- Materials should not influence enzymatic reactions
- Materials should not lead to a detachment of enzyme and support
- Materials should be non- corrosive

Particularly preferred materials include, but are not limited to, stainless steel, glass and any kind of plastic material, that meet the above-mentioned criteria.

**Stirred-tank batch reactors (****Figure 8A****)** may consist of a tank containing a rotating stirrer. The tank may be fitted with fixed baffles to improve the stirring efficiency in the tank. The tank may be loaded with the immobilized restriction endonuclease in a suitable reaction buffer and a plasmid DNA substrate. In such a reactor, the immobilized restriction endonuclease and the plasmid DNA molecules have identical residence times. After enzymatic reaction occurred, and after emptying of the batch reactor, the immobilized restriction endonuclease, the residual plasmid DNA substrate and the linearized template DNA product have to be separated. This can be done e.g. by a filter device or membrane with a pore size smaller than the size of the immobilized restriction endonuclease and bigger than the size of the linearized template DNA. For example, elongated molecules such as linearized template DNA may find their way through pores that will retain a globular species of the same molecular weight such as plasmid DNA (Latulippel and Zydney (2011) Journal of Colloid and Interface Science. 357(2):, Pages 548-553). Preferred in this context are cellulose membranes having nominal molecular weight cutoffs of 100 to 300 kDa. Alternatively, immobilized restriction endonucleases and linearized template DNA may be separated via centrifugation, and eventually, immobilized enzymes may be re-used for another reaction cycle.

A stirred-tank batch reactor is particularly preferred in the context of the present invention. In this context it is particularly preferred to use immobilized EcoRI, e.g immobilized to sepharose, for linearization of plasmid DNA.

In another preferred embodiment, the linearization reactor containing immobilized restriction endonuclease is a continuous stirred-tank batch reactor.

**Continuous stirred-tank batch reactors (****Figure 8B****)** may be constructed similar to stirred-tank batch reactors (see above, cf Figure 8A) with the main difference that continuous flow from inlet and outlet tubes may be applied. One feature of such a reactor type is that the immobilized restriction endonuclease and the plasmid DNA molecules do not have identical residence times in the reactor. Reaction medium, composed of restriction buffer and plasmid DNA, may be pumped into the tank via an inlet that may be located at the bottom of the tank, and reaction buffer containing the digested linearized template DNA product may be moved off via an outlet attached at the top. Inlet and outlet flow may be controlled by a pumping device in such a way that the enzymatic reaction can occur. Moreover, outlet tubes may have molecular weight cutoff filters to avoid contamination of the product by immobilized restriction endonucleases. Preferred in this context are cellulose membranes having nominal molecular weight cutoffs of 100 to 300 kDa (see above). One advantage of such an embodiment is that the immobilized restriction endonuclease does not have to be separated from the linearized template DNA product. The purified linearized template DNA may be subsequently used for *in vitro* transcription of RNA.

In another preferred embodiment, the linearization reactor containing an immobilized restriction endonuclease is a stirred tank ultrafiltration reactor.

A **stirred tank-ultrafiltration reactor (****Figure 8** **C**) may be constructed similar to stirred-tank batch reactors (see above, cf Figures 8A and 8B), with the major difference that a small ultrafiltration device is connected to the reaction chamber where the separation of product (linearized template DNA) and immobilized restriction endonuclease takes place. This separation may be facilitated via an ultrafiltration device. In ultrafiltration, the membranes comprise a discrete porous network. The mixed solution is pumped across the membrane, smaller molecules pass through the pores ("elongated" linear DNA) while larger molecules (plasmid DNA, immobilized restriction enzymes) are retained. Typical operating pressures for ultrafiltration are 1 to 10 bar. The retention properties of ultrafiltration membranes are expressed as molecular weight cutoff (MWCO). This value refers to the approximate molecular weight (MW) of a dilute globular solute (i.e., a typical protein) which is 90% retained by the membrane. However, a molecule's shape can have a direct effect on its retention by a membrane. For example, elongated molecules such as linearized template DNA may find their way through pores that will retain a globular species of the same molecular weight such as plasmid DNA (Latulippel and Zydney (2011) Journal of Colloid and Interface Science 357(2): 548-553). Preferred in this context are cellulose membranes having nominal molecular weight cutoffs of 100 to 300 kDa. Eventually, the immobilized restriction enzyme may be captured in the ultrafiltration device and returned back to the reaction chamber, whereas the filtrate, containing the linearized template DNA, may be used as a template for *in vitro* transcription of RNA.

In another preferred embodiment the linearization reactor containing an immobilized restriction enzyme is a recirculation batch reactor.

**Recirculation batch reactors (****Figure 8 D)** may consist of a first tank, connected via inlet and outlet tubes to a second chamber. The first tank is loaded with immobilized restriction endonucleases. The second chamber contains the reaction buffer and the substrate (i.e. plasmid DNA). In such a recirculating batch reactor, the immobilized restriction endonuclease is densely packed in the first tank through which reaction medium (containing buffer and substrate) is constantly circulating. After enzymatic reaction occurred, the reaction medium that contains reaction buffer and linearized template DNA, can be emptied and used for purification of linearized template DNA. Linearized template DNA may be purified via silica membranes. One advantage of such an embodiment is that the immobilized restriction endonuclease does not have to be separated from the linearized template DNA by other means. The purified linearized template DNA may be used for *in vitro* transcription of RNA.In another preferred embodiment, the linearization reactor containing immobilized restriction endonuclease is a continuous packed bed reactor.

**Continuous packed bed reactors (****Figure 8 E)** may consist of a container filled with immobilized restriction endonuclease catalyst particles. The container may be densely packed, thereby forming a bed containing catalyst particles. One feature of such a reactor type is that the immobilized restriction endonuclease and the plasmid DNA molecules do not have identical residence times in the reactor. Reaction medium, composed of restriction buffer and plasmid DNA, may be pumped into the packed bed reactor via an inlet that may be located at the bottom of the tank, and reaction buffer containing the digested linearized template DNA product may be moved off via an outlet attached at the top of the tank. Inlet and outlet flow may be controlled by a pumping device in such a way that the enzymatic reaction can occur. Moreover, outlet tubes may have molecular weight cutoff filters to avoid contamination of the product by immobilized restriction endonuclease or plasmid DNA. One advantage of such an embodiment is that the immobilized restriction endonuclease does not have to be separated from the linearized template DNA product by other means. The purified linearized template DNA may be used for *in vitro* transcription of RNA.

Particular examples of enzyme reactors according to the present invention are provided in Figures 9 and 10.

Optionally, the enzyme reactor comprises
i) a linearization module (1 or 10) for carrying out linearization reactions;
ii) a capture module (13) for temporarily capturing the linearized plasmid DNA; and
iii) a feed module (12) for controlling the feed of components of a reaction mix into the linearization module (1 or 10); and optionally
iiii) an RNA *in vitro* transcription (IVT) module (15) for the *in vitro* transcription of RNA from the linearized DNA template generated in the linearization module (1, 10).

The different modules of the enzyme reactor are connected to each other so that for example the product of the linearization reaction can be transferred to the IVT module, optionally via the capture module. However, the enzyme reactor is configured such that the reactions in the different modules can be performed independently of each other and the reaction mixtures in the different modules are not mixed.

The IVT module (15) and the feed module (12) may have a cooling device. Furthermore, all components of the feed module (12) are optionally heated to reaction temperature (heater) before they are fed into the linearization module via an inlet tube.

The IVT module (15) preferably comprises all components necessary for performing an RNA *in vitro* transcription from a linear DNA template (e.g., RNA polymerase, nucleotide mixture etc.).

According to a preferred embodiment of the present invention, the enzyme reactor comprises at least one sensor unit. Data collection and analyses by the at least one sensor unit allows the control of the integrated pump system (actuator) for repeated feeds of components of the reaction mix, e.g. buffer components or nucleotides (e.g., ATP) (Pump: 14).

The linearization module further comprises a filtration membrane (6) to separate the linear DNA template (11) from the reaction mix and to subsequently collect the linear DNA template in the capture module (13). The introduction of a filtration membrane in a flow system, for example an ultrafiltration membrane, is used for separation of high molecular weight components, such as e.g. immobilized or non-immobilized enzymes and/or polynucleotides.

Suitable filtration membranes may consist of various materials known to a person skilled in the art (van de Merbel, 1999. J. Chromatogr. A 856(1-2):55-82). For example, membranes may consist of regenerated or modified cellulose or of synthetic materials. The latter include polysulfone (PSU), polyacrylo-nitrile (PAN), polymethylmethacrylate (PMMA), mixtures of polyarylether-sulfones, polyvinylpyrrolidone and polyamide (Polyamix, RTM). For example, the polysulfones include polyethersulfone (poly(oxy-1,4-10 phenylsulfonyl-1,4-phenyl), abbreviated PES). In some exemplary embodiments, polyethersulfone may be utilized as a semipermeable membrane. In some cases PES membranes include increased hydrophilicity (and/or the improved wettability of the membrane with water) compared to PSU membranes. In some embodiments, the wettability of PES membranes can, for example, be further increased by the inclusion of the water-soluble polymer polyvinylpyrrolidone.

An important parameter that influences the flux of molecules across the filtration membrane is the pore size or pore-size distribution. A filtration membrane is usually characterized by its molecular weight cut-off (MWCO) value, i.e. a specific size limitation, which is defined as the molecular mass of the smallest compound, which is retained for more than 90%. For each application, a proper MWCO value needs to be selected so that high molecular weight compounds are sufficiently retained, but at the same time a rapid transport of the analyte is ensured. The filtration membrane of the filtration unit (6) may be an ultrafiltration membrane, and preferably has a molecular weight cut-off in a range from 10 to 500 kDa, 10 to 400 kDa, 10 to 300 kDa, 10 to 200 kDa or 10 to 100 kDa, further preferably the filtration membrane has a molecular weight cut-off in a range of 50 to 500 kDa, further preferably in a range of 100 to 500 kDA. In a specific embodiment the MWCO is 300 kDA. Optionally, the filtration membrane is selected from the group consisting of regenerated cellulose, modified cellulose, PES, PSU, PAN, PMMA, polyvinyl alcohol (PVA) and polyarylethersulfone (PAES).

The capture module (13) optionally comprises a resin to capture the produced linear template DNA and to separate produced linear template DNA from other soluble components of the reaction mix. Optionally, the capture module (13) comprises a sensor unit to measure the concentration of the produced linear template DNA, means for purifying the linear template DNA and/or means for eluting the captured linear template DNA, preferably by means of an elution buffer.

In a preferred embodiment, the enzyme reactor further comprises a re-circulation pipeline re-circulation pipeline (8) for optionally returning the filtrated reaction mix (comprising linear template DNA) back to the enzyme batch reactor via the feed module (12) until a desired linearization degree is obtained.

In a preferred embodiment, the enzyme reactor further comprises several sensor units which may be present in the linearization module (1 or 10), the capture module (13), the feed module (12) or the *in vitro* transcription module (15). The sensor units are suitable for the real-time measurement of the concentration of separated nucleic acid molecules, the concentration of nucleoside triphosphates, and/or further reaction parameters, such as pH value, reactant concentration, in- and out-flow, temperature and/or salinity, optionally, the said sensor units measure the concentration of separated nucleic acids by photometric analysis.

According to some embodiments, the enzyme reactor, more specifically, the sensor unit comprises at least one ion-selective electrode, preferably for measuring the concentration of one or more types of ions in a liquid comprised in at least one compartment of the enzyme reactor, wherein the ion is preferably selected from the group consisting of H⁺, Na⁺, K⁺, Mg²⁺, Ca2⁺, Cl⁻ and PO₄³⁻.

In the context of the present invention, the term "ion-selective electrode" relates to a transducer (e.g. a sensor) that converts the activity of a specific ion dissolved in a solution into an electrical potential, wherein the electrical potential may be measured, for instance, by using a volt meter or a pH meter. In particular, the term 'ion-selective electrode' as used herein comprises a system which comprises or consists of a membrane having selective permeability, wherein the membrane typically separates two electrolytes. An ion-selective electrode as used herein typically comprises a sensing part, which preferably comprises a membrane having selective permeability and a reference electrode. The membrane is typically an ion-selective membrane, which is characterized by different permeabilities for different types of ions. Preferably, the at least one ion-selective electrode of the enzyme reactor comprises a membrane selected from the group consisting of a glass membrane, a solid state membrane, a liquid based membrane, and a compound membrane.

In preferred embodiments, the at least one ion-selective electrode comprises or consists of a system comprising a membrane, preferably a membrane as described herein, more preferably an electrochemical membrane, having different permeabilities for different types of ions, wherein the membrane, preferably a membrane as described herein, more preferably an electrochemical membrane, preferably separates two electrolytes. In one embodiment, the membrane comprises or consists of a layer of a solid electrolyte or an electrolyte solution in a solvent immiscible with water. The membrane is preferably in contact with an electrolyte solution on one or both sides. In a preferred embodiment, the ion-selective electrode comprises an internal reference electrode. Such internal reference electrode may be replaced in some embodiments, for example by a metal contact or by an insulator and a semiconductor layer. An ion-selective electrode permits highly sensitive, rapid, exact and non-destructive measurement of ion activities or ion concentrations in different media. Apart from direct measurements of ion activities or ion concentrations they can serve, in particular by using a calibration curve, for continuous monitoring of concentration changes, as elements for control of dosage of agents or as very accurate indicator electrodes in potentiometric titrations.

In preferred embodiments, the enzyme reactor comprises at least one ion-selective electrode, preferably as described herein, for measuring the concentration of one or more types of ions in at least one compartment of the enzyme reactor.

Preferably, the at least one ion-selective electrode is connected to a potentiometer, preferably a multi-channel potentiometer (for instance, a CITSens Ion Potentiometer 6-channel, high-20 resolution; C-CIT Sensors AG, Switzerland). In a preferred embodiment, the at least one ion-selective electrode is preferably a tube electrode, more preferably selected from the group consisting of a Mg²⁺ selective tube electrode, a Na⁺ selective tube electrode, a Cl⁻ selective tube electrode, a PO₄³⁻ selective tube electrode, a pH-selective tube electrode and a Ca²⁺ selective tube electrode, preferably used in connection with a potentiometer. Even more preferably, the enzyme reactor (1) comprises at least one ion-selective electrode, wherein the at least one ion-selective electrode is preferably selected from the group consisting of a CITSens Ion Mg²⁺ selective mini-tube electrode, a CITSens Ion Na⁺ selective mini-tube electrode, a CITSens Ion Cl⁻ selective mini-tube electrode, a CITSens Ion PO₄³⁻ selective mini-tube electrode, a CITSens Ion pH-selective mini-tube electrode and a CITSens Ion Ca²⁺ selective mini-tube electrode (all from C-CIT Sensors AG, Switzerland), preferably in connection with a potentiometer, more preferably with a multi-channel potentiometer, such as a CITSens Ion Potentiometer 6-channel, high-resolution (C-CIT Sensors AG, Switzerland).

Ion-selective electrodes have numerous advantages for practical use. For example, they do not affect the tested solution, thus allowing non-destructive measurements. Furthermore, ion-selective electrodes are mobile, suitable for direct determinations as well as titration sensors, and cost effective. The major advantage of the use of an ion-selective electrode in an enzyme reactor (e.g. a polyadenylation reactor) is the possibility to measure *in situ* without sample collection and in a non-destructive manner.

The ion-selective electrodes allow very specifically to monitor the linearization reaction, and in particular the reaction catalyzed by the immobilized restriction endonuclease according to the invention.

The sensor units may further be equipped for the analysis of critical process parameters, such as pH-value, conductivity and nucleotide concentration in the reaction mix. Preferably, the sensor unit of the IVT module (15) comprises a sensor, such as an UV flow cell for UV 260/280nm, for the real-time measurement of the nucleotide concentration during the *in vitro* transcription reaction. Preferably, the sensor of the sensor units measures the nucleotide concentration, as a process parameter, by photometric analysis.

In addition to the described online measurements of the sensor units, the same measurements may be performed in separate analysis modules (as at-line controls). E.g., the progress of the linearization reaction may be analyzed at-line via gel electrophoresis, photometry etc.

In a preferred embodiment, the enzyme reactor as described above additionally comprises a reaction unit for the simultaneous production of RNA from a DNA template via enzymatic RNA *in vitro* transcription.

In embodiments where the linearization reaction and the RNA *in vitro* reaction take place in one reactor and/or in one reaction unit, it is particularly preferred to use immobilized restriction endonucleases that are immobilized onto a solid support via thioether linkages (R-S-R), because common reaction buffers of RNA polymerases contain reducing agents (e.g., DTT), that may reduce disulfide bonds (which would cause an enzyme leakage), but do not reduce thioether linkages.

Further provided is a kit comprising a restriction endonuclease which is immobilized onto a solid support, preferably as described herein, and at least one buffer selected from the group consisting of reaction buffer, storage buffer and combinations thereof.

In one embodiment of that aspect of the invention, the kit additionally comprises an RNA polymerase and a suitable buffer for RNA *in vitro* transcription.

In preferred embodiments of this aspect, the RNA produced according to the present invention may be used in gene therapy, (genetic) vaccination or immunotherapy.

### Examples

The Examples shown in the following are merely illustrative and shall describe the present invention in a further way. These Examples shall not be construed to limit the present invention thereto.

In the examples the following plasmid DNA is used for digestion with the restriction endonucleases:
P1040 according to SEQ ID No. 18 coding for the RNA PpLuc (GC)GA- -A64-C30-histone stem-loop N5 according to SEQ ID No. 19. The corresponding plasmid map is shown in figure 11.

### Example 1: Immobilization of EcoRI on Thiopropyl Sepharose 6B

The goal of this proof-of-principle experiment was to obtain stably immobilized and functional restriction endonuclease. *Escherichia coli* EcoRI mutants were generated, mutant 1 (referred to as "mut1"), mutant 2 (referred to as "mut2") and mutant 3 (referred to as "mut3") and characterized for enzymatic activity. As immobilization strategy, immobilization onto Thiopropyl Sepharose 6B solid supports was tested. The reaction conditions, respectively the pH, were chosen such that the formation of disulfide linkages (R-S-S-R) via sulfhydryl groups (-SH) of cysteine residues present on the EcoRI mutant proteins was promoted. The obtained immobilized EcoRI mutants were further characterized and tested for their enzymatic activity, i.e. the ability to cut a suitable substrate. A detailed description of the immobilization procedure and a discussion of the results are provided below.

### 1.1. Design of Escherichia coli EcoRI mut1 and mut2 and mut3 proteins:

The three EcoRI mutants (mut1, according to SEQ ID No: 23; mut2, according to SEQ ID No: 31; mut3, according to SEQ ID No: 32) were generated by c-LEcta (codon optimization, gene synthesis, sub cloning, protein expression, protein purification via a C-terminal 6H-tag).

All mutant enzymes were engineered to comprise a C-terminal extension comprising an oligohistidine purification tag (6H-tag, HIS-tag) and a C-terminal-most cysteine residue. In addition, sequences of the mut2 and mut3 enzyme comprise a C-terminal flexible linker element (GGGGSGGGGS). EcoRI mut3 was changed in a way that the natural cysteine residue was substituted with an alanine residue (C128A). The protein design of mut3 was chosen to allow for a directed way of immobilization, exclusively facilitated via the C-terminal-most cysteine residue.

### 1.2. Initial activity characterization of engineered mutant EcoRI proteins:

To characterize the enzymatic activity of the engineered mutant EcoRI proteins mut1, mut2 and mut3, a linear double stranded DNA was used as template (*E.coli* phage lambda DNA, 48.5 kb; comprising five predicted EcoRI restriction sites) was digested with each of the mutant EcoRI proteins. 1 µg lambda DNA was digested for 1h at 37°C with different dilutions of the recombinant mutant enzymes mut1, mut2, mut3 in a commercially available 1x EcoRI buffer (Thermo Scientific, ER0275). Commercially available EcoRI enzyme (Thermo Scientific, ER0275) was used as positive control. Afterwards, digestion was monitored on a 1% agarose gel via gel electrophoresis (AGE, performed according to protocols as generally known in the art) (see **Figure 12**).

### 1.3. Re-buffering of recombinant EcoRI mut1, mut2 and mut3 proteins:

The obtained purified recombinant EcoRI mutant proteins were reconstituted in storage buffer (10mM KPO₄,pH 7.5, 500 mM NaCl, 1 mM EDTA, 0.2% Triton X-100). For immobilization experiments, recombinant mutant EcoRI proteins were re-buffered in immobilization buffer (100 mM K₂HPO₄-KH₂PO₄, pH 7.5, 500 mM NaCl, 1mM EDTA) using Vivaspin-20 (10 kDa molecular weight cutoff (MWCO); Sartorius) columns to obtain a final protein concentration of 1 µg/ul (determined by the Qubit Protein Assay Kit). The obtained re-buffered EcoRI mutants mut1, mut2 and mut3 were used in immobilization experiments (see below).

### 1.4. Re-buffering of Thiopropyl Sepharose 6B:

After swelling of 3g freeze-dried Thiopropyl Sepharose 6B beads (GE Healthcare, 17-0420-01) in water for injection (WFI) and subsequent pelletizing, the Thiopropyl Sepharose 6B beads were filled up to 24g with WFI. For immobilization experiments, the beads were thoroughly washed with immobilization buffer, pelletized, and supernatant was discarded. The obtained re-buffered beads were used in immobilization experiments (see below).

### 1.5. Immobilization procedure:

0.45 mg recombinant re-buffered EcoRI mutant proteins were transferred into immobilization buffer comprising re-buffered Thiopropyl Sepharose 6B beads (referred to as "TS") in a total volume of 10 ml and rotated for 1h at room temperature to allow immobilization. To monitor the course of immobilization, samples were taken from the supernatant during immobilization reaction (0', 15'; see **Figure 13**). The immobilized EcoRI mutants (referred to as "EcoRI-TS mut1", "EcoRI-TS mut2" and "EcoRI-TS mut3") were pelletized and the supernatants were stored at 4-8°C. EcoRI-TS mutants were thoroughly washed with storage buffer and stored in storage buffer at 4-8°C.

### 1.5. Activity test of immobilized EcoRI mutants (EcoRI-TS mut1, EcoRI-TS mut2, EcoRI-TS mut3):

To test the obtained immobilized EcoRI-TS variants for their enzymatic activity, 1 µg lambda-DNA and 1µg of plasmid DNA (SEQ ID No. 18; see Figure 11) were digested using different dilutions of the EcoRI-TS mut1, EcoRI-TS mut2 and EcoRI-TS mut3 beads (37°C, 1h). A 1% AGE was performed to assess enzymatic activity compared to commercially available soluble EcoRI enzyme. The result is shown in **Figure 14****.**

### Results and discussion:

As shown in **Figure 12****,** all three engineered soluble EcoRI mutants (mut1, mut2, mut3) are functional, showing a comparable enzymatic activity. All mutant enzymes were able to linearize a large 48.5 kb DNA template into the predicted fragments (21.225 bp, 7.421 bp, 5.804 bp, 5.643 bp, 4,878 bp, 3.530 bp). All three EcoRI mutant generated the five expected digestion products up to a dilution factor of the enzyme of 10⁻⁴. Moreover, a weak activity was observed for the dilution factor 10⁻⁵. The data suggests that all engineered mutant *E.coli* EcoRI proteins are active in solution. The data also implies that the cysteine residues C218 is not crucial for the activity of the enzyme (in mut3 this cysteine has been substituted with alanine). Moreover the data shows that the addition of a C-terminal element comprising a linker and/or a HIS-tag and a C-terminal most Cysteine does not impede enzymatic activity. These results suggest that the inventive engineering of *E. coli* EcoRI may be broadly transferrable to generate other functional restriction endonuclease mutants, e.g. EcoRI mutants according to SEQ ID Nos. 4-9, 15-17, 20-22, 24-30 and 33-34.

As shown in **Figure 13****,** all three engineered soluble EcoRI mutants (mut1, mut2, mut3) enzymes could be successfully immobilized on Thiopropyl Sepharose 6B beads ("TS"). The data suggest that the method of immobilization is broadly applicable also to other restriction endonucleases comprising at least one cysteine residue (either natural or introduced).

As shown in **Figure 14****,** all three immobilized EcoRI mutants (EcoRI-TS mut1, EcoRI-TS mut2, EcoRI-TS mut3) are functional and enzymatically active. However, differences in activity were observed. EcoRI-TS mut1 (EcoRI mutant enzyme lacking a flexible C-terminal linker element) was at least 10 times less active than the mutants comprising a C-terminal flexible linker element (incomplete digestion of lambda DNA in **Figure 14A** and incomplete linearization of plasmid DNA in **Figure 14B** indicated by asterisks). For the interpretation of the data it has to be emphasized that a complete digestion of lambda DNA leads to 5 distinct bands **(****Figure 14A****),** and a complete linearization of the plasmid DNA leads to one band **(****Figure 14B****).**

Overall, this data suggests that an immobilization of EcoRI via flexible linker elements results in more active EcoRI-TS beads. This data implies that the use of linker elements is beneficial for EcoRI activity. In this context, also other restriction endonuclease mutant enzymes comprising a linker element may lead to functional immobilized enzymes (e.g., according to SEQ ID Nos. 7-9, 21, 27-30, 33-34). Also, it has to be noted, that other linker elements may also be used (e.g., according to SEQ ID Nos. 35-59).

### Example 2: Use of EcoRI-TS beads in a linearization reactor for production of in vitro transcribed RNA

The EcoRI-TS beads obtained according to **Example 1** were used in a prototype linearization reactor to test the suitability of the EcoRI-TS beads in a reactor setting. Moreover, the generated linear DNA was used as a template for RNA *in vitro* transcription.

### 2.1. Design of the prototype linearization reactor:

The reactor setup was essentially according to Figure 10, comprising a syringe pump (14), a feeding module (feed syringe, 12), a packed bed tank reactor comprising the EcoRI-TS beads (10), and a capture module (collector syringe, 13). The reactor was operated with a bed volume of 200 µl and a feed flow of 10 µl/min. In the tested setup (see below), 1 µg of plasmid DNA was digested in a theoretical total incubation time of 20 minutes.

### 2.2. Test of EcoRI-TS beads in a prototype linearization reactor:

Suspensions of EcoRI-TS variants were transferred to the reactor tube via syringe.

Subsequently, the reactor was washed with commercially available 1xEcoRI buffer (5x bed volume) for 5 minutes (200µl/min). The flow-through was collected for protein analysis. Then, digestion solution (100µl p1040 in 1xEcoRI buffer) was pumped via syringe in the reactor tube with a flow rate of 10µl/min. The flow-through was collected for protein and plasmid DNA analysis. Afterwards the reactor was again washed with commercially available 1xEcoRI buffer (5x bed volume) for 5 minutes (200µl/min). The flow-through was collected for protein and plasmid DNA analysis. To confirm plasmid DNA linearization by EcoRI-TS mutants, 1% AGE was performed (see **Figure 15**). To assess the stability of EcoRI-TS binding, the flow-through samples were analysed via SDS-PAGE and subsequent silver staining (see **Figure 16**). The linearized DNA was used in an RNA *in vitro* transcription reaction.

### 2.3 RNA in vitro transcription using a DNA template generated in the linearization reactor:

Linearized DNA obtained from the linearization reactor was used as template in a subsequent RNA *in vitro* transcription. 1µg linearized DNA was incubated with 4.000 Units T7 RNA polymerase, 20mM Cap analog, 20 mM MgCl₂ and 20mM sequence optimized NTP mix for 2h at 37°C. The reaction was stopped by adding 0.1M CaCl₂ and 0.2 µl DNase was added for 1h at 37°C. The *in vitro* transcribed RNA was analyzed on 1% AGE **(****Figure 17A****).** To assess the integrity as well as the purity of the obtained *in vitro* transcribed RNA, a IPRP-HPLC analysis was performed (PLRP-S 4000 A 5µM 50x4.6 mM, Part-No: PL1512-1503). The results of the HPLC are shown in **Figure 17B****.**

### Results and discussion:

As shown in **Figure 15****,** the plasmid DNA was successfully linearized in a linearization reactor using immobilized EcoRI restriction enzymes (EcoRI-TS mut1-3). This data shows that the generated EcoRI-TS beads are suitable for use in an enzyme reactor. EcoRI-TS mut1 (EcoRI mutant enzyme lacking a flexible C-terminal linker element) was less active than the mutants comprising a C-terminal flexible linker element, because also other bands are visible on the agarose gel (Figure 15, lane 3). This data suggests that an immobilization of EcoRI via flexible linker elements (as performed for EcoRI-TS mut2 and 3) results in more active EcoRI-TS beads.

As shown in **Figure 16****,** the immobilization strategy (see Example 1) of the present invention led to the production of stable EcoRI-TS beads. After analysis of the flow-through sample on a silver stained SDS protein gel, no protein leakage could be observed. This data suggests that the EcoRI-TS beads described herein are stable and active, and therefore, suitable for the use in a bioreactor setting.

As shown in **Figure 17****,** RNA was successfully generated via RNA *in vitro* transcription using the linearized DNA from the linearization reactor. However, differences were observed regarding the purity of the RNA product. The RNA generated from the EcoRI-TS mut1 (EcoRI mutant enzyme lacking a flexible C-terminal linker element) linearized DNA template showed also larger RNA byproducts (see arrows in Figure 17B, lane 2). This is very likely due to incomplete linearization (cf. Figure 15).

Overall, the results show that the inventive EcoRI-TS beads are suitable for use in a linearization reactor. It has been observed that a C-terminal linker element is beneficial for enzyme activity, the quality of the plasmid DNA linearization, and hence, the quality of the *in vitro* transcribed RNA.

### Example 3:

### EcoRI (C218A 12xG linked Cys at position 290) immobilized on Thiol Sepharose 4B in a continuous packed bed reactor

Recombinant EcoRI having a C218A substitution and a cysteine residue linked to the C-terminus via a 12xG linker according to SEQ ID No. 8 was obtained from Genscript, Piscataway, USA. 3 mg of this enzyme were transferred to 10 ml coupling buffer (0.1 M Tris-HCl pH 7.5, 0.5 M NaCl, 1 mM EDTA). EDTA is added to the buffer to remove trace amounts of heavy metal ions, which may catalyze the oxidation of thiols. De-gassing of the buffer was performed to avoid oxidation of free thiol groups. The final concentration of the protein in coupling buffer is 300 µg/ml.

### Coupling of mutant EcoRI to Thiol Sepharose 4B HiTrap columns (GE Healthcare)

Recombinant EcoRI was coupled to HiTrap columns that had been pre-packed with 5 ml bed volumes of activated Thiol Sepharose 4B (agarose-(glutathione-2-pyridyl disulfide); GE Healthcare) corresponding to 5 µmol of activated thiol groups. The Thiol Sepharose 4B HiTrap column was connected to an input and an output tank. The flow was adjusted to 5 cm/h using a peristaltic pump. Moreover, the output-tank was connected to the input-tank to optionally facilitate continuous flow in a closed continuous flow system (setup illustrated in Figure 10).

First, the column was washed 3 times with coupling buffer, with a 10-fold excess of buffer to resin bed volume. Then, the EcoRI solution was used for coupling (5 ml 4B Thiol Sepharose resin corresponds to a molar ratio of enzyme to resin's thiol groups of approximately 1:10). With a flow-through rate of approximately 5 cm/h, coupling was allowed to happen for 2 hours. After coupling occurred, the column was washed three times with coupling buffer at a 10-fold excess of buffer to resin bed volume. After washing, the flow-through was analyzed for trace protein using a NanoDrop 2000 at an absorbance wavelength of 280 nm. Additionally, coupling efficiency was directly measured as the release of thiopyridone (a by-product of enzyme coupling to the activated thiolated support), monitored at 343 nm. If coupling efficiency was less than desired, the flow-through was recycled from the output tank into the input tank onto the column for additional rounds to achieve the desired coupling efficiency (>50 %). Next, excess reactive sites were blocked by washing the resin with 50 mM cysteine (in coupling buffer) for 30 min, followed by three additional washes with 25 ml coupling buffer/Triton-X (0.1 M Tris-HCl pH 7.5, 0.5 M NaCl, 1 mM EDTA, 0.5% Triton X-100).

### Loading of plasmid DNA into the linearization reactor

The plasmid DNA was linearized on the column by firstly equilibrating the resin with 10 bed volumes of 1 x EcoRI digestion buffer (100 mM Tris-HCl, 50 mM NaCl, 10 mM MgCl₂, 0.025% Triton X-100, pH 7.5) followed by adding the plasmid DNA (DNA) according to SEQ ID No. 18 onto the column in 1 x EcoRI digestion buffer (DNA concentration: 100 µg/ml) at a flow rate of 0.1 ml/min. As a quality-control measure, cutting activity within different flow-through fractions was monitored by fast capillary DNA electrophoresis. Optionally, if linearization efficiency was lower than desired, the flow-through was recycled from the output tank into the input tank onto the column for additional rounds to achieve the desired linearization efficiency (>99%). The final flow-through containing the linear DNA was subjected to ultrafiltration methods to remove buffer components and to replace it with ultra-pure water. Efficiency of linearization was controlled by agarose gel electrophoresis.

Afterwards, linearized template DNA at a concentration of 1 mg/ml was directly used as template for *in vitro* transcription reactions.

### Cleaning and re-use of the linearization reactor

After digestion, the linearization reactor was washed several times with coupling buffer and incubated in EcoRI storage buffer (10mM Tris-HCl pH7.4, 10 mM KPO₄, 300 mM NaCl, 0.1 mM EDTA, 0.15% Triton X-100, pH 7.5). Following that, a new plasmid DNA solution can be loaded on the EcoRI linearization reactor. Alternatively, the EcoRI linearization reactor can be stored at 4°C for several weeks in EcoRI storage buffer.

### In vitro transcription:

For *in vitro* transcription 2 µg of linearized template DNA were incubated in 80 mM HEPES, 24 mM MgCl₂, 2 mM spermidine, 40 mM DTT, 4 U RNAsin/µg DNA, 4 mM ATP, 4 mM CTP, 4 mM UTP, 1.45 mM GTP, 5,8 mM CAP, 100 U T7 polymerase/µg DNA, and 5 U pyrophosphatase/ml for 2 hours at 37°C. Subsequently, the plasmid DNA was digested by adding 12 µl DNAse 1(1 U/µl) and 0,4 µl CaCl₂ (0.1 M) to the *in vitro* transcription reaction mixture, mixing and incubating it for 1 hour at 37°C.

After DNAse I digestion LiCl precipitation was performed.. The pellet was resuspended in 50 µl water for injection (WFI). The RNA concentration was determined by photometry and the RNA was detected by RNA agarose gel electrophoresis.

### Example 4:

### EcoRI (C218A K277C) immobilized on Thiol Sepharose 4B in a batch reactor

Recombinant EcoRI (C218A, K277C) restriction endonuclease according to SEQ ID No. 5 was obtained from Genscript, Piscataway, USA. 3 mg of this enzyme were directly transferred to 10 ml coupling buffer (0.1 M Tris-HCl pH 7.5, 0.5 M NaCl, 1 mM EDTA). EDTA is added to the buffer to remove trace amounts of heavy metal ions, which may catalyze oxidation of thiols. De-gassing of the buffer was performed to avoid oxidation of free thiol groups. The final concentration of the protein solution in coupling buffer was 300 µg/ml. This enzyme solution was used for coupling to Thiol Sepharose 4B (GE Healthcare).

### Column preparation with Thiol Sepharose 4B

First, 3 g freeze-dried Thiol Sepharose 4B was suspended in 20 ml ddH₂O and gently added to a 20 ml resin column. After washing the resin 10 times with 20 ml ddH₂O, the Thiol Sepharose resin was equilibrated 3 times with 10 ml coupling buffer for 10 minutes and the bottom of the column was closed. After flow-through of the coupling buffer, the column was closed and 10 ml of enzyme solution were added. Coupling of EcoRI to the resin was allowed to take place for 2 h at room temperature. After coupling, the column was washed three times with 50 ml coupling buffer. The flow-through was analyzed for trace protein using a NanoDrop 2000 at an absorbance wavelength of 280 nm. Additionally, coupling efficiency was directly measured as the release of thiopyridone (a by-product of enzyme coupling to the activated thiolated support), monitored at 343 nm. Next, excess reactive sites were blocked by washing the resin with 50 mM cysteine (in coupling buffer) for 30min, followed by three additional washes with 25 ml coupling buffer/Triton-X (0.1 M Tris-HCl pH 7.5, 0.5 M NaCl, 1 mM EDTA, 0.5% Triton X-100) to remove weakly coupled proteins.

### Loading of plamid DNA to the linearization reactor

Next, the resin was equilibrated three times with 15 ml 1 x EcoRI digestion buffer (100 mM Tris-HCl, 50 mM NaCl, 10 mM MgCl₂, 0.025% Triton X-100, pH 7.5) for 10 minutes. After flow-through of the buffer, 10 ml purified plasmid DNA in 1x EcoRI digestion buffer (DNA concentration: 100 µg/ml) was added and incubated for 1h at 25°C. Cutting activity within different flow-through fractions was monitored by fast capillary DNA electrophoresis. Flow-through containing the linear DNA was subjected to ultrafiltration methods to remove digestion buffer components and to replace it with ultra-pure ddH₂O water. Efficiency of linearization was controlled by agarose gel electrophoresis. Afterwards, linearized template DNA at a concentration of 1 mg/ml was directly used as template for *in vitro* transcription reactions.

### Cleaning and re-use of the linearization reactor

After digestion occurred, the linearization reactor is washed several times with coupling buffer and incubated in EcoRI storage buffer (10mM Tris-HCl pH7.4, 10 mM KPO4, 300 mM NaCl, 0.1 mM EDTA, 0.15% Triton X-100, pH 7.5). Following that, a new plasmid DNA solution can be loaded on the EcoRI linearization reactor. Alternatively, the EcoRI linearization reactor can be stored at 4°C for several weeks in EcoRI storage buffer.

### In vitro transcription:

The *in vitro* transcription reaction was performed as described in Example 1.

### Example 5: BciIV (C271S, 359C) immobilized on Thiol Sepharose 4B in a batch reactor

### Reconstitution of recombinant BciIV

Recombinant BciIV (C271S, 359C) restriction endonuclease according to SEQ ID No. 14 was obtained from Genscript, Piscataway, USA. 3 mg of this enzyme were transferred to 10 ml coupling buffer (0.1 M Tris-HCl pH 7.5, 0.5 M NaCl, 1 mM EDTA). EDTA is added to the buffer to remove trace amounts of heavy metal ions, which may catalyze oxidation of thiols. De-gassing of the buffer was performed to avoid oxidation of free thiol groups. The final concentration of the protein solution in coupling buffer was 300 µg/ml. The enzyme solution was then used for coupling to Thiol Sepharose 4B (GE Healthcare).

### Column preparation with Thiol Sepharose 4B

First, 3 g freeze-dried Thiol Sepharose 4B was suspended in 20 ml ddH₂O and gently added to a 20 ml resin column. After washing the resin 10 times with 20 ml ddH₂O, the Thiol Sepharose resin was equilibrated 3 times with 10 ml coupling buffer for 10 minutes and the bottom of the column was closed. After flow-through of the coupling buffer, the column was closed and 10 ml of enzyme solution were added. Coupling of BciIV to the resin was allowed to take place for 2 h at room temperature. After coupling, the column was washed three times with 50 ml coupling buffer. The flow-through was analyzed for trace protein using a NanoDrop 2000 at an absorbance wavelength of 280 nm. Additionally, coupling efficiency was directly measured as the release of thiopyridone (a by-product of enzyme coupling to the activated thiolated support), monitored at 343 nm. Next, excess reactive sites were blocked by washing the resin with 50 mM cysteine (in coupling buffer) for 30min, followed by three additional washes with 25 ml coupling buffer/Triton-X (0.1 M Tris-HCl pH 7.5, 0.5 M NaCl, 1 mM EDTA, 0.5% Triton X-100) to remove weakly coupled proteins.

### Loading of plasmid DNA to the linearization reactor

Next, the resin was equilibrated with 15 ml 1 x BciVI digestion buffer (50 mM Tris-acetate (pH 7.9), 15 mM magnesium acetate, 100 mM potassium acetate) for 10 minutes. After flow-through of the buffer, 10 ml purified plasmid DNA in 1x BciIV digestion buffer (DNA concentration: 100 µg/ml) were added and incubated for 1h at 25°C. Cutting activity within different flow-through fractions was monitored by fast capillary DNA electrophoresis. Flow-through containing the linear DNA was subjected to ultrafiltration methods to remove digestion buffer components and to replace it with ultra-pure ddH₂O water. Efficiency of linearization was controlled by agarose gel electrophoresis. Afterwards, linearized template DNA at a concentration of 1 mg/ml was directly used as template for *in vitro* transcription reactions.

### Cleaning and re-use of the linearization reactor

After digestion occurred, the linearization reactor was washed several times with coupling buffer and incubated in BciIV storage buffer (10 mM Tris-HCl (pH 7.4 at 25°C), 300 mM NaCl, 1 mM DTT, 1 mM EDTA) . Following that, a new plasmid DNA template can be loaded on the BciIV linearization reactor. Alternatively, the BciIV linearization reactor can be stored at 4°C for several weeks in BciIVstorage buffer.

### In vitro transcription:

The *in vitro* transcription reaction was performed as described in Example 1.

## Claims

1. Method for *in vitro* transcription of a linear template DNA, comprising the steps of:
a) contacting a plasmid DNA having at least one recognition sequence for a restriction enzyme with said restriction enzyme, wherein the restriction enzyme is immobilized to a solid support, thereby linearizing the plasmid DNA and providing the linear template DNA; and
b) transcribing the linear template DNA into RNA *in vitro*, and
c) obtaining the resulting RNA.

2. Method according to claim 1, wherein the restriction enzyme is a type II restriction enzyme, preferably wherein the restriction enzyme is selected from the group consisting of EcoRI, BciVI, XbaI, NdeI, AflII, SacI, KpnI, SmaI, BamHI, XbaI, SalI, SbfI, PstI and HindIII.

3. Method according to claim 1 or 2, wherein the restriction enzyme is immobilized by covalent binding, preferably wherein the covalent binding is a disulfide bridge or a thioether bond.

4. Method according to claim 3, wherein the restriction enzyme is immobilized by covalent binding to a thiol-activated support material, haloacetyl functionalized solid support, pyridyl disulfide-functionalized solid support, maleimide-activated solid support, epoxy activated solid support or a mixture thereof.

5. Method according to claim 4, wherein the restriction enzyme is bound to the thiol-activated support material via a thiol group of at least one cysteine residue, preferably wherein the at least one cysteine residue which is bound to the thiol-activated support material is not present in the wild-type restriction enzyme.

6. Method according to claim 5, wherein the at least one cysteine residue which is bound to the thiol-activated support material has been introduced by substituting an amino acid within the wild-type restriction enzyme with cysteine or by inserting a cysteine into the wild-type restriction enzyme, preferably by adding a cysteine residue to the N or C terminus of the restriction enzyme.

7. Method according to any of the preceding claims, wherein the restriction enzyme is selected from the group consisting of:
a) EcoRI according to SEQ ID No. 1, wherein the lysine on position 277 of SEQ ID No. 1 is replaced with cysteine or a functional variant of EcoRI having at least 80% sequence identity to SEQ ID No. 1, wherein the lysine on a position corresponding to position 277 of SEQ ID No. 1 is replaced with cysteine;
b) EcoRI according to SEQ ID No. 1 or a functional variant thereof having at least 80% sequence identity to SEQ ID No. 1, wherein a cysteine is added to the C-terminus, preferably via a linker;
c) HindIII according to SEQ ID No. 2, wherein the leucine on position 300 of SEQ ID No. 2 is substituted with cysteine or a functional variant of HindIII having at least 80% sequence identity to SEQ ID No. 2, wherein the leucine on a position corresponding to position 300 of SEQ ID No. 2 is substituted with cysteine;
d) HindIII according to SEQ ID No. 2, wherein a cysteine residue is added to the C-terminus, preferably via a linker, or a functional variant of HindIII having at least 80% sequence identity to SEQ ID No. 2, wherein a cysteine residue is added to the C-terminus, preferably via a linker; and
e) BciVI according to SEQ ID No. 3, wherein a cysteine residue is added to the C-terminus, preferably via a linker or a functional variant thereof having at least 80% sequence identity to SEQ ID No. 3, wherein a cysteine residue is added to the C-terminus, preferably via a linker.

8. Method according to claim 7, wherein the linker has the sequence GGGGSGGGGS (SEQ ID No. 49).

9. Method according to any of claims 6 to 8, wherein, if present, one or more cysteine residues in the wild-type restriction enzyme are replaced with another amino acid.

10. Method according to claim 9, wherein the restriction enzyme is selected from the group consisting of:
a) EcoRI according to SEQ ID No. 1, wherein the cysteine position 218 of SEQ ID No. 1 is replaced with another amino acid or a functional variant of EcoRI having at least 80% sequence identity to SEQ ID No. 1, wherein the cysteine on a position corresponding to position 218 of SEQ ID No. 1 is replaced with another amino acid; or
b) BciVI according to SEQ ID No. 3, wherein the cysteine on position 271 of SEQ ID No. 3 is replaced with another amino acid or a functional variant of BciVI having at least 80% sequence identity to SEQ ID No. 3, wherein the cysteine on a position corresponding to position 271 of SEQ ID No. 3 is replaced with another amino acid.

11. Method according to any of the preceding claims, wherein the restriction enzyme is selected from the group consisting of:
a) EcoRI according to SEQ ID No. 1, wherein the lysine on position 277 of SEQ ID No. 1 is replaced with cysteine and the cysteine on position 218 of SEQ ID No. 1 is replaced with alanine, valine or serine or a functional variant of EcoRI having at least 80% sequence identity to SEQ ID No. 1, wherein the lysine on a position corresponding to position 277 of SEQ ID No. 1 is replaced with cysteine and the cysteine on a position corresponding to position 218 of SEQ ID No. 1 is replaced with alanine, valine or serine;
b) EcoRI according to SEQ ID No.1, wherein a cysteine is added to the C-terminus via a linker comprising 12 glycine residues and the cysteine on position 218 of SEQ ID No. 1 is replaced with alanine, valine or serine or a functional variant of EcoRI having at least 80% sequence identity to SEQ ID No. 1 wherein a cysteine is added to the C-terminus via a linker comprising 12 glycine residues and the cysteine on a position corresponding to position 218 of SEQ ID No. 1 is replaced with alanine, valine or serine;
c) EcoRI according to SEQ ID No.1, wherein a cysteine is added to the C-terminus via a linker having the sequence GGGGSGGGGS and the cysteine on position 218 of SEQ ID No. 1 is replaced with alanine, valine or serine or a functional variant of EcoRI having at least 80% sequence identity to SEQ ID No. 1 wherein a cysteine is added to the C-terminus via a linker having the sequence GGGGSGGGGS and the cysteine on a position corresponding to position 218 of SEQ ID No. 1 is replaced with alanine, valine or serine
d) BciVI according to SEQ ID No. 3, wherein a cysteine residue is added to the C-terminus and the cysteine on position 271 of SEQ ID No. 3 is replaced with serine, valine or alanine or a functional variant of BciVI having at least 80% sequence identity to SEQ ID No. 3, wherein a cysteine residue is added to the C-terminus and the cysteine on a position corresponding to position 271 of SEQ ID No. 3 is replaced with serine, valine or alanine.

12. Method according to any of the preceding claims, wherein the restriction endonuclease further comprises a purification tag, preferably wherein the purification tag comprises six histidine residues.

13. Method according to any of the preceding claims, wherein the method is performed in an enzyme reactor.

14. Method according to any of the preceding claims, further comprising a step of purifying the linear template DNA after step a), preferably wherein the linear template DNA is purified by filtration.

15. Enzyme reactor comprising a restriction enzyme which is immobilized to a solid support and a module for transcribing the linearized template DNA into RNA *in vitro*, wherein the module for *in vitro* transcription comprises a RNA polymerase, a nucleotide mixture and an *in vitro* transcription buffer.

16. Enzyme reactor according to claim 15, further comprising a filter suitable for separating linearized template DNA from plasmid DNA.

17. Use of an enzyme reactor according to claim 15 or 16 for preparing a linear template DNA for *in vitro* transcription.

18. Use of an enzyme reactor according to claim 15 or 16 for *in vitro* transcription of linearized template DNA into RNA.

19. Kit comprising:
a restriction endonuclease which is immobilized onto a solid support, and a suitable restriction buffer, further comprising a RNA polymerase, a nucleotide mixture and an *in vitro* transcription buffer.

## Patentansprüche

1. Verfahren zur *in vitro* Transkription einer linearen Template-DNA, umfassend die Schritte von:
a) Kontaktieren einer Plasmid-DNA, die mindestens eine Erkennungssequenz für ein Restriktionsenzym besitzt, mit dem besagten Restriktionsenzym, wobei das Restriktionsenzym auf einem festen Träger immobilisiert ist, wodurch die Plasmid-DNA linearisiert wird und die lineare Template-DNA erhalten wird; und
b) Transkribieren der linearen Template-DNA in RNA *in vitro*, und
c) Erhalten der resultierenden RNA.

2. Verfahren nach Anspruch 1, wobei das Restriktionsenzym ein Typ-II-Restriktionsenzym ist, vorzugsweise wobei das Restriktionsenzym aus der Gruppe bestehend aus EcoRI, BciVI, XbaI, NdeI, AflII, SacI, KpnI, SmaI, BamHI, XbaI, SalI, SbfI, PstI und HindIII ausgewählt ist.

3. Verfahren nach Anspruch 1 oder 2, wobei das Restriktionsenzym durch kovalentes Binden immobilisiert ist, vorzugsweise wobei das kovalente Binden eine Disulfidbrücke oder eine Thioetherbindung ist.

4. Verfahren nach Anspruch 3, wobei das Restriktionsenzym durch kovalentes Binden an ein Thiol-aktiviertes Trägermaterial, einen Haloacetylfunktionalisierten festen Träger, einen Pyridyldisulfid-funktionalisierten festen Träger, einen Maleimid-aktivierten festen Träger, einen Epoxy-aktivierten festen Träger, oder eine Mischung daraus immobilisiert ist.

5. Verfahren nach Anspruch 4, wobei das Restriktionsenzym über eine Thiolgruppe mindestens eines Cysteinrests an das Thiol-aktivierte Trägermaterial gebunden ist, vorzugsweise wobei der mindestens eine Cytsteinrest, der an das Thiol-aktivierte Trägermaterial gebunden ist, nicht im Wildtyp-Restriktionsenzym vorkommt.

6. Verfahren nach Anspruch 5, wobei der mindestens eine Cysteinrest, der an das Thiol-aktivierte Trägermaterial gebunden ist, durch Substitution einer Aminosäure innerhalb des Wildtyp-Restriktionsenzyms durch Cystein oder durch Einfügen eines Cysteins in das Wildtyp-Restriktionsenzym eingeführt wurde, vorzugsweise durch Hinzufügen eines Cysteinrests an den N- oder C-Terminus des Restriktionsenzyms.

7. Verfahren nach einem der vorhergehenden Ansprüche, wobei das Restriktionsenzym ausgewählt ist aus der Gruppe bestehend aus:
a) EcoRI nach SEQ ID No. 1, wobei das Lysin an Position 277 von SEQ ID No. 1 durch Cystein ersetzt ist, oder eine funktionelle Variante von EcoRI mit mindestens 80% Sequenzidentität zu SEQ ID No. 1, wobei das Lysin an einer Position, die Position 277 von SEQ ID No. 1 entspricht, durch ein Cystein ersetzt ist;
b) EcoRI nach SEQ ID No.1 oder eine funktionelle Variante davon mit mindestens 80% Sequenzidentität zu SEQ ID No. 1, wobei ein Cystein an den C-Terminus hinzugefügt ist, vorzugsweise über einen Linker;
c) HindIII nach SEQ ID No. 2, wobei das Leucin an Position 300 von SEQ ID No. 2 durch Cystein substituiert ist, oder eine funktionelle Variante von HindIII mit mindestens 80% Sequenzidentität zu SEQ ID No. 2, wobei das Leucin an einer Position, die Position 300 von SEQ ID No. 2 entspricht, durch ein Cystein substituiert ist;
d) HindIII nach SEQ ID No. 2, wobei ein Cysteinrest an den C-Terminus hinzugefügt ist, vorzugsweise über einen Linker, oder eine funktionelle Variante von HindIII mit mindestens 80% Sequenzidentität zu SEQ ID No. 2, wobei ein Cysteinrest an den C-Terminus hinzugefügt ist, vorzugsweise über einen Linker; und
e) BciVI nach SEQ ID No. 3, wobei ein Cysteinrest an den C-Terminus hinzugefügt ist, vorzugsweise über einen Linker oder eine funktionelle Variante davon mit mindestens 80% Sequenzidentität zu SEQ ID No. 3, wobei ein Cysteinrest an den C-Terminus hinzugefügt ist, vorzugsweise über einen Linker.

8. Verfahren nach Anspruch 7, wobei der Linker die Sequenz GGGGSGGGGS (SEQ ID No. 49) hat.

9. Verfahren nach einem der Ansprüche 6 bis 8, wobei, wenn vorhanden, eine oder mehrere Cysteinreste in dem Wildtyp-Restriktionsenzym durch eine andere Aminosäure ersetzt sind.

10. Verfahren nach Anspruch 9, wobei das Restriktionsenzym ausgewählt ist aus der Gruppe bestehend aus:
a) EcoRI nach SEQ ID No. 1, wobei das Cystein an Position 218 von SEQ ID No. 1 durch eine andere Aminosäure ersetzt ist, oder eine funktionelle Variante von EcoRI mit mindestens 80% Sequenzidentität zu SEQ ID No. 1, wobei das Cystein an einer Position, die Position 218 von SEQ ID No. 1 entspricht, durch eine andere Aminosäure ersetzt ist; oder
b) BciVI nach SEQ ID No. 3, wobei das Cystein an Position 271 von SEQ ID No. 3 durch eine andere Aminosäure ersetzt ist, oder eine funktionelle Variante von BciVI mit mindestens 80% Sequenzidentität zu SEQ ID No. 3, wobei das Cystein an einer Position, die Position 271 von SEQ ID No. 3 entspricht, durch eine andere Aminosäure ersetzt ist.

11. Verfahren nach einem der vorhergehenden Ansprüche, wobei das Restriktionsenzym ausgewählt ist aus der Gruppe bestehend aus:
a) EcoRI nach SEQ ID No. 1, wobei das Lysin an Position 277 von SEQ ID No. 1 durch Cystein ersetzt ist und das Cystein an Position 218 von SEQ ID No. 1 durch eine Alanin, Valin, oder Serin ersetzt ist, oder eine funktionelle Variante von EcoRI mit mindestens 80% Sequenzidentität zu SEQ ID No. 1, wobei das Lysin an einer Position, die Position 277 von SEQ ID No. 1 entspricht, durch Cystein ersetzt ist und das Cystein an einer Position, die Position 218 von SEQ ID No. 1 entspricht, durch Alanin, Valin oder Serin ersetzt ist;
b) EcoRI nach SEQ ID No.1, wobei ein Cystein an den C-Terminus über einen Linker, der 12 Glycinreste umfasst, hinzugefügt ist, und das Cystein an Position 218 von SEQ ID No. 1 durch Alanin, Valin, oder Serin ersetzt ist, oder eine funktionelle Variante von EcoRI mit mindestens 80% Sequenzidentität zu SEQ ID No. 1, wobei ein Cystein an den C-Terminus über einen Linker, der 12 Glycinreste umfasst, hinzugefügt ist, und das Cystein an einer Position, die Position 218 von SEQ ID No. 1 entspricht, durch Alanin, Valin, oder Serin ersetzt ist;
c) EcoRI nach SEQ ID No.1, wobei ein Cystein an den C-Terminus über einen Linker mit der Sequenz GGGGSGGGGS hinzugefügt ist, und das Cystein an Position 218 von SEQ ID No. 1 durch ein Alanin, Valin, oder Serin ersetzt ist, oder eine funktionelle Variante von EcoRI mit mindestens 80% Sequenzidentität zu SEQ ID No. 1, wobei ein Cystein an den C-Terminus über einen Linker mit der Sequenz GGGGSGGGGS hinzugefügt ist, und das Cystein an einer Position, die Position 218 von SEQ ID No. 1 entspricht, durch Alanin, Valin, oder Serin ersetzt ist
d) BciVI nach SEQ ID No. 3, wobei ein Cysteinrest an den C-Terminus hinzugefügt ist und das Cystein an Position 271 von SEQ ID No. 3 durch Serin, Valin oder Alanin ersetzt ist, oder eine funktionelle Variante von BciVI mit mindestens 80% Sequenzidentität zu SEQ ID No. 3, wobei ein Cysteinrest an den C-Terminus hinzugefügt ist und das Cystein an einer Position, die Position 271 von SEQ ID No. 3 entspricht, durch Serin, Valin oder Alanin ersetzt ist.

12. Verfahren nach einem der vorhergehenden Ansprüche, wobei die Restriktionsendonuklease weiterhin ein Aufreinigungstag umfasst, vorzugsweise wobei das Aufreinigungstag sechs Histidinreste umfasst.

13. Verfahren nach einem der vorhergehenden Ansprüche, wobei das Verfahren in einem Ezymreaktor durchgeführt wird.

14. Verfahren nach einem der vorhergehenden Ansprüche, weiterhin umfassend einen Schritt des Aufreinigens der linearen Template-DNA nach Schritt a), vorzugsweise wobei die lineare Template-DNA durch Filtration aufgereinigt wird.

15. Enzymreaktor umfassend ein Restriktionsenzym, das auf einem festen Träger immobilisiert ist, und ein Modul für das Transkribieren der linearisierten Template-DNA in RNA *in vitro*, wobei das Modul für *in vitro* Transkription eine RNA-Polymerase, eine Nukleotidmischung und einen *in vitro* Transkriptionspuffer umfasst.

16. Enzymreaktor nach Anspruch 15, weiterhin umfassend einen Filter, der für das Abtrennen linearisierter Template-DNA von Plasmid-DNA geeignet ist.

17. Verwendung eines Enzymreaktors nach Anspruch 15 oder 16 zur Herstellung einer linearisierten Template-DNA für *in vitro* Transkription.

18. Verwendung eines Enzymreaktors nach Anspruch 15 oder 16 zur *in vitro* Transkription von linearisierter Template-DNA in RNA.

19. Kit umfassend:
eine Restriktionsendonuklease, die auf einem festen Träger immobilisiert ist, und
einen geeigneten Restriktionspuffer, weiterhin umfassend eine RNA Polymerase,
eine Nukleotidmischung, und einen *in vitro* Transkriptionspuffer.

## Revendications

1. Procédé de transcription *in vitro* d'un ADN matrice linéaire, comprenant les étapes consistant à :
a) mettre en contact un ADN plasmidique ayant au moins une séquence de reconnaissance pour une enzyme de restriction avec ladite enzyme de restriction, dans lequel l'enzyme de restriction est immobilisée sur un support solide, linéarisant ainsi l'ADN plasmidique et fournissant l'ADN matrice linéaire ; et
b) transcrire l'ADN matrice linéaire en ARN *in vitro,* et
c) obtenir l'ARN résultant.

2. Procédé selon la revendication 1, dans lequel l'enzyme de restriction est une enzyme de restriction de type II, de préférence dans lequel l'enzyme de restriction est choisi dans le groupe constitué par EcoRI, BciVI, XbaI, NdeI, AflII, SacI, KpnI, SmaI, BamHI, XbaI, SalI, SbfI, PstI et HindIII.

3. Procédé selon la revendication 1 ou 2, dans lequel l'enzyme de restriction est immobilisée par liaison covalente, de préférence dans lequel la liaison covalente est un pont disulfure ou une liaison thioéther.

4. Procédé selon la revendication 3, dans lequel l'enzyme de restriction est immobilisée par liaison covalente à un matériau de support activé par un thiol, un support solide à fonctionnalité halogénoacétyle, un support solide à fonctionnalité disulfure de pyridyle, un support solide activé par maléimide, un support solide activé par époxy ou un de leurs mélanges.

5. Procédé selon la revendication 4, dans lequel l'enzyme de restriction est liée au matériau de support activé par un thiol via un groupe thiol d'au moins un résidu cystéine, de préférence dans lequel l'au moins un résidu cystéine qui est lié au matériau de support activé par un thiol n'est pas présent dans l'enzyme de restriction sauvage.

6. Procédé selon la revendication 5, dans lequel l'au moins un résidu cystéine qui est lié au matériau de support activé par un thiol a été introduit par substitution d'un acide aminé dans l'enzyme de restriction sauvage ou en insérant une cystéine dans l'enzyme de restriction sauvage, de préférence en ajoutant un résidu cystéine à l'extrémité N ou C terminale de l'enzyme d restriction.

7. Procédé selon l'une quelconque des revendications précédentes, dans lequel l'enzyme de restriction est choisie dans le groupe constitué par :
a) EcoRI selon SEQ ID n° 1, dans lequel la lysine en position 277 de SEQ ID n° 1 est remplacée avec une cystéine ou un variant fonctionnel d'EcoRI ayant au moins 80 % d'identité de séquence à SEQ ID n° 1, dans lequel la lysine sur une position correspondant à la position 277 de SEQ ID n° 1 est remplacée avec une cystéine ;
b) EcoRI selon SEQ ID n° 1 ou un de ses variants fonctionnels ayant au moins 80 % d'identité de séquence à SEQ ID n° 1, dans lequel une cystéine est ajoutée à l'extrémité C terminale, de préférence via un lieur ;
c) HindIII selon SEQ ID n° 2, dans lequel la leucine en position 300 de SEQ ID n° 2 est substituée avec une cystéine ou un variant fonctionnel de HindIII ayant au moins 80 % d'identité de séquence à SEQ ID n° 2, dans lequel la leucine sur une position correspondant à la position 300 de SEQ ID n° 2 est substituée avec une cystéine ;
d) HindIII selon SEQ ID n° 2, dans lequel un résidu cystéine est ajouté à l'extrémité C-terminale, de préférence via un lieur, ou un variant fonctionnel de HindIII ayant au moins 80 % d'identité de séquence à SEQ ID n° 2, dans lequel une cystéine est ajoutée à l'extrémité C-terminale, de préférence via un lieur ; et
e) BciVI selon SEQ ID n° 3, dans lequel un résidu cystéine est ajouté à l'extrémité C-terminale, de préférence via un lieur, ou un de ses variants fonctionnels ayant au moins 80 % d'identité de séquence à SEQ ID n° 3, dans lequel une cystéine est ajoutée à l'extrémité C-terminale, de préférence via un lieur.

8. Procédé selon la revendication 7, dans lequel le lieur a la séquence GGGGSGGGGS (SEQ ID n° 49).

9. Procédé selon l'une quelconque des revendications 6 à 8, dans lequel, s'ils sont présents, un ou plusieurs résidus cystéine dans l'enzyme de restriction sauvage sont remplacés avec un autre acide aminé.

10. Procédé selon la revendication 9, dans lequel l'enzyme de restriction est choisie dans le groupe constitué par :
a) EcoRI selon SEQ ID n° 1, dans lequel la position de cystéine 218 de SEQ ID n° 1 est remplacée avec un autre acide aminé ou un variant fonctionnel de EcoRI ayant au moins 80 % d'identité de séquence à SEQ ID n° 1, dans lequel la cystéine sur une position correspondant à la position 218 de SEQ ID n° 1 est remplacée avec un autre acide aminé ; ou
b) BciVI selon SEQ ID n° 3, dans lequel la cystéine en position 271 de SEQ ID n° 3 est remplacée avec un autre acide aminé ou un variant fonctionnel de BciVI ayant au moins 80 % d'identité de séquence à SEQ ID n° 3, dans lequel la cystéine sur une position correspondant à la position 271 de SEQ ID n° 3 est remplacée avec un autre acide aminé.

11. Procédé selon l'une quelconque des revendications précédentes, dans lequel l'enzyme de restriction est choisie dans le groupe constitué par :
a) EcoRI selon SEQ ID n° 1, dans lequel la lysine en position 277 de SEQ ID n° 1 est remplacée avec une cystéine et la cystéine en position 218 de SEQ ID n° 1 est remplacée avec une alanine, une valine ou une sérine ou un variant fonctionnel de EcoRI ayant au moins 80 % d'identité de séquence à SEQ ID n° 1, dans lequel la lysine sur une position correspondant à la position 277 de SEQ ID n° 1 est remplacée avec une cystéine et la cystéine sur une position correspondant à la position 218 de SEQ ID n° 1 est remplacée avec une alanine, une valine ou une sérine ;
b) EcoRI selon SEQ ID n° 1, dans lequel une cystéine est ajoutée à l'extrémité C-terminale via un lieur comprenant 12 résidus glycine et la cystéine en position 218 de SEQ ID n° 1 est remplacée avec une alanine, une valine ou une sérine ou un variant fonctionnel de EcoRI ayant au moins 80 % d'identité de séquence à SEQ ID n° 1, dans lequel une cystéine est ajoutée à l'extrémité C-terminale via un lieur comprenant 12 résidus de glycine et la cystéine sur une position correspondant à la position 218 de SEQ ID n° 1 est remplacée avec une alanine, une valine ou une sérine ;
c) EcoRI selon SEQ ID n° 1, dans lequel une cystéine est ajoutée à l'extrémité C-terminale via un lieur ayant la séquence GGGGSGGGGS et la cystéine en position 218 de SEQ ID n° 1 est remplacée avec une alanine, une valine ou une sérine ou un variant fonctionnel de EcoRI ayant au moins 80 % d'identité de séquence à SEQ ID n° 1 dans lequel une cystéine est ajoutée à l'extrémité C-terminale via un lieur ayant la séquence GGGGSGGGGS et la cystéine sur une position correspondant à la position 218 de SEQ ID n° 1 est remplacée avec une alanine, une valine ou une sérine
d) BciVI selon SEQ ID n° 3, dans lequel un résidu cystéine est ajouté à l'extrémité C-terminale et la cystéine en position 271 de SEQ ID n° 3 est remplacée avec une alanine, une valine ou une sérine ou un variant fonctionnel de BciVI ayant au moins 80 % d'identité de séquence à SEQ ID n° 3, dans lequel un résidu cystéine est ajouté à l'extrémité C-terminale et la cystéine sur une position correspondant à la position 271 de SEQ ID n° 3 est remplacée avec une sérine, une valine ou une alanine.

12. Procédé selon l'une quelconque des revendications précédentes, dans lequel l'endonucléase de restriction comprend en outre une étiquette de purification, de préférence dans lequel l'étiquette de purification comprend six résidus histidine.

13. Procédé selon l'une quelconque des revendications précédentes, dans lequel le procédé est effectué dans un réacteur d'enzyme.

14. Procédé selon l'une quelconque des revendications précédentes, comprenant en outre une étape de purification de l'ADN matrice linéaire après l'étape a), de préférence dans lequel l'ADN matrice linéaire est purifié par filtration.

15. Réacteur d'enzyme comprenant une enzyme de restriction qui est immobilisée sur un support solide et un module pour transcrire l'ADN matrice linéarisé en ARN *in vitro,* dans lequel le module pour la transcription *in vitro* comprend une ARN polymérase, un mélange de nucléotides et un tampon de transcription *in vitro.*

16. Réacteur d'enzyme selon la revendication 15, comprenant en outre un filtre approprié pour séparer l'ADN matrice linéarisé de l'ADN plasmidique.

17. Utilisation d'un réacteur d'enzyme selon la revendication 15 ou 16 pour préparer un ADN matrice linéarisé pour la transcription *in vitro.*

18. Utilisation d'un réacteur d'enzyme selon la revendication 15 ou 16 pour la transcription *in vitro* de l'ADN matrice linéarisé en ARN.

19. Kit comprenant :
une endonucléase de restriction qui est immobilisée sur un support solide, et un tampon de restriction approprié, comprenant en outre une ARN polymérase, un mélange de nucléotides et un tampon de transcription *in vitro.*


### SEQUENCE LISTING

<110> CureVac AG
<120> Method for in vitro transcription using an immobilized restriction enzyme
<130> C 9809-WO2/RN
<150> PCT/EP2015/059559
   <151> 30.04.2015
<160> 80
<170> PatentIn version 3.5
<210> 1
   <211> 277
   <212> PRT
   <213> Escherichia coli
<400> 1
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
   165 170 175
   Glu Asn Ile Ser Ile Thr Arg Pro Asp Gly Arg Val Val Asn Leu Glu
   180 185 190
   Tyr Asn Ser Gly Ile Leu Asn Arg Leu Asp Arg Leu Thr Ala Ala Asn
   195 200 205
   Tyr Gly Met Pro Ile Asn Ser Asn Leu Cys Ile Asn Lys Phe Val Asn
   210 215 220
   His Lys Asp Lys Ser Ile Met Leu Gln Ala Ala Ser Ile Tyr Thr Gln
   225 230 235 240
   Gly Asp Gly Arg Glu Trp Asp Ser Lys Ile Met Phe Glu Ile Met Phe
   245 250 255
   Asp Ile Ser Thr Thr Ser Leu Arg Val Leu Gly Arg Asp Leu Phe Glu
   260 265 270
   Gln Leu Thr Ser Lys
   275
<210> 2
   <211> 300
   <212> PRT
   <213> Haemophilus influenzae
<400> 2
   Met Lys Lys Ser Ala Leu Glu Lys Leu Leu Ser Leu Ile Glu Asn Leu
   1 5 10 15
   Thr Asn Gln Glu Phe Lys Gln Ala Thr Asn Ser Leu Ile Ser Phe Ile
   20 25 30
   Tyr Lys Leu Asn Arg Asn Glu Val Ile Glu Leu Val Arg Ser Ile Gly
   35 40 45
   Ile Leu Pro Glu Ala Ile Lys Pro Ser Ser Thr Gln Glu Lys Leu Phe
   50 55 60
   Ser Lys Ala Gly Asp Ile Val Leu Ala Lys Ala Phe Gln Leu Leu Asn
   65 70 75 80
   Leu Asn Ser Lys Pro Leu Glu Gln Arg Gly Asn Ala Gly Asp Val Ile
   85 90 95
   Ala Leu Ser Lys Glu Phe Asn Tyr Gly Leu Val Ala Asp Ala Lys Ser
               100                 105                 110
   Phe Arg Leu Ser Arg Thr Ala Lys Asn Gln Lys Asp Phe Lys Val Lys
           115                 120                 125
   Ala Leu Ser Glu Trp Arg Glu Asp Lys Asp Tyr Ala Val Leu Thr Ala
       130                 135                 140
   Pro Phe Phe Gln Tyr Pro Thr Thr Lys Ser Gln Ile Phe Lys Gln Ser
   145                 150                 155                 160
   Leu Asp Glu Asn Val Leu Leu Phe Ser Trp Glu His Leu Ala Ile Leu
                   165                 170                 175
   Leu Gln Leu Asp Leu Glu Glu Thr Asn Ile Phe Ser Phe Glu Gln Leu
               180                 185                 190
   Trp Asn Phe Pro Lys Lys Gln Ser Lys Lys Thr Ser Val Ser Asp Ala
           195                 200                 205
   Glu Asn Asn Phe Met Arg Asp Phe Asn Lys Tyr Phe Met Asp Leu Phe
       210                 215                 220
   Lys Ile Asp Lys Asp Thr Leu Asn Gln Leu Leu Gln Lys Glu Ile Asn
   225                 230                 235                 240
   Phe Ile Glu Glu Arg Ser Leu Ile Glu Lys Glu Tyr Trp Lys Lys Gln
                   245                 250                 255
   Ile Asn Ile Ile Lys Asn Phe Thr Arg Glu Glu Ala Ile Glu Ala Leu
               260                 265                 270
   Leu Lys Asp Ile Asn Met Ser Ser Lys Ile Glu Thr Ile Asp Ser Phe
           275                 280                 285
   Ile Lys Gly Ile Lys Ser Asn Asp Arg Leu Tyr Leu
       290                 295                 300
<210> 3
   <211> 358
   <212> PRT
   <213> Bacillus circulans
<400> 3
   Met Asn Phe Phe Ser Leu His Pro Asn Val Tyr Ala Thr Gly Arg Pro
   1               5                   10                  15
   Lys Gly Leu Ile Gly Met Leu Glu Asn Val Trp Val Ser Asn His Thr
               20                  25                  30
   Pro Gly Glu Gly Thr Leu Tyr Leu Ile Ser Gly Phe Ser Asn Tyr Asn
           35                  40                  45
   Gly Gly Val Arg Phe Tyr Glu Thr Phe Thr Glu His Ile Asn Gln Gly
       50                  55                  60
   Gly Arg Val Ile Ala Ile Leu Gly Gly Ser Thr Ser Gln Arg Leu Ser
   65                  70                  75                  80
   Ser Arg Gln Val Val Glu Glu Leu Leu Asn Arg Gly Val Glu Val His
                   85                  90                  95
   Ile Ile Asn Arg Lys Arg Ile Leu His Ala Lys Leu Tyr Gly Thr Ser
               100                 105                 110
   Asn Asn Leu Gly Glu Ser Leu Val Val Ser Ser Gly Asn Phe Thr Gly
           115                 120                 125
   Pro Gly Met Ser Gln Asn Ile Glu Ala Ser Leu Leu Leu Asp Asn Asn
       130                 135                 140
   Thr Thr Gln Ser Met Gly Phe Ser Trp Asn Asp Met Ile Ser Glu Met
   145                 150                 155                 160
   Leu Asn Gln Asn Trp His Ile His Asn Met Thr Asn Ala Thr Asp Ala
                   165                 170                 175
   Ser Pro Gly Trp Asn Leu Leu Tyr Asp Glu Arg Thr Thr Asn Leu Thr
               180                 185                 190
   Leu Asp Glu Thr Glu Arg Val Thr Leu Ile Val Thr Leu Gly His Ala
           195                 200                 205
   Asp Thr Ala Arg Ile Gln Ala Ala Pro Gly Thr Thr Ala Gly Gln Gly
       210                 215                 220
   Thr Gln Tyr Phe Trp Leu Ser Lys Asp Ser Tyr Asp Phe Phe Pro Pro
   225                 230                 235                 240
   Leu Thr Ile Arg Asn Arg Arg Gly Thr Lys Ala Thr Tyr Ser Ser Leu
                   245                 250                 255
   Ile Asn Met Asn Tyr Ile Asp Ile Asn Tyr Thr Asp Thr Gln Cys Arg
               260                 265                 270
   Val Thr Phe Glu Ala Glu Asn Asn Phe Asp Phe Arg Leu Gly Thr Gly
   275 280 285
   Lys Leu Arg Tyr Thr Gly Val Ala Lys Ser Asn Asp Ile Ala Ala Ile
   290 295 300
   Thr Arg Val Gly Asp Ser Asp Tyr Glu Leu Arg Ile Ile Lys Gln Gly
   305 310 315 320
   Thr Pro Glu His Ser Gln Leu Asp Pro Tyr Ala Val Ser Phe Ile Gly
   325 330 335
   Asn Arg Gly Lys Arg Phe Gly Tyr Ile Ser Asn Glu Glu Phe Gly Arg
   340 345 350
   Ile Ile Gly Val Thr Phe
   355
<210> 4
   <211> 277
   <212> PRT
   <213> Artificial sequence
<220>
   <223> EcoRI K277C
<400> 4
   Met Ser Asn Lys Lys Gln Ser Asn Arg Leu Thr Glu Gln His Lys Leu
   1 5 10 15
   Ser Gln Gly Val Ile Gly Ile Phe Gly Asp Tyr Ala Lys Ala His Asp
   20 25 30
   Leu Ala Val Gly Glu Val Ser Lys Leu Val Lys Lys Ala Leu Ser Asn
   35 40 45
   Glu Tyr Pro Gln Leu Ser Phe Arg Tyr Arg Asp Ser Ile Lys Lys Thr
   50 55 60
   Glu Ile Asn Glu Ala Leu Lys Lys Ile Asp Pro Asp Leu Gly Gly Thr
   65 70 75 80
   Leu Phe Val Ser Asn Ser Ser Ile Lys Pro Asp Gly Gly Ile Val Glu
   85 90 95
   Val Lys Asp Asp Tyr Gly Glu Trp Arg Val Val Leu Val Ala Glu Ala
   100 105 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Cys Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Cys
           275
<210> 5
   <211> 277
   <212> PRT
   <213> Artificial sequence
<220>
   <223> EcoRI K277C, C218A
<400> 5
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ala Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Cys
           275
<210> 6
   <211> 277
   <212> PRT
   <213> Artificial sequence
<220>
   <223> EcoRI K277C, C218S
<400> 6
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ser Ile Asn Lys Phe Val Asn
           210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Cys
           275
<210> 7
   <211> 290
   <212> PRT
   <213> Artificial sequence
<220>
   <223> EcoRI 278(12xG)C
<400> 7
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Cys Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Gly Gly Gly Gly Gly Gly Gly
           275                 280                 285
   Gly Cys
       290
<210> 8
   <211> 290
   <212> PRT
   <213> Artificial sequence
<220>
   <223> EcoRI 278(12xG)C, C218A
<400> 8
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ala Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Gly Gly Gly Gly Gly Gly Gly
           275                 280                 285
   Gly Cys
       290
<210> 9
   <211> 290
   <212> PRT
   <213> Artificial sequence
<220>
   <223> EcoRI 278(12xG)C, C218S
<400> 9
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ser Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Gly Gly Gly Gly Gly Gly Gly
           275                 280                 285
   Gly Cys
       290
<210> 10
   <211> 300
   <212> PRT
   <213> Artificial sequence
<220>
   <223> HindIII L300C
<400> 10
   Met Lys Lys Ser Ala Leu Glu Lys Leu Leu Ser Leu Ile Glu Asn Leu
   1               5                   10                  15
   Thr Asn Gln Glu Phe Lys Gln Ala Thr Asn Ser Leu Ile Ser Phe Ile
               20                  25                  30
   Tyr Lys Leu Asn Arg Asn Glu Val Ile Glu Leu Val Arg Ser Ile Gly
           35                  40                  45
   Ile Leu Pro Glu Ala Ile Lys Pro Ser Ser Thr Gln Glu Lys Leu Phe
       50                  55                  60
   Ser Lys Ala Gly Asp Ile Val Leu Ala Lys Ala Phe Gln Leu Leu Asn
   65                  70                  75                  80
   Leu Asn Ser Lys Pro Leu Glu Gln Arg Gly Asn Ala Gly Asp Val Ile
                   85                  90                  95
   Ala Leu Ser Lys Glu Phe Asn Tyr Gly Leu Val Ala Asp Ala Lys Ser
               100                 105                 110
   Phe Arg Leu Ser Arg Thr Ala Lys Asn Gln Lys Asp Phe Lys Val Lys
           115                 120                 125
   Ala Leu Ser Glu Trp Arg Glu Asp Lys Asp Tyr Ala Val Leu Thr Ala
       130                 135                 140
   Pro Phe Phe Gln Tyr Pro Thr Thr Lys Ser Gln Ile Phe Lys Gln Ser
   145                 150                 155                 160
   Leu Asp Glu Asn Val Leu Leu Phe Ser Trp Glu His Leu Ala Ile Leu
                   165                 170                 175
   Leu Gln Leu Asp Leu Glu Glu Thr Asn Ile Phe Ser Phe Glu Gln Leu
               180                 185                 190
   Trp Asn Phe Pro Lys Lys Gln Ser Lys Lys Thr Ser Val Ser Asp Ala
           195                 200                 205
   Glu Asn Asn Phe Met Arg Asp Phe Asn Lys Tyr Phe Met Asp Leu Phe
       210                 215                 220
   Lys Ile Asp Lys Asp Thr Leu Asn Gln Leu Leu Gln Lys Glu Ile Asn
   225                 230                 235                 240
   Phe Ile Glu Glu Arg Ser Leu Ile Glu Lys Glu Tyr Trp Lys Lys Gln
                   245                 250                 255
   Ile Asn Ile Ile Lys Asn Phe Thr Arg Glu Glu Ala Ile Glu Ala Leu
               260                 265                 270
   Leu Lys Asp Ile Asn Met Ser Ser Lys Ile Glu Thr Ile Asp Ser Phe
           275                 280                 285
   Ile Lys Gly Ile Lys Ser Asn Asp Arg Leu Tyr Cys
       290                 295                 300
<210> 11
   <211> 301
   <212> PRT
   <213> Artificial sequence
<220>
   <223> HindIII 301C
<400> 11
   Met Lys Lys Ser Ala Leu Glu Lys Leu Leu Ser Leu Ile Glu Asn Leu
   1               5                   10                  15
   Thr Asn Gln Glu Phe Lys Gln Ala Thr Asn Ser Leu Ile Ser Phe Ile
                   20                  25                  30
   Tyr Lys Leu Asn Arg Asn Glu Val Ile Glu Leu Val Arg Ser Ile Gly
           35                  40                  45
   Ile Leu Pro Glu Ala Ile Lys Pro Ser Ser Thr Gln Glu Lys Leu Phe
       50                  55                  60
   Ser Lys Ala Gly Asp Ile Val Leu Ala Lys Ala Phe Gln Leu Leu Asn
   65                  70                  75                  80
   Leu Asn Ser Lys Pro Leu Glu Gln Arg Gly Asn Ala Gly Asp Val Ile
                   85                  90                  95
   Ala Leu Ser Lys Glu Phe Asn Tyr Gly Leu Val Ala Asp Ala Lys Ser
               100                 105                 110
   Phe Arg Leu Ser Arg Thr Ala Lys Asn Gln Lys Asp Phe Lys Val Lys
           115                 120                 125
   Ala Leu Ser Glu Trp Arg Glu Asp Lys Asp Tyr Ala Val Leu Thr Ala
       130                 135                 140
   Pro Phe Phe Gln Tyr Pro Thr Thr Lys Ser Gln Ile Phe Lys Gln Ser
   145                 150                 155                 160
   Leu Asp Glu Asn Val Leu Leu Phe Ser Trp Glu His Leu Ala Ile Leu
                   165                 170                 175
   Leu Gln Leu Asp Leu Glu Glu Thr Asn Ile Phe Ser Phe Glu Gln Leu
               180                 185                 190
   Trp Asn Phe Pro Lys Lys Gln Ser Lys Lys Thr Ser Val Ser Asp Ala
           195                 200                 205
   Glu Asn Asn Phe Met Arg Asp Phe Asn Lys Tyr Phe Met Asp Leu Phe
       210                 215                 220
   Lys Ile Asp Lys Asp Thr Leu Asn Gln Leu Leu Gln Lys Glu Ile Asn
   225                 230                 235                 240
   Phe Ile Glu Glu Arg Ser Leu Ile Glu Lys Glu Tyr Trp Lys Lys Gln
                   245                 250                 255
   Ile Asn Ile Ile Lys Asn Phe Thr Arg Glu Glu Ala Ile Glu Ala Leu
               260                 265                 270
   Leu Lys Asp Ile Asn Met Ser Ser Lys Ile Glu Thr Ile Asp Ser Phe
           275                 280                 285
   Ile Lys Gly Ile Lys Ser Asn Asp Arg Leu Tyr Leu Cys
       290                 295                 300
<210> 12
   <211> 359
   <212> PRT
   <213> Artificial sequence
<220>
   <223> BciVI 359C
<400> 12
   Met Asn Phe Phe Ser Leu His Pro Asn Val Tyr Ala Thr Gly Arg Pro
   1               5                   10                  15
   Lys Gly Leu Ile Gly Met Leu Glu Asn Val Trp Val Ser Asn His Thr
               20                  25                  30
   Pro Gly Glu Gly Thr Leu Tyr Leu Ile Ser Gly Phe Ser Asn Tyr Asn
           35                  40                  45
   Gly Gly Val Arg Phe Tyr Glu Thr Phe Thr Glu His Ile Asn Gln Gly
       50                  55                  60
   Gly Arg Val Ile Ala Ile Leu Gly Gly Ser Thr Ser Gln Arg Leu Ser
   65                  70                  75                  80
   Ser Arg Gln Val Val Glu Glu Leu Leu Asn Arg Gly Val Glu Val His
                   85                  90                  95
   Ile Ile Asn Arg Lys Arg Ile Leu His Ala Lys Leu Tyr Gly Thr Ser
               100                 105                 110
   Asn Asn Leu Gly Glu Ser Leu Val Val Ser Ser Gly Asn Phe Thr Gly
           115                 120                 125
   Pro Gly Met Ser Gln Asn Ile Glu Ala Ser Leu Leu Leu Asp Asn Asn
       130                 135                 140
   Thr Thr Gln Ser Met Gly Phe Ser Trp Asn Asp Met Ile Ser Glu Met
   145                 150                 155                 160
   Leu Asn Gln Asn Trp His Ile His Asn Met Thr Asn Ala Thr Asp Ala
                   165                 170                 175
   Ser Pro Gly Trp Asn Leu Leu Tyr Asp Glu Arg Thr Thr Asn Leu Thr
                   180                 185                 190
   Leu Asp Glu Thr Glu Arg Val Thr Leu Ile Val Thr Leu Gly His Ala
           195                 200                 205
   Asp Thr Ala Arg Ile Gln Ala Ala Pro Gly Thr Thr Ala Gly Gln Gly
       210                 215                 220
   Thr Gln Tyr Phe Trp Leu Ser Lys Asp Ser Tyr Asp Phe Phe Pro Pro
   225                 230                 235                 240
   Leu Thr Ile Arg Asn Arg Arg Gly Thr Lys Ala Thr Tyr Ser Ser Leu
                   245                 250                 255
   Ile Asn Met Asn Tyr Ile Asp Ile Asn Tyr Thr Asp Thr Gln Cys Arg
               260                 265                 270
   Val Thr Phe Glu Ala Glu Asn Asn Phe Asp Phe Arg Leu Gly Thr Gly
           275                 280                 285
   Lys Leu Arg Tyr Thr Gly Val Ala Lys Ser Asn Asp Ile Ala Ala Ile
       290                 295                 300
   Thr Arg Val Gly Asp Ser Asp Tyr Glu Leu Arg Ile Ile Lys Gln Gly
   305                 310                 315                 320
   Thr Pro Glu His Ser Gln Leu Asp Pro Tyr Ala Val Ser Phe Ile Gly
                   325                 330                 335
   Asn Arg Gly Lys Arg Phe Gly Tyr Ile Ser Asn Glu Glu Phe Gly Arg
               340                 345                 350
   Ile Ile Gly Val Thr Phe Cys
           355
<210> 13
   <211> 359
   <212> PRT
   <213> Artificial sequence
<220>
   <223> BciVI 359C,C271A
<400> 13
   Met Asn Phe Phe Ser Leu His Pro Asn Val Tyr Ala Thr Gly Arg Pro
   1               5                   10                  15
   Lys Gly Leu Ile Gly Met Leu Glu Asn Val Trp Val Ser Asn His Thr
               20                  25                  30
   Pro Gly Glu Gly Thr Leu Tyr Leu Ile Ser Gly Phe Ser Asn Tyr Asn
           35                  40                  45
   Gly Gly Val Arg Phe Tyr Glu Thr Phe Thr Glu His Ile Asn Gln Gly
       50                  55                  60
   Gly Arg Val Ile Ala Ile Leu Gly Gly Ser Thr Ser Gln Arg Leu Ser
   65                  70                  75                  80
   Ser Arg Gln Val Val Glu Glu Leu Leu Asn Arg Gly Val Glu Val His
                   85                  90                  95
   Ile Ile Asn Arg Lys Arg Ile Leu His Ala Lys Leu Tyr Gly Thr Ser
               100                 105                 110
   Asn Asn Leu Gly Glu Ser Leu Val Val Ser Ser Gly Asn Phe Thr Gly
           115                 120                 125
   Pro Gly Met Ser Gln Asn Ile Glu Ala Ser Leu Leu Leu Asp Asn Asn
       130                 135                 140
   Thr Thr Gln Ser Met Gly Phe Ser Trp Asn Asp Met Ile Ser Glu Met
   145                 150                 155                 160
   Leu Asn Gln Asn Trp His Ile His Asn Met Thr Asn Ala Thr Asp Ala
                   165                 170                 175
   Ser Pro Gly Trp Asn Leu Leu Tyr Asp Glu Arg Thr Thr Asn Leu Thr
               180                 185                 190
   Leu Asp Glu Thr Glu Arg Val Thr Leu Ile Val Thr Leu Gly His Ala
           195                 200                 205
   Asp Thr Ala Arg Ile Gln Ala Ala Pro Gly Thr Thr Ala Gly Gln Gly
       210                 215                 220
   Thr Gln Tyr Phe Trp Leu Ser Lys Asp Ser Tyr Asp Phe Phe Pro Pro
   225                 230                 235                 240
   Leu Thr Ile Arg Asn Arg Arg Gly Thr Lys Ala Thr Tyr Ser Ser Leu
                   245                 250                 255
   Ile Asn Met Asn Tyr Ile Asp Ile Asn Tyr Thr Asp Thr Gln Ala Arg
               260                 265                 270
   Val Thr Phe Glu Ala Glu Asn Asn Phe Asp Phe Arg Leu Gly Thr Gly
               275                 280                 285
   Lys Leu Arg Tyr Thr Gly Val Ala Lys Ser Asn Asp Ile Ala Ala Ile
       290                 295                 300
   Thr Arg Val Gly Asp Ser Asp Tyr Glu Leu Arg Ile Ile Lys Gln Gly
   305                 310                 315                 320
   Thr Pro Glu His Ser Gln Leu Asp Pro Tyr Ala Val Ser Phe Ile Gly
                   325                 330                 335
   Asn Arg Gly Lys Arg Phe Gly Tyr Ile Ser Asn Glu Glu Phe Gly Arg
               340                 345                 350
   Ile Ile Gly Val Thr Phe Cys
           355
<210> 14
   <211> 359
   <212> PRT
   <213> Artificial sequence
<220>
   <223> BciVI 359C, C271S
<400> 14
   Met Asn Phe Phe Ser Leu His Pro Asn Val Tyr Ala Thr Gly Arg Pro
   1               5                   10                  15
   Lys Gly Leu Ile Gly Met Leu Glu Asn Val Trp Val Ser Asn His Thr
               20                  25                  30
   Pro Gly Glu Gly Thr Leu Tyr Leu Ile Ser Gly Phe Ser Asn Tyr Asn
           35                  40                  45
   Gly Gly Val Arg Phe Tyr Glu Thr Phe Thr Glu His Ile Asn Gln Gly
       50                  55                  60
   Gly Arg Val Ile Ala Ile Leu Gly Gly Ser Thr Ser Gln Arg Leu Ser
   65                  70                  75                  80
   Ser Arg Gln Val Val Glu Glu Leu Leu Asn Arg Gly Val Glu Val His
                   85                  90                  95
   Ile Ile Asn Arg Lys Arg Ile Leu His Ala Lys Leu Tyr Gly Thr Ser
               100                 105                 110
   Asn Asn Leu Gly Glu Ser Leu Val Val Ser Ser Gly Asn Phe Thr Gly
           115                 120                 125
   Pro Gly Met Ser Gln Asn Ile Glu Ala Ser Leu Leu Leu Asp Asn Asn
       130                 135                 140
   Thr Thr Gln Ser Met Gly Phe Ser Trp Asn Asp Met Ile Ser Glu Met
   145                 150                 155                 160
   Leu Asn Gln Asn Trp His Ile His Asn Met Thr Asn Ala Thr Asp Ala
                   165                 170                 175
   Ser Pro Gly Trp Asn Leu Leu Tyr Asp Glu Arg Thr Thr Asn Leu Thr
               180                 185                 190
   Leu Asp Glu Thr Glu Arg Val Thr Leu Ile Val Thr Leu Gly His Ala
           195                 200                 205
   Asp Thr Ala Arg Ile Gln Ala Ala Pro Gly Thr Thr Ala Gly Gln Gly
       210                 215                 220
   Thr Gln Tyr Phe Trp Leu Ser Lys Asp Ser Tyr Asp Phe Phe Pro Pro
   225                 230                 235                 240
   Leu Thr Ile Arg Asn Arg Arg Gly Thr Lys Ala Thr Tyr Ser Ser Leu
                   245                 250                 255
   Ile Asn Met Asn Tyr Ile Asp Ile Asn Tyr Thr Asp Thr Gln Ser Arg
               260                 265                 270
   Val Thr Phe Glu Ala Glu Asn Asn Phe Asp Phe Arg Leu Gly Thr Gly
           275                 280                 285
   Lys Leu Arg Tyr Thr Gly Val Ala Lys Ser Asn Asp Ile Ala Ala Ile
       290                 295                 300
   Thr Arg Val Gly Asp Ser Asp Tyr Glu Leu Arg Ile Ile Lys Gln Gly
   305                 310                 315                 320
   Thr Pro Glu His Ser Gln Leu Asp Pro Tyr Ala Val Ser Phe Ile Gly
                   325                 330                 335
   Asn Arg Gly Lys Arg Phe Gly Tyr Ile Ser Asn Glu Glu Phe Gly Arg
               340                 345                 350
   Ile Ile Gly Val Thr Phe Cys
           355
<210> 15
   <211> 278
   <212> PRT
   <213> Artificial sequence
<220>
   <223> EcoRI 278C
<400> 15
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Cys Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Cys
           275
<210> 16
   <211> 278
   <212> PRT
   <213> Artificial sequence
<220>
   <223> EcoRI 278C, C218A
<400> 16
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ala Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Cys
           275
<210> 17
   <211> 278
   <212> PRT
   <213> Artificial sequence
<220>
   <223> EcoRI 278C, C218S
<400> 17
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ser Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Cys
           275
<210> 18
   <211> 3907
   <212> DNA
   <213> Artificial sequence
<220>
   <223> plasmid P1040
<400> 18
   cagctgcatt aatgaatcgg ccaacgcgcg gggagaggcg gtttgcgtat tgggcgctct 60
   tccgcttcct cgctcactga ctcgctgcgc tcggtcgttc ggctgcggcg agcggtatca 120
   gctcactcaa aggcggtaat acggttatcc acagaatcag gggataacgc aggaaagaac 180
   atgtgagcaa aaggccagca aaaggccagg aaccgtaaaa aggccgcgtt gctggcgttt 240
   ttccataggc tccgcccccc tgacgagcat cacaaaaatc gacgctcaag tcagaggtgg 300
   cgaaacccga caggactata aagataccag gcgtttcccc ctggaagctc cctcgtgcgc 360
   tctcctgttc cgaccctgcc gcttaccgga tacctgtccg cctttctccc ttcgggaagc 420
   gtggcgcttt ctcatagctc acgctgtagg tatctcagtt cggtgtaggt cgttcgctcc 480
   aagctgggct gtgtgcacga accccccgtt cagcccgacc gctgcgcctt atccggtaac 540
   tatcgtcttg agtccaaccc ggtaagacac gacttatcgc cactggcagc agccactggt 600
   aacaggatta gcagagcgag gtatgtaggc ggtgctacag agttcttgaa gtggtggcct 660
   aactacggct acactagaag aacagtattt ggtatctgcg ctctgctgaa gccagttacc 720
   ttcggaaaaa gagttggtag ctcttgatcc ggcaaacaaa ccaccgctgg tagcggtggt 780
   ttttttgttt gcaagcagca gattacgcgc agaaaaaaag gatctcaaga agatcctttg 840
   atcttttcta cggggtctga cgctcagtgg aacgaaaact cacgttaagg gattttggtc 900
   atgagattat caaaaaggat cttcacctag atccttttaa attaaaaatg aagttttaaa 960
   tcaatctaaa gtatatatga gtaaacttgg tctgacagtt accaatgctt aatcagtgag 1020
   gcacctatct cagcgatctg tctatttcgt tcatccatag ttgcctgact ccccgtcgtg 1080
   tagataacta cgatacggga gggcttacca tctggcccca gtgctgcaat gataccgcga 1140
   gacccacgct caccggctcc agatttatca gcaataaacc agccagccgg aagggccgag 1200
   cgcagaagtg gtcctgcaac tttatccgcc tccatccagt ctattaattg ttgccgggaa 1260
   gctagagtaa gtagttcgcc agttaatagt ttgcgcaacg ttgttgccat tgctacaggc 1320
   atcgtggtgt cacgctcgtc gtttggtatg gcttcattca gctccggttc ccaacgatca 1380
   aggcgagtta catgatcccc catgttgtgc aaaaaagcgg ttagctcctt cggtcctccg 1440
   atcgttgtca gaagtaagtt ggccgcagtg ttatcactca tggttatggc agcactgcat 1500
   aattctctta ctgtcatgcc atccgtaaga tgcttttctg tgactggtga gtactcaacc 1560
   aagtcattct gagaatagtg tatgcggcga ccgagttgct cttgcccggc gtcaatacgg 1620
   gataataccg cgccacatag cagaacttta aaagtgctca tcattggaaa acgttcttcg 1680
   gggcgaaaac tctcaaggat cttaccgctg ttgagatcca gttcgatgta acccactcgt 1740
   gcacccaact gatcttcagc atcttttact ttcaccagcg tttctgggtg agcaaaaaca 1800
   ggaaggcaaa atgccgcaaa aaagggaata agggcgacac ggaaatgttg aatactcata 1860
   ctcttccttt ttcaatatta ttgaagcatt tatcagggtt attgtctcat gagcggatac 1920
   atatttgaat gtatttagaa aaataaacaa ataggggttc cgcgcacatt tccccgaaaa 1980
   gtgccacctg acgtctaata cgactcacta tagggagaaa gcttaccatg gaggacgcca 2040
   agaacatcaa gaagggcccg gcgcccttct acccgctgga ggacgggacc gccggcgagc 2100
   agctccacaa ggccatgaag cggtacgccc tggtgccggg cacgatcgcc ttcaccgacg 2160
   cccacatcga ggtcgacatc acctacgcgg agtacttcga gatgagcgtg cgcctggccg 2220
   aggccatgaa gcggtacggc ctgaacacca accaccggat cgtggtgtgc tcggagaaca 2280
   gcctgcagtt cttcatgccg gtgctgggcg ccctcttcat cggcgtggcc gtcgccccgg 2340
   cgaacgacat ctacaacgag cgggagctgc tgaacagcat ggggatcagc cagccgaccg 2400
   tggtgttcgt gagcaagaag ggcctgcaga agatcctgaa cgtgcagaag aagctgccca 2460
   tcatccagaa gatcatcatc atggacagca agaccgacta ccagggcttc cagtcgatgt 2520
   acacgttcgt gaccagccac ctcccgccgg gcttcaacga gtacgacttc gtcccggaga 2580
   gcttcgaccg ggacaagacc atcgccctga tcatgaacag cagcggcagc accggcctgc 2640
   cgaagggggt ggccctgccg caccggaccg cctgcgtgcg cttctcgcac gcccgggacc 2700
   ccatcttcgg caaccagatc atcccggaca ccgccatcct gagcgtggtg ccgttccacc 2760
   acggcttcgg catgttcacg accctgggct acctcatctg cggcttccgg gtggtcctga 2820
   tgtaccggtt cgaggaggag ctgttcctgc ggagcctgca ggactacaag atccagagcg 2880
   cgctgctcgt gccgaccctg ttcagcttct tcgccaagag caccctgatc gacaagtacg 2940
   acctgtcgaa cctgcacgag atcgccagcg gggcgccccc gctgagcaag gaggtgggcg 3000
   aggccgtggc caagcggttc cacctcccgg gcatccgcca gggctacggc ctgaccgaga 3060
   ccacgagcgc gatcctgatc acccccgagg gggacgacaa gccgggcgcc gtgggcaagg 3120
   tggtcccgtt cttcgaggcc aaggtggtgg acctggacac cggcaagacc ctgggcgtga 3180
   accagcgggg cgagctgtgc gtgcgggggc cgatgatcat gagcggctac gtgaacaacc 3240
   cggaggccac caacgccctc atcgacaagg acggctggct gcacagcggc gacatcgcct 3300
   actgggacga ggacgagcac ttcttcatcg tcgaccggct gaagtcgctg atcaagtaca 3360
   agggctacca ggtggcgccg gccgagctgg agagcatcct gctccagcac cccaacatct 3420
   tcgacgccgg cgtggccggg ctgccggacg acgacgccgg cgagctgccg gccgcggtgg 3480
   tggtgctgga gcacggcaag accatgacgg agaaggagat cgtcgactac gtggccagcc 3540
   aggtgaccac cgccaagaag ctgcggggcg gcgtggtgtt cgtggacgag gtcccgaagg 3600
   gcctgaccgg gaagctcgac gcccggaaga tccgcgagat cctgatcaag gccaagaagg 3660
   gcggcaagat cgccgtgtga ggactagtta taagactgac tagcccgatg ggcctcccaa 3720
   cgggccctcc tcccctcctt gcaccgagat taataaaaaa aaaaaaaaaa aaaaaaaaaa 3780
   aaaaaaaaaa aaaaaaaaaa aaaaaaaaaa aaaaaaaatg catccccccc cccccccccc 3840
   cccccccccc ccccaaaggc tcttttcaga gccaccagaa ttcggatact ctagacatat 3900
   gcttaag 3907
<210> 19
   <211> 1870
   <212> RNA
   <213> Artificial sequence
<220>
   <223> PpLuc (GC)GA- -A64-C30-histone stem-loop N5
<400> 19
   gggagaaagc uuaccaugga ggacgccaag aacaucaaga agggcccggc gcccuucuac      60
   ccgcuggagg acgggaccgc cggcgagcag cuccacaagg ccaugaagcg guacgcccug     120
   gugccgggca cgaucgccuu caccgacgcc cacaucgagg ucgacaucac cuacgcggag     180
   uacuucgaga ugagcgugcg ccuggccgag gccaugaagc gguacggccu gaacaccaac     240
   caccggaucg uggugugcuc ggagaacagc cugcaguucu ucaugccggu gcugggcgcc     300
   cucuucaucg gcguggccgu cgccccggcg aacgacaucu acaacgagcg ggagcugcug     360
   aacagcaugg ggaucagcca gccgaccgug guguucguga gcaagaaggg ccugcagaag     420
   auccugaacg ugcagaagaa gcugcccauc auccagaaga ucaucaucau ggacagcaag     480
   accgacuacc agggcuucca gucgauguac acguucguga ccagccaccu cccgccgggc     540
   uucaacgagu acgacuucgu cccggagagc uucgaccggg acaagaccau cgcccugauc     600
   augaacagca gcggcagcac cggccugccg aagggggugg cccugccgca ccggaccgcc     660
   ugcgugcgcu ucucgcacgc ccgggacccc aucuucggca accagaucau cccggacacc     720
   gccauccuga gcguggugcc guuccaccac ggcuucggca uguucacgac ccugggcuac     780
   cucaucugcg gcuuccgggu gguccugaug uaccgguucg aggaggagcu guuccugcgg     840
   agccugcagg acuacaagau ccagagcgcg cugcucgugc cgacccuguu cagcuucuuc     900
   gccaagagca cccugaucga caaguacgac cugucgaacc ugcacgagau cgccagcggg     960
   ggcgccccgc ugagcaagga ggugggcgag gccguggcca agcgguucca ccucccgggc    1020
   auccgccagg gcuacggccu gaccgagacc acgagcgcga uccugaucac ccccgagggg    1080
   gacgacaagc cgggcgccgu gggcaaggug gucccguucu ucgaggccaa ggugguggac   1140
   cuggacaccg gcaagacccu gggcgugaac cagcggggcg agcugugcgu gcgggggccg    1200
   augaucauga gcggcuacgu gaacaacccg gaggccacca acgcccucau cgacaaggac    1260
   ggcuggcugc acagcggcga caucgccuac ugggacgagg acgagcacuu cuucaucguc    1320
   gaccggcuga agucgcugau caaguacaag ggcuaccagg uggcgccggc cgagcuggag    1380
   agcauccugc uccagcaccc caacaucuuc gacgccggcg uggccgggcu gccggacgac    1440
   gacgccggcg agcugccggc cgcgguggug gugcuggagc acggcaagac caugacggag    1500
   aaggagaucg ucgacuacgu ggccagccag gugaccaccg ccaagaagcu gcggggcggc    1560
   gugguguucg uggacgaggu cccgaagggc cugaccggga agcucgacgc ccggaagauc    1620
   cgcgagaucc ugaucaaggc caagaagggc ggcaagaucg ccgugugagg acuaguuaua    1680
   agacugacua gcccgauggg ccucccaacg ggcccuccuc cccuccuugc accgagauua    1740
   auaaaaaaaa aaaaaaaaaa aaaaaaaaaa aaaaaaaaaa aaaaaaaaaa aaaaaaaaaa    1800
   aaaaaaugca uccccccccc cccccccccc cccccccccc ccaaaggcuc uuuucagagc    1860
   caccagaauu                                                           1870
<210> 20
   <211> 277
   <212> PRT
   <213> Artificial sequence
<220>
   <223> EcoRI K277C, C218V
<400> 20
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
   130 135 140
   Arg Ser His Lys Asn Ile Ser Glu Ile Ala Asn Phe Met Leu Ser Glu
   145 150 155 160
   Ser His Phe Pro Tyr Val Leu Phe Leu Glu Gly Ser Asn Phe Leu Thr
   165 170 175
   Glu Asn Ile Ser Ile Thr Arg Pro Asp Gly Arg Val Val Asn Leu Glu
   180 185 190
   Tyr Asn Ser Gly Ile Leu Asn Arg Leu Asp Arg Leu Thr Ala Ala Asn
   195 200 205
   Tyr Gly Met Pro Ile Asn Ser Asn Leu Val Ile Asn Lys Phe Val Asn
   210 215 220
   His Lys Asp Lys Ser Ile Met Leu Gln Ala Ala Ser Ile Tyr Thr Gln
   225 230 235 240
   Gly Asp Gly Arg Glu Trp Asp Ser Lys Ile Met Phe Glu Ile Met Phe
   245 250 255
   Asp Ile Ser Thr Thr Ser Leu Arg Val Leu Gly Arg Asp Leu Phe Glu
   260 265 270
   Gln Leu Thr Ser Cys
   275
<210> 21
   <211> 290
   <212> PRT
   <213> Artificial sequence
<220>
   <223> EcoRI 278(12xG)C, C218V
<400> 21
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Val Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Gly Gly Gly Gly Gly Gly Gly
           275                 280                 285
   Gly Cys
       290
<210> 22
   <211> 278
   <212> PRT
   <213> Artificial sequence
<220>
   <223> EcoRI 278C, C218V
<400> 22
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Val Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Cys
           275
<210> 23
   <211> 284
   <212> PRT
   <213> Artificial sequence
<220>
   <223> EcoRI 278-HisTag-C
<400> 23
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Cys Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys His His His His His His Cys
           275                 280
<210> 24
   <211> 284
   <212> PRT
   <213> Artificial sequence
<220>
   <223> EcoRI 278HisTag-C, C218A
<400> 24
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ala Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys His His His His His His Cys
           275                 280
<210> 25
   <211> 284
   <212> PRT
   <213> Artificial sequence
<220>
   <223> EcoRI 278HisTag-C, C218S
<400> 25
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ser Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys His His His His His His Cys
           275                 280
<210> 26
   <211> 284
   <212> PRT
   <213> Artificial sequence
<220>
   <223> EcoRI 278HisTag-C, C218V
<400> 26
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Val Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys His His His His His His Cys
           275                 280
<210> 27
   <211> 288
   <212> PRT
   <213> Artificial sequence
<220>
   <223> EcoRI 278-FlexLinker-C
<400> 27
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Cys Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Ser Gly Gly Gly Gly Ser Cys
           275                 280                 285
<210> 28
   <211> 288
   <212> PRT
   <213> Artificial sequence
<220>
   <223> EcoRI 278FlexLinker-C, C218A
<400> 28
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ala Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Ser Gly Gly Gly Gly Ser Cys
               275                 280                 285
<210> 29
   <211> 288
   <212> PRT
   <213> Artificial sequence
<220>
   <223> EcoRI 278FlexLinker-C, C218S
<400> 29
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ser Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Ser Gly Gly Gly Gly Ser Cys
           275                 280                 285
<210> 30
   <211> 288
   <212> PRT
   <213> Artificial sequence
<220>
   <223> EcoRI 278FlexLinker-C, C218V
<400> 30
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
   115 120 125
   Gly Lys Arg Gly Asp Gln Asp Leu Met Ala Ala Gly Asn Ala Ile Glu
   130 135 140
   Arg Ser His Lys Asn Ile Ser Glu Ile Ala Asn Phe Met Leu Ser Glu
   145 150 155 160
   Ser His Phe Pro Tyr Val Leu Phe Leu Glu Gly Ser Asn Phe Leu Thr
   165 170 175
   Glu Asn Ile Ser Ile Thr Arg Pro Asp Gly Arg Val Val Asn Leu Glu
   180 185 190
   Tyr Asn Ser Gly Ile Leu Asn Arg Leu Asp Arg Leu Thr Ala Ala Asn
   195 200 205
   Tyr Gly Met Pro Ile Asn Ser Asn Leu Val Ile Asn Lys Phe Val Asn
   210 215 220
   His Lys Asp Lys Ser Ile Met Leu Gln Ala Ala Ser Ile Tyr Thr Gln
   225 230 235 240
   Gly Asp Gly Arg Glu Trp Asp Ser Lys Ile Met Phe Glu Ile Met Phe
   245 250 255
   Asp Ile Ser Thr Thr Ser Leu Arg Val Leu Gly Arg Asp Leu Phe Glu
   260 265 270
   Gln Leu Thr Ser Lys Gly Gly Gly Gly Ser Gly Gly Gly Gly Ser Cys
   275 280 285
<210> 31
   <211> 294
   <212> PRT
   <213> Artificial sequence
<220>
   <223> EcoRI 278-FlexLinker-HisTag-C
<400> 31
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Cys Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Ser Gly Gly Gly Gly Ser His
           275                 280                 285
   His His His His His Cys
   290
<210> 32
   <211> 294
   <212> PRT
   <213> Artificial sequence
<220>
   <223> EcoRI 278-FlexLinker-HisTag-C, C218A
<400> 32
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
                   195                 200                 205
   Tyr Gly Met Pro Ile Asn Ser Asn Leu Ala Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Ser Gly Gly Gly Gly Ser His
           275                 280                 285
   His His His His His Cys
       290
<210> 33
   <211> 294
   <212> PRT
   <213> Artificial sequence
<220>
   <223> EcoRI 278-FlexLinker-HisTag-C, C218S
<400> 33
   Met Ser Asn Lys Lys Gln Ser Asn Arg Leu Thr Glu Gln His Lys Leu
   1               5                   10                  15
   Ser Gln Gly Val Ile Gly Ile Phe Gly Asp Tyr Ala Lys Ala His Asp
               20                  25                  30
   Leu Ala Val Gly Glu Val Ser Lys Leu Val Lys Lys Ala Leu Ser Asn
           35                  40                  45
   Glu Tyr Pro Gln Leu Ser Phe Arg Tyr Arg Asp Ser Ile Lys Lys Thr
       50                  55                  60
   Glu Ile Asn Glu Ala Leu Lys Lys Ile Asp Pro Asp Leu Gly Gly Thr
   65                  70                  75                  80
   Leu Phe Val Ser Asn Ser Ser Ile Lys Pro Asp Gly Gly Ile Val Glu
                   85                  90                  95
   Val Lys Asp Asp Tyr Gly Glu Trp Arg Val Val Leu Val Ala Glu Ala
               100                 105                 110
   Lys His Gln Gly Lys Asp Ile Ile Asn Ile Arg Asn Gly Leu Leu Val
           115                 120                 125
   Gly Lys Arg Gly Asp Gln Asp Leu Met Ala Ala Gly Asn Ala Ile Glu
       130                 135                 140
   Arg Ser His Lys Asn Ile Ser Glu Ile Ala Asn Phe Met Leu Ser Glu
   145                 150                 155                 160
   Ser His Phe Pro Tyr Val Leu Phe Leu Glu Gly Ser Asn Phe Leu Thr
                   165                 170                 175
   Glu Asn Ile Ser Ile Thr Arg Pro Asp Gly Arg Val Val Asn Leu Glu
               180                 185                 190
   Tyr Asn Ser Gly Ile Leu Asn Arg Leu Asp Arg Leu Thr Ala Ala Asn
           195                 200                 205
   Tyr Gly Met Pro Ile Asn Ser Asn Leu Ser Ile Asn Lys Phe Val Asn
       210                 215                 220
   His Lys Asp Lys Ser Ile Met Leu Gln Ala Ala Ser Ile Tyr Thr Gln
   225                 230                 235                 240
   Gly Asp Gly Arg Glu Trp Asp Ser Lys Ile Met Phe Glu Ile Met Phe
                   245                 250                 255
   Asp Ile Ser Thr Thr Ser Leu Arg Val Leu Gly Arg Asp Leu Phe Glu
               260                 265                 270
   Gln Leu Thr Ser Lys Gly Gly Gly Gly Ser Gly Gly Gly Gly Ser His
           275                 280                 285
   His His His His His Cys
       290
<210> 34
   <211> 294
   <212> PRT
   <213> Artificial sequence
<220>
   <223> EcoRI 278-FlexLinker-HisTag-C, C218V
<400> 34
<210> 35
   <211> 26
   <212> PRT
   <213> Artificial sequence
<220>
   <223> linker
<400> 35
   Ser Gly Gly Gly Ser Gly Gly Gly Gly Ser Gly Gly Gly Gly Ser Gly
   1               5                   10                  15
   Gly Gly Gly Ser Gly Gly Gly Ser Leu Gln
               20                  25
<210> 36
   <211> 12
   <212> PRT
   <213> Artificial sequence
<220>
   <223> Rigid-linker_AEAAAKEAAAKA
<400> 36
   Ala Glu Ala Ala Ala Lys Glu Ala Ala Ala Lys Ala
   1               5                   10
<210> 37
   <211> 46
   <212> PRT
   <213> Artificial sequence
<220>
   <223> Rigid-linker
<400> 37
   Ala Glu Ala Ala Ala Lys Glu Ala Ala Ala Lys Glu Ala Ala Ala Lys
   1 5 10 15
   Glu Ala Ala Ala Lys Ala Leu Glu Ala Glu Ala Ala Ala Lys Glu Ala
   20 25 30
   Ala Ala Lys Glu Ala Ala Ala Lys Glu Ala Ala Ala Lys Ala
   35 40 45
<210> 38
   <211> 5
   <212> PRT
   <213> Artificial sequence
<220>
   <223> Rigid-linker (EAAAK)1
<400> 38
   Glu Ala Ala Ala Lys
   1 5
<210> 39
   <211> 10
   <212> PRT
   <213> Artificial sequence
<220>
   <223> Rigid-linker (EAAAK)2
<400> 39
   Glu Ala Ala Ala Lys Glu Ala Ala Ala Lys
   1 5 10
<210> 40
   <211> 15
   <212> PRT
   <213> Artificial sequence
<220>
   <223> Rigid-linker (EAAAK)3
<400> 40
   Glu Ala Ala Ala Lys Glu Ala Ala Ala Lys Glu Ala Ala Ala Lys
   1 5 10 15
<210> 41
   <211> 4
   <212> PRT
   <213> Artificial sequence
<220>
   <223> Linker-GGG(GC)
<400> 41
   Gly Gly Gly Gly
   1
<210> 42
   <211> 5
   <212> PRT
   <213> Artificial sequence
<220>
   <223> Linker-GGGGG(GC)
<400> 42
   Gly Gly Gly Gly Gly
   1 5
<210> 43
   <211> 6
   <212> PRT
   <213> Artificial sequence
<220>
   <223> Gly-linker (G)6
<400> 43
   Gly Gly Gly Gly Gly Gly
   1 5
<210> 44
   <211> 8
   <212> PRT
   <213> Artificial sequence
<220>
   <223> Gly-linker (G)8
<400> 44
   Gly Gly Gly Gly Gly Gly Gly Gly
   1 5
<210> 45
   <211> 5
   <212> PRT
   <213> Artificial sequence
<220>
   <223> Linker GGGS
<400> 45
   Gly Gly Gly Gly Ser
   1 5
<210> 46
   <211> 6
   <212> PRT
   <213> Artificial sequence
<220>
   <223> Linker-G4SG
<400> 46
   Gly Gly Gly Gly Ser Gly
   1 5
<210> 47
   <211> 9
   <212> PRT
   <213> Artificial sequence
<220>
   <223> Linker-G4SG4(GC)
<400> 47
   Gly Gly Gly Gly Ser Gly Gly Gly Gly
   1 5
<210> 48
   <211> 10
   <212> PRT
   <213> Artificial sequence
<220>
   <223> Linker-(G4S)2
<400> 48
   Gly Gly Gly Gly Ser Gly Gly Gly Gly Ser
   1 5 10
<210> 49
   <211> 10
   <212> PRT
   <213> Artificial sequence
<220>
   <223> Linker (GGGGS)2
<400> 49
   Gly Gly Gly Gly Ser Gly Gly Gly Gly Ser
   1 5 10
<210> 50
   <211> 15
   <212> PRT
   <213> Artificial sequence
<220>
   <223> Linker-(GGGGS)3
<400> 50
   Gly Gly Gly Gly Ser Gly Gly Gly Gly Ser Gly Gly Gly Gly Ser
   1               5                   10                  15
<210> 51
   <211> 20
   <212> PRT
   <213> Artificial sequence
<220>
   <223> Linker (GGGGS)4
<400> 51
   Gly Gly Gly Gly Ser Gly Gly Gly Gly Ser Gly Gly Gly Gly Ser Gly
   1               5                   10                  15
   Gly Gly Gly Ser
               20
<210> 52
   <211> 16
   <212> PRT
   <213> Artificial sequence
<220>
   <223> Linker-GGGSGGGGSGGGGSGG(GC)
<400> 52
   Gly Gly Gly Ser Gly Gly Gly Gly Ser Gly Gly Gly Gly Ser Gly Gly
   1               5                   10                  15
<210> 53
   <211> 15
   <212> PRT
   <213> Artificial sequence
<220>
   <223> Linker-GGSGGGGSGGGGSGG
<400> 53
   Gly Gly Ser Gly Gly Gly Gly Ser Gly Gly Gly Gly Ser Gly Gly
   1               5                   10                  15
<210> 54
   <211> 4
   <212> PRT
   <213> Artificial sequence
<220>
   <223> Linker-GSG
<400> 54
   Gly Ser Gly Ser
   1
<210> 55
   <211> 21
   <212> PRT
   <213> Artificial sequence
<220>
   <223> Linker(GC)
<400> 55
   Gly Ser Gly Gly Gly Gly Ser Ala Trp Ser His Pro Gln Phe Glu Lys
   1 5 10 15
   Gly Gly Gly Gly Ser
   20
<210> 56
   <211> 5
   <212> PRT
   <213> Artificial sequence
<220>
   <223> Rigid-linker
<400> 56
   Pro Ala Pro Ala Pro
   1 5
<210> 57
   <211> 11
   <212> PRT
   <213> Artificial sequence
<220>
   <223> Linker-PPPGSRHDKIH(GC)
<400> 57
   Pro Pro Pro Gly Ser Arg His Asp Lys Ile His
   1 5 10
<210> 58
   <211> 4
   <212> PRT
   <213> Artificial sequence
<220>
   <223> Linker-SGG(GC)
<400> 58
   Ser Gly Gly Gly
   1
<210> 59
   <211> 4
   <212> PRT
   <213> Artificial sequence
<220>
   <223> Linker-SGS
<400> 59
   Gly Ser Gly Ser
   1
<210> 60
   <211> 17
   <212> PRT
   <213> Artificial sequence
<220>
   <223> 19-A
<400> 60
   Glu Trp Leu Lys Ala Phe Tyr Glu Lys Val Leu Glu Lys Leu Lys Glu
   1 5 10 15
   Leu
<210> 61
   <211> 15
   <212> PRT
   <213> Artificial sequence
<220>
   <223> Avi-Tag
<400> 61
   Gly Leu Asn Asp Ile Phe Glu Ala Gln Lys Ile Glu Trp His Glu
   1 5 10 15
<210> 62
   <211> 26
   <212> PRT
   <213> Artificial sequence
<220>
   <223> Calmodulin Tag
<400> 62
   Lys Arg Arg Trp Lys Lys Asn Phe Ile Ala Val Ser Ala Ala Asn Arg
   1 5 10 15
   Phe Lys Lys Ile Ser Ser Ser Gly Ala Leu
   20 25
<210> 63
   <211> 13
   <212> PRT
   <213> Artificial sequence
<220>
   <223> E-tag
<400> 63
   Gly Ala Pro Val Pro Tyr Pro Asp Pro Leu Glu Pro Arg
   1 5 10
<210> 64
   <211> 16
   <212> PRT
   <213> Artificial sequence
<220>
   <223> ELK16-Tag
<400> 64
   Leu Glu Leu Glu Leu Lys Leu Lys Leu Glu Leu Glu Leu Lys Leu Lys
   1 5 10 15
<210> 65
   <211> 8
   <212> PRT
   <213> Artificial sequence
<220>
   <223> FLAG tag
<400> 65
   Asp Tyr Lys Asp Asp Asp Asp Lys
   1 5
<210> 66
   <211> 6
   <212> PRT
   <213> Artificial sequence
<220>
   <223> Poly-Glutamate-Tag
<400> 66
   Glu Glu Glu Glu Glu Glu
   1 5
<210> 67
   <211> 9
   <212> PRT
   <213> Artificial sequence
<220>
   <223> HA-tag
<400> 67
   Tyr Pro Tyr Asp Val Pro Asp Tyr Ala
   1 5
<210> 68
   <211> 6
   <212> PRT
   <213> Artificial sequence
<220>
   <223> HIS tag
<400> 68
   His His His His His His
   1 5
<210> 69
   <211> 16
   <212> PRT
   <213> Artificial sequence
<220>
   <223> Isopeptag
<400> 69
   Thr Asp Lys Asp Met Thr Ile Thr Phe Thr Asn Lys Lys Asp Ala Glu
   1 5 10 15
<210> 70
   <211> 10
   <212> PRT
   <213> Artificial sequence
<220>
   <223> Myc Tag
<400> 70
   Glu Gln Lys Leu Ile Ser Glu Glu Asp Leu
   1 5 10
<210> 71
   <211> 15
   <212> PRT
   <213> Artificial sequence
<220>
   <223> S-tag
<400> 71
   Lys Glu Thr Ala Ala Ala Lys Phe Glu Arg Gln His Met Asp Ser
   1               5                   10                  15
<210> 72
   <211> 38
   <212> PRT
   <213> Artificial sequence
<220>
   <223> SBP tag
<400> 72
   Met Asp Glu Lys Thr Thr Gly Trp Arg Gly Gly His Val Val Glu Gly
   1               5                   10                  15
   Leu Ala Gly Glu Leu Glu Gln Leu Arg Ala Arg Leu Glu His His Pro
               20                  25                  30
   Gln Gly Gln Arg Glu Pro
           35
<210> 73
   <211> 13
   <212> PRT
   <213> Artificial sequence
<220>
   <223> Spy Tag
<400> 73
   Ala His Ile Val Met Val Asp Ala Tyr Lys Pro Thr Lys
   1               5                   10
<210> 74
   <211> 13
   <212> PRT
   <213> Artificial sequence
<220>
   <223> SofTag 1
<400> 74
   Ser Leu Ala Glu Leu Leu Asn Ala Gly Leu Gly Gly Ser
   1 5 10
<210> 75
   <211> 8
   <212> PRT
   <213> Artificial sequence
<220>
   <223> Sof Tag 2
<400> 75
   Thr Gln Asp Pro Ser Arg Val Gly
   1 5
<210> 76
   <211> 8
   <212> PRT
   <213> Artificial sequence
<220>
   <223> Strep-II-Tag
<400> 76
   Trp Ser His Pro Gln Phe Glu Lys
   1 5
<210> 77
   <211> 11
   <212> PRT
   <213> Artificial sequence
<220>
   <223> T7 Epitope tag
<400> 77
   Met Ala Ser Met Thr Gly Gly Gln Gln Met Gly
   1 5 10
<210> 78
   <211> 14
   <212> PRT
   <213> Artificial sequence
<220>
   <223> V5 Tag
<400> 78
   Gly Lys Pro Ile Pro Asn Pro Leu Leu Gly Leu Asp Ser Thr
   1 5 10
<210> 79
   <211> 11
   <212> PRT
   <213> Artificial sequence
<220>
   <223> VSV Tag
<400> 79
   Tyr Thr Asp Ile Glu Met Asn Arg Leu Gly Lys
   1               5                   10
<210> 80
   <211> 8
   <212> PRT
   <213> Artificial sequence
<220>
   <223> Xpress Tag
<400> 80
   Asp Leu Tyr Asp Asp Asp Asp Lys
   1               5